# EUROPEAN PATENT APPLICATION

(11) **EP 4 389 913 A1**
(43) Date of publication of application: **26.06.2024**
(21) Application number: 22216603.5
(22) Date of filing: 23.12.2022
(51) Int. Cl.: C12Q 1/6816

(54) **A PCR-ELISA ANALOGOUS ANTIBODY-FREE METHOD FOR DETECTING A TARGET NUCLEIC ACID IN A SAMPLE**

(71) Applicant: Universität Heidelberg, 69117 Heidelberg (DE)
(72) Inventor: KRÄMER, Roland, 48165 Münster (DE); BONENBERGER, Miles, 69123 Heidelberg (DE)
(74) Representative: Vossius & Partner Patentanwälte Rechtsanwälte mbB

(57) **Abstract**

A method for detecting a target nucleic acid in a sample, the method comprising the following steps: (a) coupling of an amplification product of the target nucleic acid, wherein said amplification product is generated if said target nucleic acid is in the sample, comprising modified nucleotides having a first reactive moiety to a second reactive moiety that is linked to a cysteine protease activator molecule to produce amplification products of the target nucleic acid labelled with a cysteine protease activator molecule; (b) optionally immobilizing the labelled amplification products on a surface; (c) contacting the optionally immobilized, labelled amplification products with a reversibly inactivated cysteine protease, wherein the catalytic cysteine residue of the reversibly inactivated cysteine protease is blocked by a disulfide bond or a selenylsulfide bond, and reactivating the proteolytic activity of said cysteine protease by said cysteine protease activator molecule by disulfide bond or selenylsulfide bond reduction; (d) contacting the reactivated cysteine protease with a cysteine protease substrate, wherein said substrate provides a detectable signal when hydrolysed by said reactivated cysteine protease; and (e) monitoring said detectable signal, wherein the presence of the detectable signal is indicative of the presence of said target nucleotide sequence in said sample.

## Description

The present invention relates to a method for detecting a target nucleic acid in a sample, the method comprising the following steps: (a) coupling of an amplification product of the target nucleic acid, wherein said amplification product is generated if said target nucleic acid is in the sample, comprising modified nucleotides having a first reactive moiety to a second reactive moiety that is linked to a cysteine protease activator molecule to produce amplification products of the target nucleic acid labelled with a cysteine protease activator molecule; (b) optionally immobilizing the labelled amplification products on a surface; (c) contacting the optionally immobilized, labelled amplification products with a reversibly inactivated cysteine protease, wherein the catalytic cysteine residue of the reversibly inactivated cysteine protease is blocked by a disulfide bond or a selenylsulfide bond, and reactivating the proteolytic activity of said cysteine protease by said cysteine protease activator molecule by disulfide bond or selenylsulfide bond reduction; (d) contacting the reactivated cysteine protease with a cysteine protease substrate, wherein said substrate provides a detectable signal when hydrolysed by said reactivated cysteine protease; and (e) monitoring said detectable signal, wherein the presence of the detectable signal is indicative of the presence of said target nucleic acid in said sample.

The process of DNA detection was pushed forward in 1985 when Kary B. Mullis published the first report of his newly developed "Enzymatic Amplification of [...] Genomic Sequences [...]", a method for which he was rewarded with the Nobel Prize in 1993 (Saiki et al., 1985). He and his group optimized the process further and finally termed the method "Specific Synthesis of DNA *in Vitro* via a Polymerase-Catalyzed Chain Reaction" two years later (Mullis & Faloona, 1987). Besides the accumulation of target DNA by enzymatic amplification (copying) via a polymerase for various applications like DNA cloning and sequencing, polymerase chain reaction (PCR) enables the detection of minute DNA amounts. Shortly after the first publications, Mullis and his group utilized the method to identify the Human Immunodeficiency Virus (HIV) in patients, something that had only been done before by serological tests screening for antibodies which could however be merely detected in later states of the infection (Kwok et al., 1987). The efficiency of the process was strongly increased by the same group after utilization of the thermostable Taq DNA polymerase which allowed for a detection of a single-copy genomic sequence within 10⁵ cells by an efficient 10⁷-fold amplification of the DNA (Saiki et al., 1988). PCR is a vital part of most molecular biology laboratories ever since, with many applications in the field of genetic fingerprinting and DNA profiling (e.g., paternity tests, criminal forensics). As initially shown by Mullis et al., the process allows for diagnostics of infectious diseases and identification of pathogens which has been done and optimized widely since (Sachse et al., 2003).

The process was further developed in 1996 when Heid et al. presented a method for quantification and observation of the DNA amplification in real-time which they called real-time PCR (qPCR). By addition of a fluorescence dye (SYBR Green) or by dual-labelled fluorogenic probes (TaqMan), a fluorescence increase can be detected in every PCR-cycle which allows for an estimation of DNA amounts in the sample. This method allows for a more detailed identification and quantification of pathogens and is routinely used for diagnostics in clinical microbiology laboratories and many other scientific fields (Espy et al., 2006; Deepak et al., 2007). Most recently, the worldwide demand for this technique increased drastically due to the severe acute respiratory syndrome coronavirus 2 (SARS-CoV-2) pandemic. Tailored conditions for the detection of this virus were immediately established and millions of tests are performed each day (Corman et al., 2020). While qPCR is a fast, easily handled, very accurate and relatively low error-prone technique for identification of DNA samples, it is still one of the most expensive detection methods requiring non ubiquitous machinery and expensive chemicals. It is therefore of great interest to develop similar specific but cheaper alternatives which could be utilized in laboratories with standard molecular biological equipment.

Before development of the molecular method PCR, pathogens were mainly identified via the serological method called Enzyme-Linked Immunosorbent Assay (ELISA) developed by Engvall et al. in 1971. This method allows for a quantitative determination of either antibodies or antigens within a sample by making use of a specific interaction between the two. The utilized detection antibodies are coupled to an enzyme which processes a fluorogenic or chromogenic substrate leading to an amplified and quantifiable signal which can be detected with standard laboratory equipment. A disadvantage of this method is however the deployment of very specific antibodies which require an elaborate production process often involving animal experiments or long-time cell cultures (Birch et al., 2006; Rodrigues et al., 2010; Stills et al., 2010). Nonetheless, this technique is widely available compared to qPCR, with lower costs and almost similar accuracy and is, among others, also applicable for the detection of the coronavirus (Dhamad et al., 2020). The ELISA does, however, only allow for the detection of proteins or other specific molecules and not DNA which is unfavourable under various conditions.

In order to bridge this gap, a first immunodetection of DNA with biotinylated RNA and monoclonal antibodies was described in 1989 by Coutlée et al. and was later adapted towards a combination of both PCR and ELISA as first described in 1993 by Albert et al. They used streptavidin coated plates and DNA probes, labelled with biotin at their 5' end, for the immobilization of labelled target DNA which was amplified via PCR in the presence of the steroid digoxigenin-11-UTP (DIG-dUTP) nucleotides. The nucleotide that bound and incorporated digoxigenin was then detected via an enzyme coupled anti-digoxigenin antibody similar to ELISA. The enzymatic cleavage of applied specific chromogenic or fluorogenic substrate then allows for specific detection of DNA. PCR-ELISA is an immunodetection method which can identify PCR products. The enzymatic processing of deployed substrate leads to a strong signal amplification which directly reflects the amount of sample immobilized on the microplate via specific DNA-probes, allowing for a comparison between samples in a semi-quantitative manner.

When compared to conventional PCR detection via agarose gels, PCR-ELISA features higher specificity, higher sensitivity with lower detection limits, semi-quantifiability rather than a rough estimation of positive and negative signal and a quicker overall process resulting in improved diagnosis times. The method does, however, not allow for an accurate estimation of absolute DNA amounts which is only possible with qPCR. On the other hand, PCR-ELISA allows for specific large-scale screenings in 96- or even 384-well plates surpassing both magnitudes of conventional PCR and qPCR, all whilst utilizing standard laboratory equipment. An exemplarily direct comparison between qPCR and PCR-ELISA was conducted by Menotti et al. who utilized both methods to detect genomic DNA of the parasite *Toxoplasma gondii* in clinical patient samples. They found that of 144 clinical symptomatic and with qPCR positively tested patient samples (Coutlée et al., 1989), showed a false negative result with PCR-ELISA. The group of Aini et al. published comparable results and described qPCR to be ten times more sensitive compared to PCR-ELISA, which in turn is ten times more specific compared to agarose gel-based detection of DNA which was also confirmed by Bagheri et al.

By now, PCR-ELISA has been successfully utilized in a wide variety of medical diagnosis, such as in clinical cancer research (Cosan et al., 2011; Chan et al., 1996; Raji et al., 2011; Yie et al., 2006), fungal infections of susceptible patients (Hadrich et al., 2011; Badiee et al., 2009; Löffler et al., 1998) as well as detection of viruses and bacteria like HIV (Bagheri et al., 2013), Hepatitis (Tahk et al., 2012; Tahk et al., 2011), Epstein-Barr (Bazzichi et al., 1998), *Mycobacterium tuberculosis* (Zhou et al., 2020) and Methicillin- resistant *Staphylococcus aureus* (MRSA) (Daeschlein et al., 2006). Furthermore, the method provided a cheap and efficient tool for genotyping of blood groups (St-Louis, 2009) and successfully identified point mutations connected to the blood ailment beta thalassemias (Gill et al., 2008), thus substituting very expensive and protracted DNA sequencing. PCR-ELISA was also used for parasite tracings like *Trypanosomes* (Chansiri et al., 2002) and *Leishmania infantum* (Kobets et al., 2010; Medeiros et al., 2017), among others. Another important sector of PCR-ELISA application is the food industry, where a vast number of foodborne pathogens like *Brucella* (Al Dahouk et al., 2004), *Escherichia coli* (Daly et al., 2002) and *Salmonella* (Perelle et al., 2004), to mention only a few, have been efficiently detected. Besides pathogens, PCR-ELISA has been deployed to detect and identify genetically modified organisms in food (Liu et al., 2004).

DNA detection with PCR-ELISA has proven to be more sensitive compared to conventional PCR and features shorter analytic time, semi-quantifiable ability, lack of possibly toxic chemicals and is feasible with basic laboratory equipment. The biggest advantage however is the ability to perform multiplex screenings that allow for the detection of various specific sequences simultaneously. The initial PCR can be conducted with various primers in order to amplify different sequences within a single sample, identifying various target DNA without cross-reactivity due to very specific hybridization DNA probes (Perelle et al., 2004; Liu et al., 2004; Laitinen et al., 2002). This increases the already existent high-throughput ability of the assay manifold, greatly surpassing the detection via agarose or even qPCR in a process that could be easily automated.

Due to the similar process based on traditional ELISA, PCR-ELISA has the above-discussed disadvantageous need for antibodies which are not only costly but have to be produced in an elaborate process, often involving animal experiments. Furthermore, the antibodies have to be coupled to the utilized enzyme, rendering the whole process non-autonomous for the standard laboratory, although progress has been made towards facilitation of the production of improved PCR-ELISA specific antibodies (Gill et al., 2006).

A vast variety of different approaches towards DNA detection have been described in the past, many of which rely on signal amplification through either interaction and accumulation of reporter compounds or through enzymatic activity (Goggins et al., 2016). Many of the described methods of DNA sensing involve hybridization of DNA strands coupled to different nano-structures, leading to conformational changes which in turn results in detectable signals (Li et al., 2008). Other elaborate methods are described, involving engineered allosteric enzymes which are activated upon DNA hybridization with target DNA (Saghatelian et al., 2003). Many of these methods are however complex and still rather experimentally time consuming or costly and thus not yet feasible for high-throughput measurements utilizing commonly available tools.

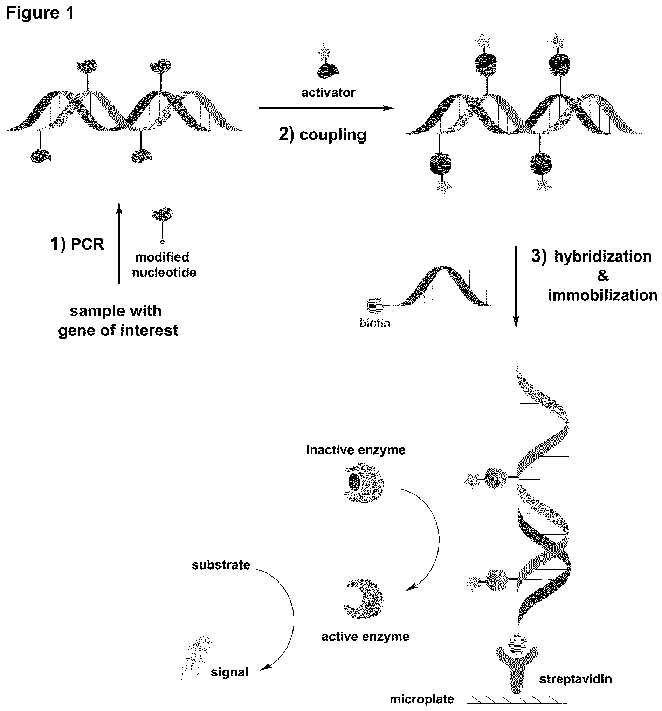

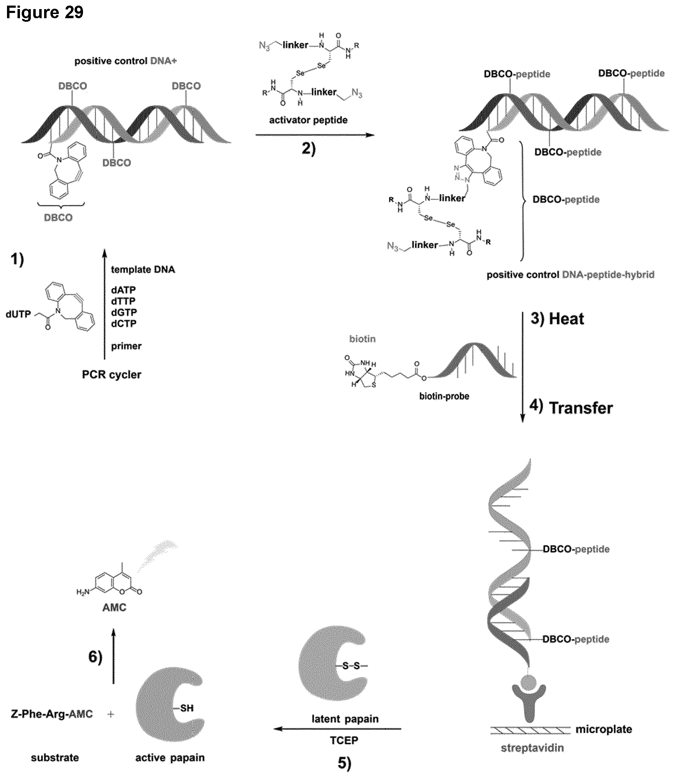

Accordingly, there is still an urgent need in the art to provide a method for detecting a target nucleic acid in a sample that eliminates these disadvantages while maintaining the efficiency and specificity of the PCR-ELISA. The present invention addresses this need and provides a new method for detecting a target nucleic acid in a sample that does not require antibodies, employs commonly available reagents as well as consumables and is compatible with standard laboratory equipment (Figures 1 and 29).

Hence, the present invention relates in a first aspect to a method for detecting a target nucleic acid in a sample , the method comprising the following steps: (a) coupling of an amplification product of the target nucleic acid, wherein said amplification product is generated if said target nucleic acid is in the sample, comprising modified nucleotides having a first reactive moiety to a second reactive moiety that is linked to a cysteine protease activator molecule to produce amplification products of the target nucleic acid labelled with a cysteine protease activator molecule; (b) optionally immobilizing the labelled amplification products on a surface; (c) contacting the optionally immobilized, labelled amplification products with a reversibly inactivated cysteine protease, wherein the catalytic cysteine residue of the reversibly inactivated cysteine protease is blocked by a disulfide bond or a selenylsulfide bond, and reactivating the proteolytic activity of said cysteine protease by said cysteine protease activator molecule by disulfide bond or selenylsulfide bond reduction; (d) contacting the reactivated cysteine protease with a cysteine protease substrate, wherein said substrate provides a detectable signal when hydrolysed by said reactivated cysteine protease; and (e) monitoring said detectable signal, wherein the presence of the detectable signal is indicative of the presence of said target nucleic acid in said sample.

The term "target" or "target nucleic acid" as used herein refers to a nucleic acid of interest which is suspected to be in a sample and to be detected in the method of the invention. As used herein, the terms "nucleic acid", "nucleotide sequence" or "polynucleotide" are used interchangeably and refer to the arrangement of either deoxyribonucleotide and ribonucleotide residues in a polymer in either single- or double-stranded form of any length. As used herein, the term "nucleic acid" also includes DNA that contains one or more modified bases. Thus, DNA with a backbone modified for stability or for other reasons is also a "nucleic acid". The term "nucleic acid" as used herein embraces such chemically, enzymatically, or metabolically modified forms of nucleic acids, as well as the chemical forms of DNA characteristic of viruses and cells.

The term "sample" as used herein refers to any biological sample or other source of nucleic acid. Accordingly, the sample may be obtained from any source, including, but not limited to, animals and plants, cells, tissues, fluids, sub-cellular organelles of animals and plants, bacteria, Archaea, Protista, fungi, viruses, environmental samples, such as drinking water, samples obtained from foods or other consumables and the like. The animal may be any vertebrate or invertebrate animal, such as fish, birds and mammals. The animal may be human, a non-human mammal or another animal. Non-human mammals include, but are not limited to, cattle, sheep, pigs, horses, domestic pets, such as cats and dogs. Bacteria, Archaea, Protista, fungi and viruses may be pathogenic or non-pathogenic organisms. The term "pathogenic" as used herein refers to an organism which is associated with, or causative of, a disease or condition of plants or animals. Non-limiting examples of pathogens include viruses, protozoa, fungi, worms, and bacteria. Further sources include, but are not limited to, pathology samples inclusive of body fluids, such as cerebrospinal fluid, blood, serum, plasma, semen, urine, lymph etc., tumour, tissue or organ biopsies. Other sources include forensic samples that are suspected of containing detectable nucleic acids. Further sources also include chemically synthesized nucleic acids.

As used herein, the terms "amplifying", "amplification" and the like generally refer to any process that results in an increase in the copy number of a molecule or set of related molecules. As it applies to nucleic acid molecules, amplification means the production of multiple copies of a nucleic acid, or a portion of the nucleic acid. Amplifying may include denaturing the target nucleic acid, annealing primers to the target nucleic acid at a temperature that is below the melting temperatures of the primers, and enzymatically elongating from the primers to generate an amplification product. The denaturing, annealing and elongating steps each can be performed one or more times. In certain cases, the denaturing, annealing and elongating steps are performed multiple times such that the amount of amplification product is increasing, often times exponentially, although exponential amplification is not required by the present methods. Amplification typically requires the presence of deoxyribonucleoside triphosphates, a DNA polymerase enzyme and an appropriate buffer and/or co-factors for optimal activity of the polymerase enzyme.

Accordingly, the term "amplification product" refers to a nucleic acid product generated by a nucleic acid amplification technique.

Nucleic acid amplification techniques in accordance with the present invention generally include techniques that amplify the target nucleic acid and are suitable to incorporate the modified nucleotide into the amplification product as defined above. Amplification techniques include but are not limited to the generation of multiple DNA copies from one or a few copies of a template DNA molecule during a polymerase chain reaction (PCR).

In accordance with the present invention, the amplification product of the target nucleic acid comprises modified nucleotides having a first reactive moiety. As said "first reactive moiety" corresponds to a modification to naturally occurring nucleotides, the nucleotides are referred to as "modified nucleotides". The term "first reactive moiety" as used herein refers to a chemical group that is capable of participating in a reaction with the "second reactive moiety" that is linked to a cysteine protease activator molecule. Upon coupling, i.e., the reaction of a "first reactive moiety" of the modified nucleotides with a "second reactive moiety" that is linked to the cysteine protease activator molecules, amplification products of the target nucleic acid labelled with a cysteine protease activator molecule are produced.

It is understood that the labelled amplification products of the target nucleic acid may comprise one or more cysteine protease activator molecules, depending on the number of modified nucleotides that were incorporated during amplification.

In accordance with the method of the present invention, the second reactive moiety and the cysteine protease activator molecule are linked via a linker. Suitable linkers in accordance with the present invention include, but are not limited to, linker structures that enable water solubility, such as polyethylene glycol linkers, alkyl linkers.

In accordance with step (b) of the first aspect of the invention, the labelled amplification products are optionally immobilized on a surface.

The term "immobilized", "immobilization" and the like as used herein generally refer to the reversible or irreversible association of the amplification product of the target nucleic acid to a surface. If reversible, the probes remain associated with the solid support for a time sufficient for the method of the invention to be carried out.

The term "surface" as used herein is used interchangeably with the terms "solid support" or "stationary phase". Numerous immobilizing surfaces suitable for the method of the present invention are well known in the art and widely described in the literature. The surface may be any of the well-known supports or matrices which are currently widely used or proposed for immobilization, separation etc. in chemical or biochemical procedures. Such materials include, but are not limited to, any synthetic organic polymer such as polystyrene, polyvinylchloride, polyethylene; or nitrocellulose or cellulose acetate; or tosyl activated surfaces; or glass or nylon or any surface carrying a group suited for coupling of nucleic acids. The surface may take the form of particles, sheets, gels, filters, membranes, microfibre strips, tubes or plates, fibres or capillaries, made for example of a polymeric material, e.g., agarose, cellulose, alginate, teflon, latex or polystyrene or magnetic beads. Particularly preferred is the use of a microplate.

The term "microplate", "microtiter plate" or "multiwell plate" and the like as used herein refers to arrays of discrete wells that come in standard formats (96, 384 and 1536 wells) which are used for examination of the physical, chemical or biological characteristics of a quantity of samples in parallel.

Attachment of the labelled amplification products of the target nucleic acid to the surface may be established by a surface binding moiety that is associated with the labelled amplification products. For example, a "surface binding moiety", as referred to herein, may be one member (or partner) of an "affinity binding pair". A variety of affinity binding pairs are known in the art and exemplary representatives of which are referred to herein below. Prominent examples of affinity binding pairs, and which are particularly preferred herein, are, without intending to be limiting, biotin and streptavidin, or DNA and a DNA binding protein (e.g., either the lac I repressor protein or the lac operator sequence to which it binds) and the like. In any of these cases, one member (or partner) of the binding pair is attached to (or is inherently part of) the solid support and the other member (or partner) is attached to (or is inherently part of) the labelled amplification product.

The term "affinity binding pair" refers to two components which recognize and bind to one another specifically (i.e., in preference to binding to other molecules). Such binding pairs when bound together form a complex. Attachment of appropriate functional groups to the surface may be performed by methods well known in the art, which include for example, attachment through hydroxyl, carboxyl, aldehyde or amino groups which may be provided by treating the solid support to provide suitable surface coatings. Solid supports presenting appropriate moieties for the attachment of the binding partner may be produced by routine methods known in the art. Attachment of appropriate functional groups to the target nucleic acid of the invention may be performed by ligation or introduced during synthesis or amplification, for example, using primers carrying an appropriate moiety, such as biotin or a particular sequence, preferably an amino acid sequence, for capture.

In accordance with step (c) of the first aspect of the invention, the optionally immobilized, labelled amplification products are contacted with a reversibly inactivated cysteine protease.

In a particularly preferred embodiment, the reversibly inactivated cysteine protease is used at a concentration between 0.1 and 2 nM, more preferably between 0.5 and 1.5 nM, and most preferably in a concentration of about 1 nM.

A "protease" is an enzyme that catalyzes the hydrolysis of proteins into smaller peptides or single amino acids. The term "cysteine protease" as used herein refers to proteases which are distinguished by presence therein of a cysteine residue at their active site (i.e., the catalytic cysteine residue) which plays a critical role in the catalytic process. Cysteine proteases share a common catalytic mechanism that involves a nucleophilic cysteine thiol in a catalytic triad or dyad (Rawat et al., 2021).

Generally, cysteine proteases that may be used in accordance with the present invention include cysteine proteases that comprise this catalytic cysteine thiol residue.

Examples of cysteine proteases include, but are not limited to, the plant cysteine proteases such as papain, bromelain, ficin, aleurain, oryzain and actinidin. For an extensive listing of cysteine proteases that may be inhibited by the cysteine protease inhibitors of the present invention, see Rawlings et Biochem. J. 290:205-218 (1993).

In a preferred embodiment in accordance with the first aspect of the present invention, the cysteine protease is papain or a papain-like protease comprising or consisting of the amino acid sequence of SEQ ID NO: 1 or of an amino acid sequence which is at least 85% identical to the core structure of the amino acid sequence of SEQ ID NO: 1, wherein the catalytic cysteine residue and the catalytic basic amino acid residue are retained.

Papain obtained from the papaya plant was first sequenced by Mitchel et al. in 1970 who found that the enzyme consists of 212 amino acids with three disulfide bridges between cysteine residues and one free cysteine thiol group. All papain-like cysteine proteases have limited sequence homology but a very conserved core structure and thus share similar active sites around the catalytically active cysteine, hence the enzyme family name (Novinec & Lenarcic, 2013). In this context it is noted that the very conserved core structure corresponds to the amino acids at positions x to y of SEQ ID NO: 1. If not stated otherwise, the following amino acid positions indicated are based on the mature peptide of SEQ ID NO: 1.

The papain precursor protein contains 345 amino acid residues (SEQ ID NO: 2) and consists of a signal sequence (positions 1-18 of SEQ ID NO: 3), a propeptide (positions 19-133 of SEQ ID NO: 4) and the mature peptide (positions 134-345 of SEQ ID NO: 1). The amino acid sequence of SEQ ID NO: 1 corresponds to the mature papain peptide from *Carica papaya.* The amino acid sequence of the wild-type version of papain, as well as the corresponding protein encoding nucleotide sequence are available from the Uniprot Database: https://www.uniprot.org under the Reference number: P00784.

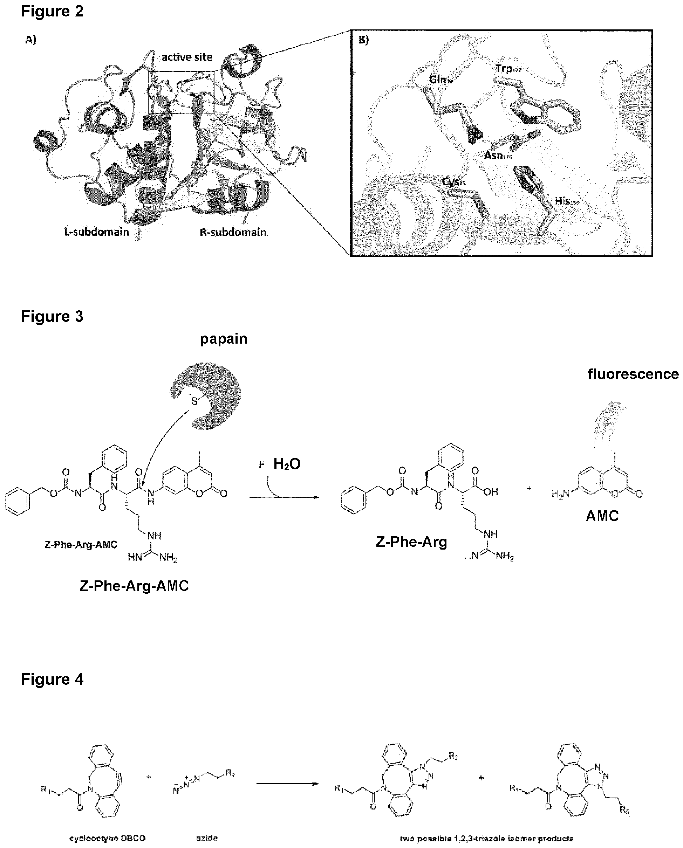

As depicted by Novinec et al. and Fernández-Lucas et al. the active site of papain lays within the V-shaped cleft between the L (left) and R (right) subdomain of papain, with α-helices of the L-sub-domain directly facing a β-barrel-like structure of the R-subdomain. A three-dimensional structure of the global and surface positively charged enzyme and its active site is depicted in Figure 2 on the basis of the published X-ray data by Pickersgill et al. The catalytically active Cys₂₅ in the L-subdomain and His₁₅₉ in the R-subdomain are directly opposed in the v-shaped cleft and hydrolyse the peptide bond in the presence of water.

Thus, in accordance with a particularly preferred embodiment, the cysteine protease is papain or a papain-like protease comprising or consisting of the amino acid sequence of SEQ ID NO: 1 or of an amino acid sequence which is at least 85% identical to the core structure of the amino acid sequence of SEQ ID NO: 1, wherein the catalytic cysteine residue and the catalytic histidine residue are retained. The catalytic cysteine and histidine residues correspond to positions 25 and 159 of the amino acid sequence of SEQ ID NO: 1.

In accordance with the above-described preferred or particularly preferred embodiments of the first aspect of the present invention, the amino acid sequence sharing at least 85% sequence identity with the core structure of the amino acid sequence of SEA ID NO: 1 displays with increasing preference least 86% sequence identity, at least 87% sequence identity, at least 88% sequence identity and at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity and at least 99% sequence identity with the core structure SEQ ID NO: 1. It is understood that in accordance with the above-mentioned particularly preferred embodiment, the catalytic cysteine and histidine residues at positions 25 and 159 of SEQ ID NO: 1 are retained.

It is furthermore understood that the papain-like proteases sharing at least 85% identity with the core structure of the amino acid sequence of SEQ ID NO: 1 maintain their catalytic activity (e.g., within at least 50%, 80%, 90% or 100% activity) (before reversible inhibition) compared to wild type papain consisting of SEQ ID NO: 1 as measured by methods known in the art.

In accordance with the present invention, the term "percent (%) sequence identity" describes the number of matches ("hits") of identical amino acids/nucleotides of two or more aligned amino acid or nucleic acid sequences as compared to the number of amino acid residues or nucleotides making up the overall length of the template nucleic acid or amino acid sequences. In other terms, using an alignment, for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

The term "enzyme" as used herein generally refers to a substance that catalyzes or promotes one or more chemical or biochemical reactions, which usually includes enzymes totally or partially composed of a polypeptide. An "enzyme" typically increases the rate of a particular chemical reaction without itself being consumed or permanently altered and without altering the equilibrium constant of the chemical reaction. At the end of a catalyzed reaction, the enzyme is substantially unchanged, and the substrates have undergone transformation into new products.

The term "protein" as used herein is used interchangeably with the term polypeptide and refers to polymers constructed from one or more chains of amino acid residues linked by peptide bonds. The group of "polypeptides" consists of molecules with more than 30 amino acids, which is in distinction to the group of peptides consisting of up to 30 amino acids. As used herein, the term "(poly)peptides" refers more generally to both "peptides" and "polypeptides". The term "(poly)peptides" also refers to chemically or post-translationally modified peptides or polypeptides. Generally, the terms apply to naturally occurring amino acid polymers, as well as to amino acid polymers in which one or more amino acid residue is an artificial chemical analogue of a corresponding naturally occurring amino acid.

In accordance with the present invention, "reversibly inactivated", "reversibly inhibited" or "latent" cysteine proteases are cysteine proteases that comprise a catalytic cysteine residue which is blocked by a disulfide bond or a selenylsulfide bond. As the catalytic cysteine thus cannot participate in catalytic processes, the proteolytic activity of such cysteine proteases is (reversibly) inactivated.

The term "cysteine protease activator molecule" as used herein generally relates to molecules which are able to reactivate a reversibly inactivated cysteine protease.

The cysteine protease activator molecule of the present invention reactivates the proteolytic activity of said cysteine protease by disulfide bond or selenylsulfide bond reduction. Upon disulfide bond or selenylsulfide bond reduction, the thiol residue of the catalytic cysteine is free to participate in proteolytic reactions.

Wishing not to be bound by theory, disulfide bond or selenylsulfide bond reduction is achieved via nucleophilic attack by a free thiol or selenol residue comprised in the cysteine protease activator molecule

In accordance with the present invention, the method further comprises (d) contacting the reactivated cysteine protease with a cysteine protease substrate, wherein said substrate provides a detectable signal when hydrolysed by said reactivated cysteine protease; and (e) monitoring said detectable signal, wherein the presence of the detectable signal is indicative of the presence of said target nucleic acid in said sample.

The phrase "cysteine protease substrate" as used herein refers to a molecule subject to an enzymatic reaction, i.e., the substance that is acted upon by the (reactivated) cysteine protease. In accordance with the present invention, the substrate provides a detectable signal when hydrolysed by said reactivated cysteine protease.

The term "detectable signal" as used herein refers to a signal that is a direct consequence of the enzymatic hydrolysis of the reactivated cysteine protease substrate, which can be monitored directly or indirectly. For example, the detectable signal may be monitored by physical, such as spectroscopic, photochemical, biochemical, immunochemical or other chemical means. Accordingly, the substrate may be, e.g., a chromogenic or fluorogenic substrate that releases a coloured or fluorescent compound upon selective cleavage by the protease and the detectable signal thus can be a chromogenic or fluorescent signal but is not limited thereto. Among the most widely used chromogenic substrates are peptide derivatives of p-nitroanilides that release yellow coloured p-nitroaniline with an absorbance maximum at 380 nm and a modest molar absorbance (ε) of 13500 M-1 cm-1. Peptide derivatives of 7-amino-4-methylcoumarin (AMC) are widely used fluorogenic substrates that display an increase of 440-460 nm fluorescence upon release of the fluorophore.

The term "monitoring" as used herein comprises detecting said signal and optionally, measuring or quantitatively measuring said signal.

The "detectable signal" is to be distinguished from a "background signal", also generally referred to herein as "background noise". Background noise may, for example, occur due to the presence of an insufficiently inhibited cysteine protease, exhibiting residual proteolytic activity even in the absence of a cysteine protease activator molecule. Background noise may also be the consequence of self-activation of said cysteine protease or the presence of other activating molecules or proteases. In these cases, monitoring the detectable signal may thus also include the determination of the amount of the detectable signal that is increased relative to a background signal that is detected in a control sample, which may be, for example, a sample without cysteine protease activator molecule or a sample without target nucleic acid.

The increased amount of detectable signal relative to a background signal consequently corresponds to the detectable signal that is a direct consequence of the hydrolysis of the cysteine protease substrate upon reactivation of the cysteine protease (i.e., its proteolytic activity) due to the presence and activity of the cysteine protease activator molecule. As the cysteine protease activator molecule is coupled with the amplification product of the target nucleic acid, the presence of the detectable signal is thus indicative of the presence of target nucleic acid in the sample. It is understood that the amplification product of the target nucleic acid is only generated if said target nucleic acid is in said sample.

In a preferred embodiment of the first aspect of the present invention, the cysteine protease substrate is Z-Phe-Arg 7-amino-4-Methylcoumarin (Z-Phe-Arg-AMC).

Z-Phe-Arg 7-amido-4-methylcoumarin (Z-Phe-Arg-AMC) is a peptidomimetic substrate for papain and other cysteine proteases (Figure 3). The catalytic activity of the cysteine protease can be quantified by release of free fluorescent 7-amino-4-methylcoumarin (AMC) which excites at 360-380 nm and emits at 440-460 nm as shown in the examples herein below.

AMC coupled substrates were found to allow for far lower detection limits compared to the yet widely applied chromogenic nitroanilide coupled substrates (Zimmerman et al. 1977). Z-Phe-Arg-AMC was first synthesized by Morita *et al.* in 1977 and found to be specifically and efficiently hydrolyzed by plasma kallikrein. Further studies revealed that the substrate is also a viable candidate for the papain-like cysteine protease cathepsin B (Barrett, 1980) as well as for papain (Tchoupé et al. 1991), with the following kinetic parameters: kcat value of 52 s⁻¹, Km 0.42 mM (Ménard et al. 1990).

In a particularly preferred embodiment, the Z-Phe-Arg-AMC concentration is between 0.1 to 2 µM, more preferably between 0.25 and 2 µm, even more preferably between 0.5 and 2 µM and most preferably, between 1 and 2 µM.

In a further preferred embodiment in accordance with the first aspect of the present invention, the method further comprises step (a1) before step (a): amplifying the target nucleic acid via polymerase chain reaction, wherein modified nucleotides having said first reactive moiety are incorporated into the amplification product.

The terms "polymerase chain reaction" or "PCR" refers to the method of K.B. Mullis, U.S. Patent Nos. 4,683,195, 4,683,202, and 4,965,188, that describe a method for increasing the concentration of a segment of a nucleic acid target sequence in a mixture. This process for amplifying the target sequence consists of introducing an excess of two oligonucleotide primers to the DNA mixture containing the desired target nucleic acid, followed by a precise sequence of thermal cycling in the presence of a DNA polymerase. The two primers are complementary to their respective strands of the double stranded target nucleic acid. To effect amplification, the mixture is denatured, and the primers then annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction".

Because the desired amplified segments of the target sequence become the predominant sequences (in terms of concentration) in the mixture, they are said to be "PCR amplified" and are "PCR products", "amplicons", "amplification products" or "amplificates".

Generally, the term "primer" as used herein refers to an oligonucleotide capable of acting as a point of initiation of DNA synthesis when annealed to a nucleic acid template under conditions in which synthesis of a primer extension product, which is complementary to a nucleic acid strand (template) is initiated, i.e., in the presence of four different nucleotide triphosphates (NTPs) and a DNA polymerase in a suitable buffer ("buffer" includes pH, ionic strength, cofactors, etc.) and at a suitable temperature.

Preferably, the primer is an oligodeoxyribonucleotide. The primer may be comprised of any combination of nucleotides or analogues thereof, including modified nucleotides, or non-natural nucleotides. The primer can also include ribonucleotides. The primer must be sufficiently long to prime the synthesis of extension products in the presence of the agent for polymerization. The exact length of the primers will depend on many factors, including temperature, application, and source of primer. The term "annealing" or "priming" as used herein refers to the apposition of an oligodeoxynucleotide or nucleic acid to a template nucleic acid, whereby the apposition enables the polymerase to polymerize nucleotides into a nucleic acid molecule which is complementary to the template nucleic acid or a portion thereof. The term "hybridizing" used herein refers to the formation of a double-stranded nucleic acid from complementary single stranded nucleic acids. There is no intended distinction between the terms "annealing" and "hybridizing", and these terms will be used interchangeably.

In a further preferred embodiment in accordance with the first aspect of the present invention, the method further comprises step (a2) after step (a) and before step (b) or step (c), if step (b) is not performed: essentially removing unreacted cysteine protease activator and/or unreacted polymerase chain reaction components if the method includes an amplification step prior to step (a).

Unreacted polymerase chain reaction components include, but are not limited to, excess primers, nucleotides and/or polymerase. The term "essentially" describes the removal of more than 80% of the unreacted cysteine protease activator and/or 80% of the unreacted polymerase chain reaction components, such as more than 85%, more than 90, more than 95, more than 98% or more than 99%, such as 100%.

In accordance with the preferred embodiment described above, "essentially removing unreacted cysteine protease activator and/or unreacted polymerase chain reaction components" may be achieved by methods known in the art, such as DNA adsorption to silica gel (e.g. using the Wizard^{®} SV Gel and PCR Clean-Up System Columns, Promega), size exclusion chromatography using, e.g., commercially available size exclusion columns, such as the Bio-Rad Micro Bio-Spin column or a G-50 Sephadex Quick Spin column as shown in the examples herein below, ethanol or isopropanol precipitation and the like.

As outlined above, immobilization in step (b) in accordance with the method of the present invention can be achieved by, e.g., the use of a surface binding moiety that is associated with the labelled amplification products of the target nucleic acid.

Thus, in a particularly preferred embodiment in accordance with the first aspect of the present invention, wherein step (a1) and step (b) is performed, step (a1) comprises amplifying the target nucleic acid in the presence of primers comprising a terminal surface binding moiety at the 5' end.

As outlined above, the surface binding moiety allows an immobilization of the amplification products of the labelled amplification product to a surface.

In an alternative embodiment, wherein step (b) is performed, the method comprises a step (b1) after step (a) and before step (b): hybridizing the labelled amplification product to a nucleic acid probe that is complementary to a portion of the amplification product, wherein the nucleic acid probe comprises a surface binding moiety.

The term "nucleic acid probe" as used herein relates to oligonucleotides capable of binding in a base-specific manner to a complementary strand of the labelled amplification product. The term "hybridization" in accordance with the above-mentioned embodiment refers to the process in which the nucleic acid probe and the amplification product bind non-covalently to form a stable duplex region.

In case the labelled amplification product of the target nucleic acid is present in a double-stranded form (as it is the case, e.g., when the labelled amplification product is produced via polymerase chain reaction as described above), the necessary single-stranded labelled amplification products may be e.g., obtained by heating the double stranded labelled amplification product to a high temperature, such as temperatures between about 60 to 90°C, in order to break the hydrogen bonds between the two strands. It is noted that the above-mentioned temperature is dependent on the specific nucleic acid sequence. It lies within the competence of the skilled person to choose those suitable temperatures, and, generally, methods to generate said single-stranded labelled amplification products from said double-stranded labelled amplification products.

The term "about" as used herein can allow for a degree of variability in a value or range, for example, within 10%, within 5%, or within 1 % of a stated value or of a stated limit of a range.

Importantly, the introduction of the biotinylated DNA probe allows for multiplex screenings where different primers can be combined within a single PCR reaction mixture in order to simultaneously identify different pathogens, for example.

In particularly preferred embodiments, the surface binding moiety is a biotin moiety and the surface in step (b) is a streptavidin solid phase. As shown in the herein disclosed examples (e.g., Example 3, in particular, in sections 3.3 and 3.4 therein), it was found by the present inventors that an incorporation of biotin (i.e., as a surface binding moiety) into the DNA amplification product was particularly suitable for effecting an enrichment of the DNA amplification products by capturing the latter with a streptavidin-based solid phase and removal of impurities, and that this can advantageously enhance the efficiency of the reactivation of the proteolytic activity of the cysteine protease. However, it is also believed by the inventors, that similar advantageous, perhaps slightly less pronounced, effects can also be achieved by using alternative affinity binding pairs.

To amplify a target nucleic acid, the nucleic acid sequence must be accessible to the components of the amplification system. In general, this accessibility is ensured by isolating the nucleic acids from the sample.

Accordingly, in a further particularly preferred embodiment in accordance with the first aspect of the present invention, the method further comprises step (a0) before step (a1): isolating nucleic acid from said sample.

Nucleic acid may be isolated from the sample by any suitable means and methods available from the art, for example, those described in Green & Sambrook, 2012, including commercially available kits.

In order to ligate the cysteine protease activator molecule to modified DNA nucleotides, the conjugation strategy needs to provide a highly selective, bioorthogonal reaction that proceeds efficiently under mild conditions in order to prevent side reactions with and degradation of the DNA amplificate. To date, several bioorthogonal conjugation strategies have been introduced for a variety of applications.

Copper-free azide-alkyne cycloaddition was first mentioned by Blomquist in 1952 and further described by Wittig at the University of Heidelberg in 1961 (Wittig & Krebs, 1961). He observed that a cyclooctyne and phenyl azide reacted "especially addition friendly" to form a 1,2,3-triazole. This spontaneous reaction is due to the ring-strain of cyclooctyne, the smallest stable cyclic alkyne, which reacts in a strain promoted [3+2] azide-alkyne cycloaddition (SPAAC, also referred to in this work as ring-strain promoted click reaction, or simply click reaction). Agard et al. synthesized a first generation cyclooctyne reagent with an attached functional group for biorthogonal conjugation. They showed that the cyclooctyne derivative reacted faster with azides compared to linear alkynes but the second-order rate constant for the reaction with benzyl azide was only similar to the Staudinger ligation with a value of 2.4 × 10⁻³ M⁻¹ s⁻¹.

Accordingly, in a further preferred embodiment in accordance with the first aspect of the present invention, the first reactive moiety of said modified nucleotides is a terminal alkyne moiety and the second reactive moiety that is linked to said cysteine protease activator molecule is an azide moiety.

Different derivatives were since presented which not only improved the reaction rate constants but also featured better water solubility (Baskin et al., 2007; Dommerholt et al., 2016; Ning et al., 2008; Sletten & Bertozzi, 2008; Wu & Fokin, 2007). One widely used derivative is dibenzocyclooctyne (DBCO) described by Debets *et al.* in 2010. They reported an average second-order rate constant for an azide-alkyne cycloaddition of 0.3 M⁻¹ s⁻¹ which is over 100 times faster compared to the first cyclooctyne derivate by Agard *et al.* as well as the Staudinger Ligation. The only general disadvantage of DBCO is its relatively poor water solubility. This problem can be circumvented by coupling of DBCO to highly water-soluble compounds. The reaction between a DBCO derivative and an azide is depicted in Figure 4.

The term "derivative" as used herein refers to a compound that is formed from the same starting compound by attaching another molecule or atom to the beginning compound.

Ring-strain promoted click reactions are widely used for biomolecule labelling and are a vital part of recent chemical biology (Jewett & Bertozzi, 2010). There are a vast number of published findings about successful labelling of DNA via ring-strain promoted click chemistry (Shelbourne et al., 2011; Merkel et al., 2015). More recently, Eeftens *et al.* worked with magnetic tweezers for studies on DNA-protein interactions and utilized a reaction toolset composed of commercially available, DBCO coupled nucleotide derivative dUTP-DBCO, which was incorporated into DNA during PCR, and an azide moiety introduced into peptide activators. This toolset proved to be most efficient and offered a promising basis on which to build the method of the present invention.

Accordingly, in a particularly preferred embodiment in accordance with the first aspect of the present invention, the first reactive moiety of the modified nucleotide is dibenzocyclooctyne (DBCO).

Consequently, the labelled amplification products of the target nucleic acid comprising one or more modified nucleotide(s) comprising a DBCO moiety can then couple via a ring-strain promoted click reaction with the azide containing cysteine protease activator molecules.

The above-described particularly preferred embodiment includes, amongst others, modified deoxynucleoside triphosphates (dNTPs) which are coupled to the DBCO moiety via a linker, such as e.g., a polyethylene glycol (PEG) linker. One example of a modified nucleotide that may be used in accordance with the present invention is 5-DBCO-PEG₄-dUTP, which is commercially available.

In yet a further preferred embodiment in accordance with the first aspect of the present invention, the method further comprises step (c1) after step (a) or after step (b), if step (b) is performed, and before step (c): reversibly inactivating a proteolytically active cysteine protease by reaction with a thiosulfonate derivative, wherein preferably, the thiosulfonate derivative is S-Methyl methanethiosulfonate.

The cysteine protease is one of the primary tools of the method of the invention and the ability to obtain a reactivatable and stable inactive form is vital to the goal of a sensitive detection assay towards specific activators. For example, for papain it was proven as early as 1966 by Sluyterman *et al.* that it is recovered partly as an inactivated mixed disulfide between Cys25 of the active site and a free cysteine. This means that papain is only available in an already reversible inactive form, but the inactivation is not complete and thus papain shows a residual activity of up to 50% when freshly prepared (Drenth et al., 1971). With respect to the method of the invention, it is therefore of interest to use an inhibitor that essentially completely and reversibly inhibits the cysteine protease.

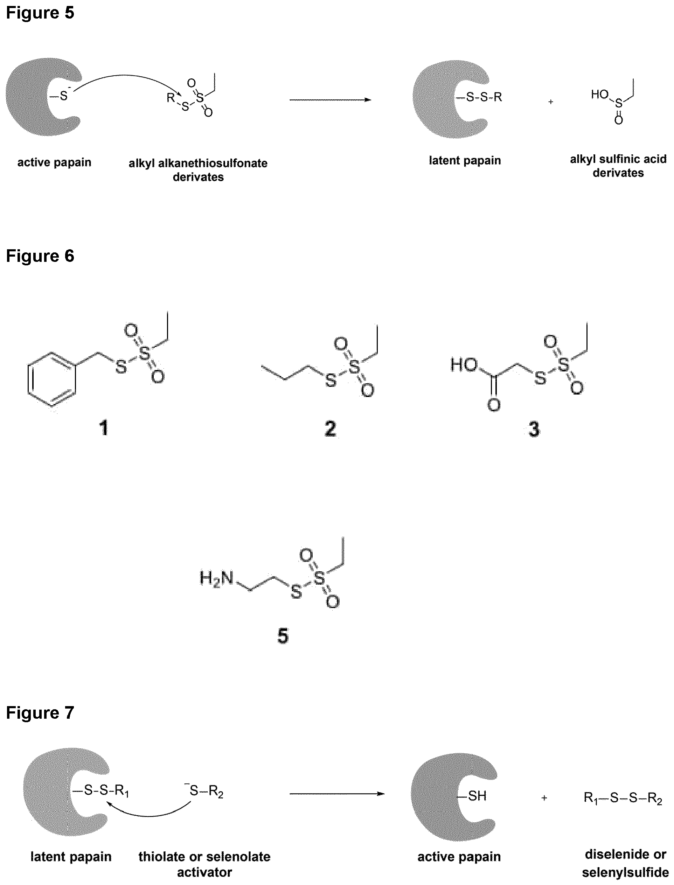

Previously, methanethiosulfonate has been tested as a reversible inhibitor on papain and it was found that it reacts rapidly and in equimolar proportions, suggesting that the Cys₂₅ is the only accessible thiol group of the enzyme (Smith et al, 1975). The reaction scheme is depicted in Figure 5.

Different thiosulfonate inhibitors were synthesized (Figure 6) and their efficiency on papain have been compared as shown in the examples herein below.

Accordingly, the term "thiosulfonate derivatives" in accordance with the above-described embodiment includes but is not limited to: S-benzyl-ethanethiosulfonate, S-propyl-ethanethiosulfonate, S-(2-acetic acid)- ethanethiosulfonate, S(-2-aminoethyl)-ethanethiosulfonate and S-Methyl methanethiosulfonate.

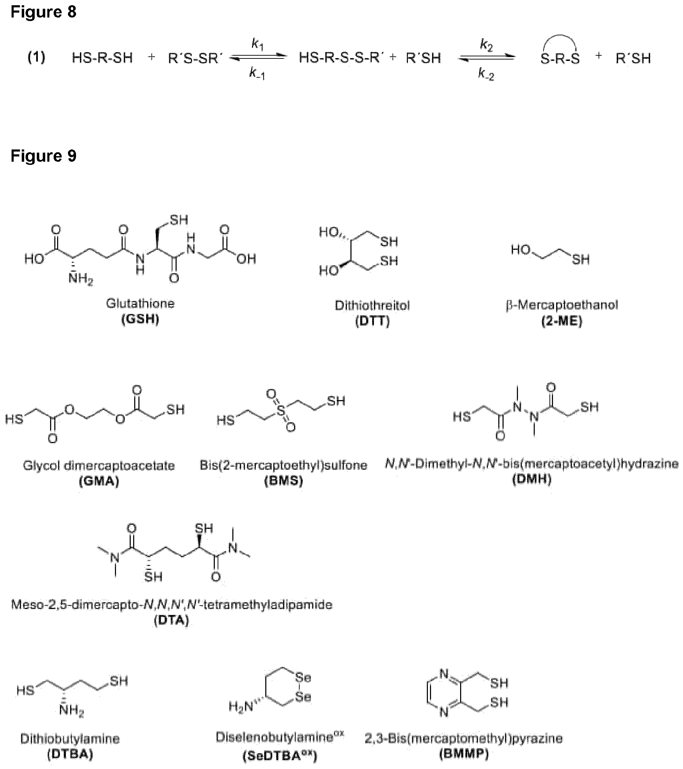

In line with the above, the disulfide nature of the reversibly inactivated cysteine protease allows for specific reactivation with free thiols or selenols, undergoing a thiol/disulfide or selenol/disulfide exchange reaction in the process (see Figure 7). A standard reaction pathway of dithiol/disulfide interchange reactions is depicted in Figure 8 equation 1. The selenylsulfide nature of reversibly inactivated cysteine proteases equally allow for specific reactivation with free thiols or selenols, undergoing thiol/selenylsulfide or selenol/selenylsulfide exchange reactions, respectively.

Thus, in accordance with a preferred embodiment of the first aspect of the invention, the cysteine protease activator molecule comprises a free thiol or selenol residue.

In the specific case of papain reactivation, only the first step of the aforementioned reaction equation 1 in Figure 8 is rate determining because the nucleophilic attack on the disulfide occurs on the sulfur of the better leaving group (e.g., methanethiol) and not on the sterically hindered Cys₂₅ (Shaked et al., 1980). Formation of a six-membered ring of the oxidized reductant is therefore not necessarily the driving force of that single-step reaction. This was illustrated by Whitesides and his group who synthesized three different dithiols, bis(2-mercaptoethyl) sulfone (BMS, see Figure 9), *N,N*'-dimethyl-N,N'-bis(mercaptoacetyl)hydrazine (DMH, see Figure 9) as well as meso-2,5-dimercapto-*N,N,N',N'-*tetramethyladipamide (DTA, see Figure 9) and compared disulfide exchange rates with different enzymes. All three reducing agents provided better reduction potentials and lower pKa values compared to DTT. DMH activated papain 32 times faster and DTA 4.5 times faster compared to DTT in their experiments.

Another activator, dithiobutylamine (DTBA), was first synthesized and presented by Lukesh et al. as a new and most efficient disulfide-reducing agent (Lukesh et al., 2012). Along with small disulfides, they tested the dithiol in a latent papain reactivation assay and discovered that it reduces the disulfide within the active site of papain 14 times faster compared to DTT which renders DTBA **(9)** a very efficient papain activator. A further advantageous feature is the accessible amine group of the compound which is ideal for conjugation to other molecules or linker structures, as can be used in the method of the invention. In a follow up work, Lukesh et al. exchanged sulfur for selenium in DTBA **(9)** and thus synthesized seleno-DTBA (SeDTBA (**11**)) which proved to be a very efficient papain activator as well when available in the reduced form (Lukesh et al., 2013). Due to the mentioned features, DTBA **(9)** and SeDTBA **(11)** were synthesized by the inventors and utilized in papain reactivation assays as shown in the examples herein below.

However, since the most potent papain inhibitors are peptide-based variants and the natural substrates are proteins or peptides, the inventors investigated if this substance class could also provide a useful and efficient tool for papain reactivation that can compete with non-peptide activators. Peptide activators feature additional advantages like the non-toxicity and non-odorous nature compared to many other applicable thiol compounds as well as better water solubility. Since there is no data about peptide-based activators used to reactivate latent papain and no published crystal structures of any of the latent papain forms as described herein below are available, virtual calculations and predictions of efficient amino acid sequences for an efficient peptide-based activator are hampered. Thus, the inventors synthesized a set of cysteine containing peptides comprising different lengths and randomly chosen sequences to cover a broad variety of different amino acids and evaluated this newly developed activator class. It was surprisingly found that peptide cysteine and selenocysteine protease activators (also referred to herein as "peptide activators") indeed are specifically suitable to be used in the method of the invention.

When comparing selenocysteine and cysteine, the redox potential differs greatly (selenocysteine E^{0'} = -0.488 V, cysteine E^{0'} = -0.233 V; Jacob et al., 2003). The acidity of selenocysteine was determined and compared to cysteine by Huber et al. in 1967 who discovered a difference of over three magnitudes (selenocysteine pKa 5.2, cysteine pKa 8.5). These traits elucidate that selenium is a Janus-faced element possessing both high nucleophilicity and high electrophilicity at the same time, making it resistant to permanent oxidation by oxygen, as described by Maroney et al. This results in big discrepancies of reaction rates in thiol/disulfide-like exchange reactions as examined by different groups (Pleasants et al., 1989; Rabenstein et al., 1996). Nauser et al. summarized the results as depicted in **Table 1.** As selenocysteine was proven to be the most potent papain activator of all screened compounds selenocysteine-based peptides were expected to result in specifically efficient cysteine protease activators.

**Table 1: Reaction rates of six different exchange reactions with sulfur and selenium. The central electrophilic atom, which is attacked by the nucleophile, is highlighted in bold notation. The leaving group of the reaction is highlighted in light grey colour. The rate constants were determined by Pleasants et al. and summarized by Nauser et al.**

| **Nucleophile** | **Electrophile** | **Reaction rate constant [M⁻¹ s⁻¹]** | |
|---|---|---|---|
| | | **pH 7 (measured)** | **pH 10 (calculated)** |
| R-S⁻ | R-S-S-R | 3.6 | 90 |
| R-Se⁻ | R-S-S-R | 1400 | 1400 |
| R-S⁻ | R-Se-Se-R | 1.3 x 10⁵ | 3.3 x 10⁶ |
| R-Se⁻ | R-Se-Se-R | 1.7 x 10⁷ | 1.7 x 10⁷ |
| R-S⁻ | R-S-Se-R | 11 | 275 |
| R-Se⁻ | R-Se-S-R | 2.6 x 10⁷ | 2.6 x 10⁷ |

It becomes clear that exchange reactions with selenium are even faster compared to thiol exchange reactions. As a nucleophile, selenium reacts more than 1000-fold faster compared to sulfur and when serving as an electrophile, nucleophilic attack on selenium is more than 10⁴-fold faster. The selenol/diselenide exchange reaction is almost 10⁷-fold faster compared to the thiol/diselenide exchange reaction. Furthermore, exchange reactions with selenium remain constant over a broader pH range due to the much lower pKa value, whereas reaction rates of sulfur increase drastically with higher pH value, though not approaching rates similar to those of selenium even at unphysiological alkaline pH. This is elucidated in an experiment by Hondal et al. where the rate of attack on a thioester was compared for cysteine and selenocysteine at pH 5 and 8 and reaction rates ratios of *kSelkS* were calculated. While the reaction with selenocysteine was 1000-fold faster at pH 5, the ratio decreased to 15- fold at pH 8 because, while rates of selenocysteine remain constant, rates of cysteine increased with increasing pH value. Selenocysteine is also much more efficient in one-electron- as well as two-electron-transfer reactions compared to cysteine as explained by Nauser et al., 2006 and Steinmann et al., 2008.

Accordingly, in a particularly preferred embodiment of the first aspect of the present invention, the cysteine protease activator molecule is: a) a non-peptide activator comprising dithiobutylamine (DTBA), seleno-dithiobutylamin (SeDTBA) or an aminothiophenol; or (b) a peptide activator comprising a cysteine moiety or a selenocysteine moiety; or (c) the cysteine protease activator molecule of the second aspect of the invention; or (d) the cysteine protease activator molecule of the third aspect of the invention

In accordance with the preferred embodiment described above, disulfide or selenylsulfide bond reduction is achieved via nucleophilic attack of the free thiol or selenol residue comprised in the cysteine protease activator molecule. Thus, it is understood that the cysteine activator molecules of item (a) comprise a free thiol or selenol residue. In case of items (b), (c) and (d), the free thiol or selenol residue is part of the cysteine or the selenocysteine moiety.

In this context it is of note that selenols readily oxidize to diselenides at physiological conditions in the presence of molecular oxygen. In this case, the selenol residue might not be readily available. Reduction of oxidized cysteine protease activators is therefore required shortly before use and realized most efficiently through dithiols, such as Tris(2-carboxyethyl)phosphine (TCEP). Burns et al. presented the phosphine TCEP to be a most efficient disulfide reducing agent with many beneficial properties and with the previously described characteristics of selenium, namely the high electrophilicity. The inventors found that TCEP can also be efficiently used for reduction of the herein described cysteine protease activator molecules comprising selenocysteine in case of oxidation to a diselenide. TCEP was further proven to be a poor papain activator making it an ideal reducing agent.

Accordingly, in yet a further preferred embodiment of the first aspect of the present invention, step c) further comprises the addition of Tris(2-carboxyethyl)phosphine (TCEP).

In a particularly preferred embodiment, the TCEP concentration is between 0 and 2 µM, preferably between 0.5 and 1.5 µM, and most preferably, the concentration is about 1 µM.

In this context it is noted that the specific concentration of TCEP depends on the concentration of the cysteine protease activator molecule and that it lies within the competence of the skilled person to choose appropriate concentrations of TCEP and cysteine protease activator molecule.

In accordance with the first aspect of the invention, the cysteine protease activator molecule is linked to the second reactive moiety as defined herein above, which is preferably an azide moiety. In case of the non-peptide activators comprising DTBA, SeDTBA or aminothiophenol of item (a), the second reactive moiety is preferably linked to the amine group of DTBA, SeDTBA or aminothiophenol. In case of the peptide activators of item (b), the second reactive moiety is preferably linked to the N-terminus of the peptide. In accordance with items (c) and (d), the second reactive moiety is preferably linked to the N-terminus (or termini) of the peptides or tripeptides (or tripeptide dimers) in accordance with the second or the third aspect of the invention.

In accordance with the first aspect of the invention, the linker linking the cysteine protease activator molecules as defined above to the second reactive moiety is furthermore preferably a polyethylene glycol linker.

In an even more preferred embodiment in accordance with item (b) of the particularly preferred embodiment described above, the peptide activator comprises or consists of 2 to 5 amino acids in length, a cysteine or a selenocysteine moiety and a C-terminal arginine, glycine or phenylalanine residue or a C-terminal arginine amide, glycine-amide or phenylalanine residue.

Examples of peptides in accordance with the above-described even more preferred embodiment of the invention are listed in **Table 2** and have been extensively tested as described in the examples herein below:

| C-terminal amide group | C-terminal carboxyl group |
|---|---|
| H₂N-CCRG-CONH₂ | H₂N-CLRCG-COOH |
| H₂N-CLCG-CONH₂ | H₂N-CWRG-COOH |
| H₂N-CLR-CONH₂ | H₂N-CLRG-COOH |
| H₂N-CLRG-CONH₂ | H₂N-CGGGG-COOH |
| H₂N-CEWG-CONH₂ | H₂N-CSGG-COOH |
| H₂N-FCLR-CONH₂ | |
| H₂N-CF-CONH₂ | |
| H₂N-CFR-CONH₂ | |
| H₂N-CL-CONH₂ | |
| H₂N-CVQG-CONH₂ | |
| H₂N-CKSG-CONH₂ | |

The inventors found that shorter peptides with *N*-terminal cysteine generally seem to be more efficient in papain reactivation. The most potent peptide with a single cysteine was found to be H₂N-CLR-CONH₂.

Thus, in an even more preferred embodiment, the cysteine protease activator molecule is the tripeptide H₂N-CLR-CONH₂.

The peptides described herein may be synthesized by any of the methods well known in the art. Suitable methods for chemically synthesizing the peptides include, for example, those described by Stuart and Young in Solid Phase Peptide Synthesis, Second Edition, Pierce Chemical Company (1984), and in Methods Enzymol., 289, Academic Press, Inc., New York (1997) or Hansen and Oddo, 2015; Stawikowski and Fields, 2002.

In summary, it was found that the method of the invention allows a simple, specific and fast analysis strategy featuring the ability to handle every single step in compliance with standard laboratory equipment while maintaining the possibility of automation of the process.

The definitions and preferred embodiments of the first aspect as far as being amenable for combination with the second aspect apply *mutatis mutandis* to the second aspect of the invention. This applies *mutatis mutandis* to the further aspects of the invention as described herein below. For instance, the definitions and preferred embodiments of the first and second aspect as far as being amenable for combination with the third aspect apply *mutatis mutandis* to the third aspect of the invention, etc.

In a second aspect, the present invention relates to a cysteine protease activator molecule for reactivating a reversibly inactivated cysteine protease comprising: (a) a tripeptide comprising a selenocysteine moiety and a C-terminal arginine or arginine-amide.

It is understood that the selenocysteine moiety comprised in the tripeptide comprises a free selenol residue, which allows for a specific reaction with reversibly inactivated cysteine proteases as described above, undergoing selenol/disulfide or selenol/selenylsulfide exchange reactions.

Specifically, the tripeptide *H₂N*-ULR-*CONH₂* was synthesized, screened and surprisingly identified as an extraordinary papain activator.

Accordingly, in a preferred embodiment in accordance with the second aspect of the present invention, the tripeptide is H₂N-ULR-COOH or H₂N-ULR-CONH₂.

As noted above, selenols readily oxidize to diselenides at physiological conditions in the presence of molecular oxygen. This dimerization effect offers several advantageous effects. On the one hand, diselenide peptides are more stable and less prone to oxidation by air which could lead not only to diselenides but also selenic acids and selenoxides. Furthermore, by attaching one peptide dimer to each modified nucleotide after amplification, not one but two equivalents of activator molecules will be present for each incorporated modified nucleotide. When used in the method of the present invention, this alone will amplify the efficiency by at least factor two because apart from the immobilized activator, the second one will be released after reduction with TCEP and thus will be more accessible for papain activation in solution.

Thus, in a further preferred embodiment of the second aspect of the present invention, the cysteine protease activator molecule further comprises a second of said tripeptides, wherein the selenocysteine moieties of said tripeptides are coupled via a diselenide bond.

In yet a further preferred embodiment in accordance with the second aspect of the present invention, the tripeptide(s) is (are) N-terminally coupled to a linker comprising an azide moiety.

The azide moiety of the above-described preferred embodiment of the invention corresponds to the "second reactive moiety" of the first aspect of the invention. Accordingly, and as outlined above, the cysteine protease activator molecules of the second aspect of the invention are suitable to be used in the method of the first aspect of the invention. Importantly, in case the cysteine protease activator molecule in accordance with the second aspect of the invention comprises two of said tripeptides, both of said tripeptides may be coupled to the above-described linker.

In a particularly preferred embodiment in accordance with the second aspect of the present invention, the linker comprises or consists of a polyethylene glycol moiety and an azide moiety.

In an even more preferred embodiment in accordance with the second aspect of the present invention, the linker is O-2-Azidoethyl-O'-[2-(succinylamino)-ethyl]dimethyleneglycol.

The O-2-Azidoethyl-O'-[2-(succinylamino)-ethyl]dimethyleneglycol linker is also referred to herein as "azido-PEGs" linker.

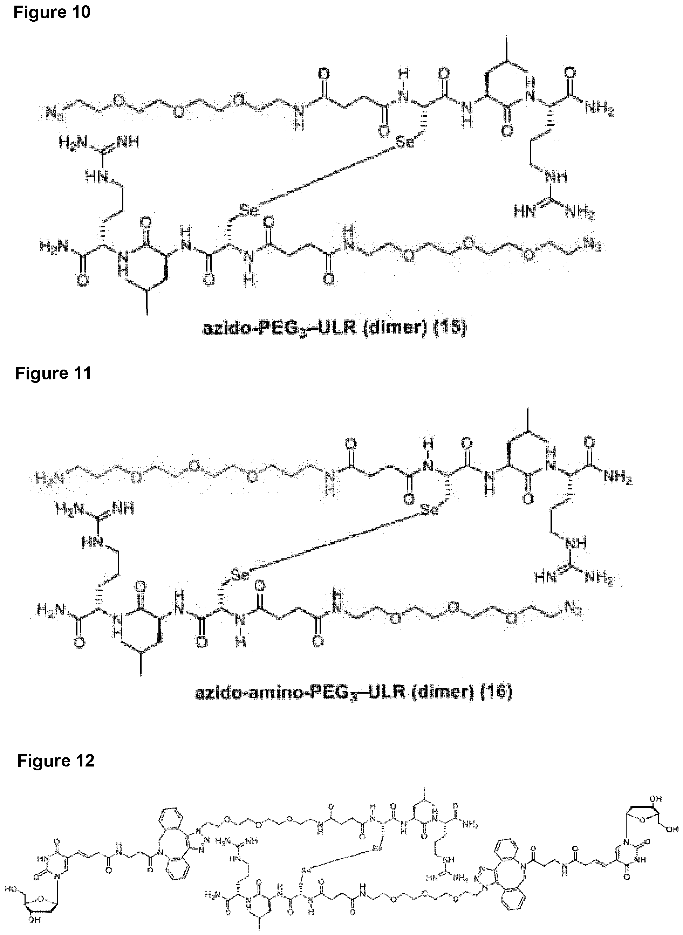

The most potent cysteine protease activator in accordance with the second aspect of the invention is shown in Figure 10 and is also referred to herein as peptide dimer azido-PEG₃-ULR.

At variance with the above-described preferred linkers, one of the tripeptide dimers in accordance with the second aspect of the invention in a further preferred embodiment may be coupled to a linker comprising a polyethylene glycol moiety and an amine moiety. One preferred example of such a linker is the 4,7,10-trioxa-1,13-tridecanediamine linker. The resulting asymmetric cysteine protease activator molecule is shown in Figure 11 and is also referred to herein as azido-amino-PEG3-ULR dimer.

The skilled person will be able to couple the cysteine protease activator molecules described herein to the linkers using methods known in the art.

In an even more preferred embodiment, the cysteine protease activator molecule is coupled via the linker to a modified nucleotide comprising DBCO.

Coupling occurs in this case via a ring-strain promoted click rection. Similarly, the above-described modified nucleotide may, for example, be a deoxynucleoside triphosphate (dNTP) which is coupled to the DBCO moiety via a polyethylene glycol (PEG) linker, such as 5-DBCO-PEG₄-dUTP. The resulting coupled cysteine protease activator molecule is shown in Figure 12 and is also referred to herein as dU-triazole-PEG₃-ULR (dimer).

Importantly, the inventors surprisingly found that the peptide dimer azido-PEG3-ULR as well as the nucleoside-peptide-hybrid dimer dU-triazole-PEG3-ULR greatly outperformed all other activators, including DTBA, SeDTBA and BMS which were presented in literature as most efficient papain activators. Even in equimolar and sub-equimolar concentrations these novel cysteine protease activators were highly efficient and allowed for a clear differentiation from the background signal.

Similarly, cysteine protease activators as defined herein comprising cysteine may also be directly coupled to modified dNTPs.

Thus, in a third aspect, the present invention relates to a cysteine protease activator molecule comprising a peptide comprising 2 to 5 amino acids in length, a cysteine moiety and a C-terminal arginine, glycine or phenylalanine or a C-terminal arginine-amide, glycine-amide or phenylalanine-amide, wherein the peptide is N-terminally coupled to a modified nucleotide having a DBCO moiety.

It is understood that the cysteine moiety comprised in the peptide comprises a free thiol residue, which allows for a specific reaction with reversibly inactivated cysteine proteases as described above, undergoing thiol/disulfide or thiol/selenylsulfide exchange reactions.

In a preferred embodiment, the peptide is N-terminally coupled to the DBCO moiety of the modified nucleotide via a linker comprising a polyethylene glycol moiety and an azide moiety.

The azide moiety of the above-described preferred embodiment of the invention corresponds to the "second reactive moiety" of the first aspect of the invention. Accordingly, and as outlined above, the cysteine protease activator molecules of the third aspect of the invention are suitable to be used in the method of the first aspect of the invention.

Preferably, the linker is O-2-Azidoethyl-O'-[2-(succinylamino)-ethyl]dimethyleneglycol.

Again, the above-described modified nucleotide comprising DBCO may, for example, be a deoxynucleoside triphosphate (dNTP) which is coupled to the DBCO moiety via a polyethylene glycol (PEG) linker, such as 5-DBCO-PEG₄-dUTP, which is commercially available.

In a fourth aspect, the present invention relates to a kit of parts for detecting a target nucleic acid in a sample comprising: (a) a reversibly inactivated cysteine protease, wherein the catalytic cysteine residue of the reversibly inactivated cysteine protease is blocked by a disulfide or selenylsulfide bond; (b) a modified nucleotide having a first reactive moiety, wherein the first reactive moiety is preferably an alkyne moiety ; (c) the cysteine protease activator molecule as defined in accordance with the first or the second aspect of the invention that is linked to a second reactive moiety, wherein preferably, the second reactive moiety is an azide moiety; (d) optionally, Tris(2-carboxyethyl)phosphine (TCEP); and (e) a cysteine protease substrate, wherein said substrate provides a detectable signal when hydrolysed by said reactivated cysteine protease.

In this specification a number of documents are cited. The disclosure of these documents, while not considered relevant for the patentability of this invention is herewith incorporated by reference in its entirety. More specifically, all referenced documents are incorporated by reference to the same extent as if each individual document was specifically and individually indicated to be incorporated by reference. The invention is herein described, by way of example only, with reference to the accompanying drawings for purposes of illustrative discussion of the preferred embodiments of the present invention. Unless otherwise defined, all technical and scientific terms used herein have the same meaning as commonly understood by one of ordinary skill in the art to which this invention belongs. In case of conflict, the patent specification including definitions, will prevail.

Regarding the embodiments characterized in this specification, in particular in the claims, it is intended that each embodiment mentioned in a dependent claim is combined with each embodiment of each claim (independent or dependent) from which said dependent claim depends. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

### The figures show:

**Fig. 1****:** Principle of a standard variation of the method of the invention. 1) Target nucleic acid of interest is amplified via PCR leading to incorporation of modified nucleotides. 2) Reactive moieties of modified and incorporated nucleotides are coupled to corresponding moieties in enzyme activator molecules. 3) Target nucleic acid with linked cysteine protease activator hybridizes with specific biotinylated DNA probes and are immobilized on streptavidin coated plates in the process. Immobilized nucleic acid with linked cysteine protease activator reactivates reversibly inhibited cysteine protease which in turn processes the substrate leading to an amplified signal.
**Fig. 2****:** Three-dimensional structure of papain (PDB: 1PPN). A) Global structure of papain with L (left) subdomain comprised of α-helices and R (right) subdomain comprised of a β-barrel like shape. The active site sits on top of the v-shaped cleft between the two subdomains. B) Amino acid residues within the active site. Cys₂₅ and His₁₅₉ form the catalytic dyad and Trp₁₇₇, Gln₁₉ as well as Asn₁₇₅ orientate and stabilize the active site.
**Fig. 3****:** Structure of the commercially purchased Z-Phe-Arg-AMC cysteine protease substrate. Hydrolysis leads to the release of AMC, which can be fluorometrically tracked.
**Fig. 4****:** Copper-free ring-strain promoted click reaction between DBCO and azide, generating two different isomers of the triazole product.
**Fig. 5****:** Reaction scheme of the reversible inhibition of papain with thiosulfonate derivatives. The nucleophilic Cys₂₅ within the papain active site attacks the thiosulfonate, forming a disulfide and releasing an alkyl sulfinic acid derivate.
**Fig. 6****:** Structure of different thiosulfonate derivates. **(1)** S-benzyl ethanethiosulfonate, **(2)** S-propyl ethanethiosulfonate, **(3)** S-(2-acetic acid) ethanethiosulfonate, **(5)** S-(2-aminoethyl) ethanethiosulfonate.
**Fig. 7****:** Reaction scheme of latent papain reactivation with thiols or selenols by thiol/disulfide or selenol/disulfide exchange reactions.
**Fig. 8****:** Reaction equations of thiol/disulfide interchange reactions. (1) Overview of both reaction steps of the reaction between a dithiol reducing agent (HS-R-SH) and a target disulfide (R'-S-S-R'). Reaction rates *k₁* (nucleophilic attack) and *k₂* (cycle formation towards oxidized disulfide form of the reducing agent) as well as the reverse reaction rates *k₋₁* and *k₋₂* are displayed for both steps.
**Fig. 9****:** Chemical structure of disulfide reducing compounds discussed in this chapter. DTBA **(9)** and SeDTBA **(11)** were synthesized in this work. DTT, 2-ME, BMS, DTBA **(9)** and SeDTBA **(11)** were utilized in papain reactivation assays in this work.
**Fig. 10****:** Structure of cysteine protease activator molecule azido-PEG₃-ULR.
**Fig. 11****:** Structure of cysteine protease activator molecule azido-amino-PEG₃-ULR.
**Fig. 12****:** Structure **of** dU-triazole-PEG₃-ULR (dimer).

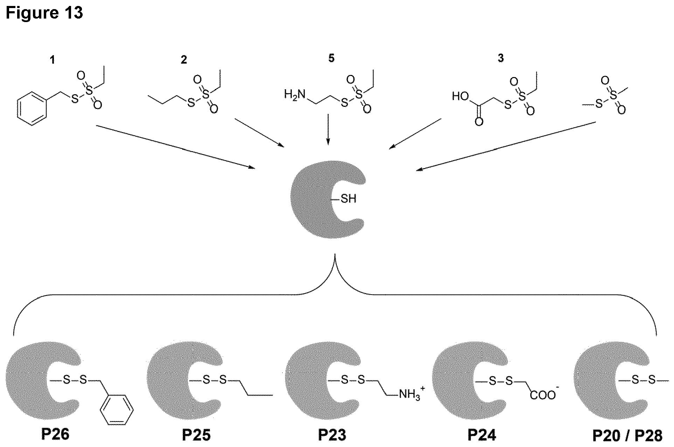

**Fig. 13****:** The four synthesized thiosulfonate derivatives 1, 2, 3 and 5 as well as methyl methanethiosulfonate were incubated with active papain to yield five different latent papain forms, which are reversibly inactivated.

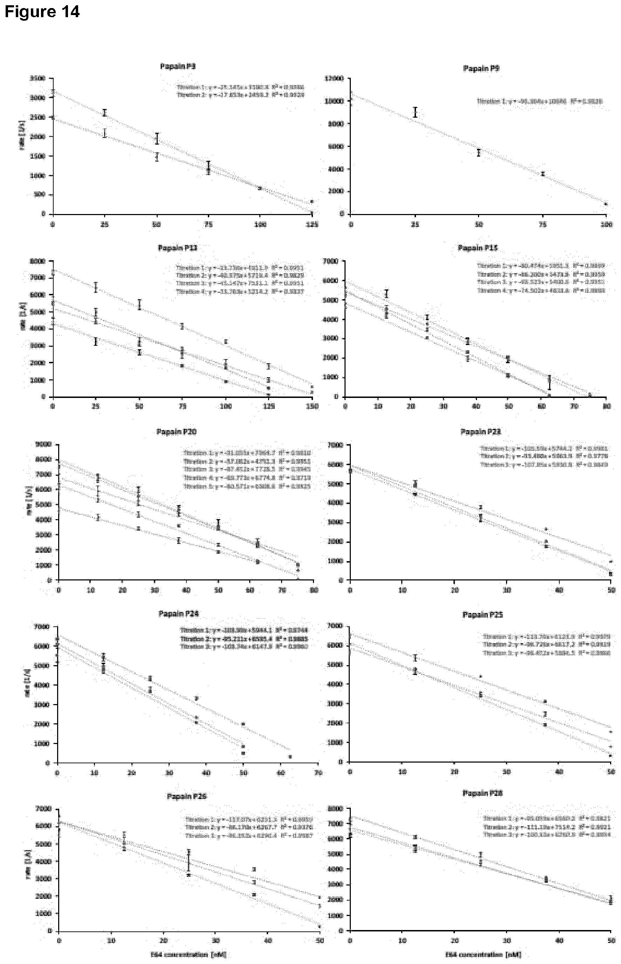

**Fig. 14****:** Resulting diagrams of the active site titration for all different latent papain batches. The slope of the linear fluorescence increase (y-axis) was plotted against the corresponding E64 concentration (x-axis) and the linear fit for all datapoints was calculated and are shown for each titration experiment individually. The function equations and coefficients of determination are given. Standard deviations were calculated from triplicate measurements in each experiment.

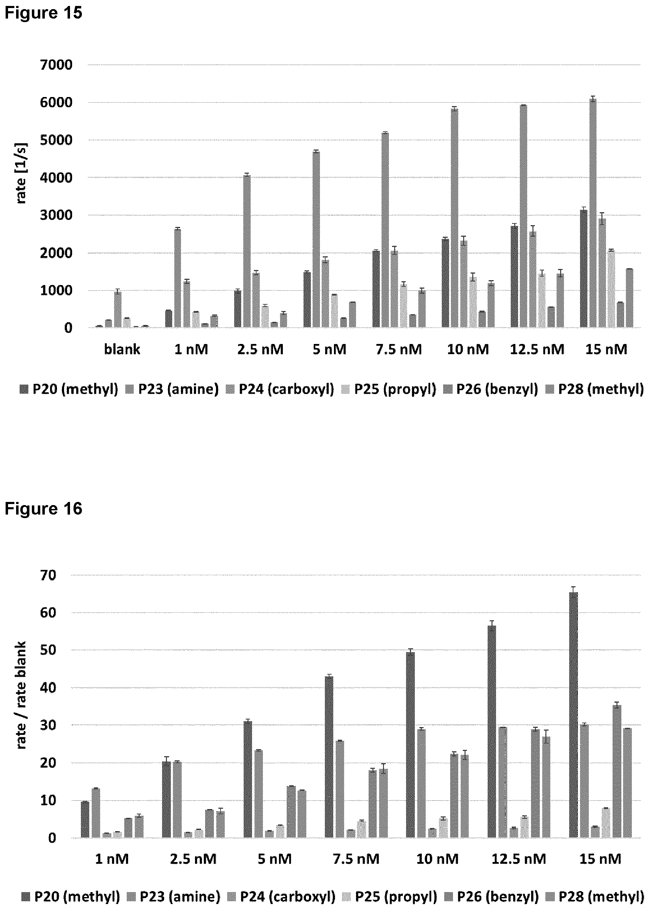

**Fig. 15****:** Reaction rates of substrate hydrolysis after the reactivation of latent papain forms P20 - P28 (all 1 nM) with a concentration series of the activator dU-triazole-PEG₃-ULR (21) from 1 nM - 15 nM. The slopes of the resulting fluorescence curves were calculated within the linear portion and compared among each other. Blank corresponds to the rate of residual papain activity under the reaction conditions without activator. Standard deviation was calculated from duplicate measurements.
**Fig. 16****:** Normalized ratio of rate/rate blank of substrate hydrolysis after the reactivation of latent papain forms P20 - P28 (all 1 nM) with a concentration series of the activator dU-triazole-PEG3-ULR **(21)** from 1 nM - 15 nM. Standard deviation was calculated from duplicate measurements.

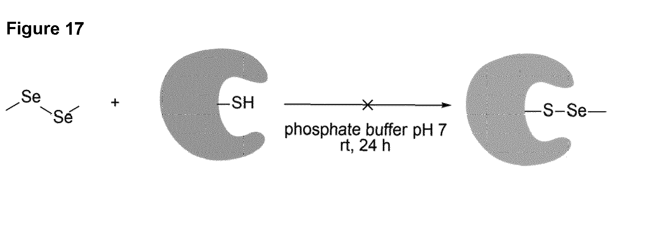

**Fig. 17****:** Scheme of an attempt to produce a latent papain form containing a selenylsulfide group. The inactivation of papain with dimethyl diselenide did not occur.

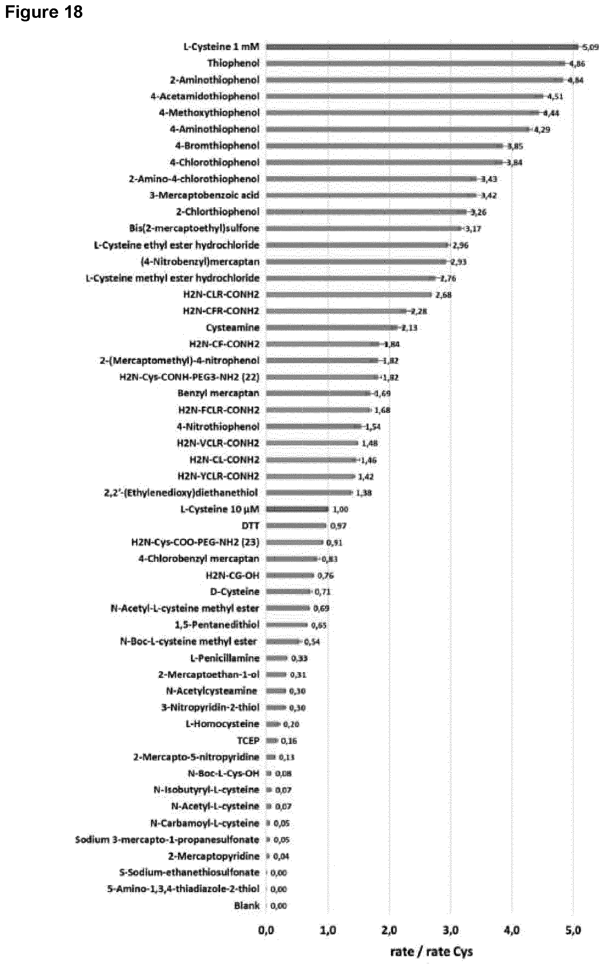

**Fig. 18****:** Normalized ratios of rate/rate 10 µM cysteine of substrate hydrolysis (2 µM) after reactivation of latent papain P3 (1 nM) with the displayed activators at a concentration of 10 µM in well buffer (0.1 mM phosphate buffer pH 7, 4 mM EDTA, 0.01% Brij35). Measurements were performed in triplicate and standard deviations are shown. Synthesized peptides are shown in green colour. The rate with 10 µM cysteine was used for normalization of rates and the rate with 1 mM cysteine was used for orientation purposes (both red). Blank represents the residual background activity of papain without addition of activator.

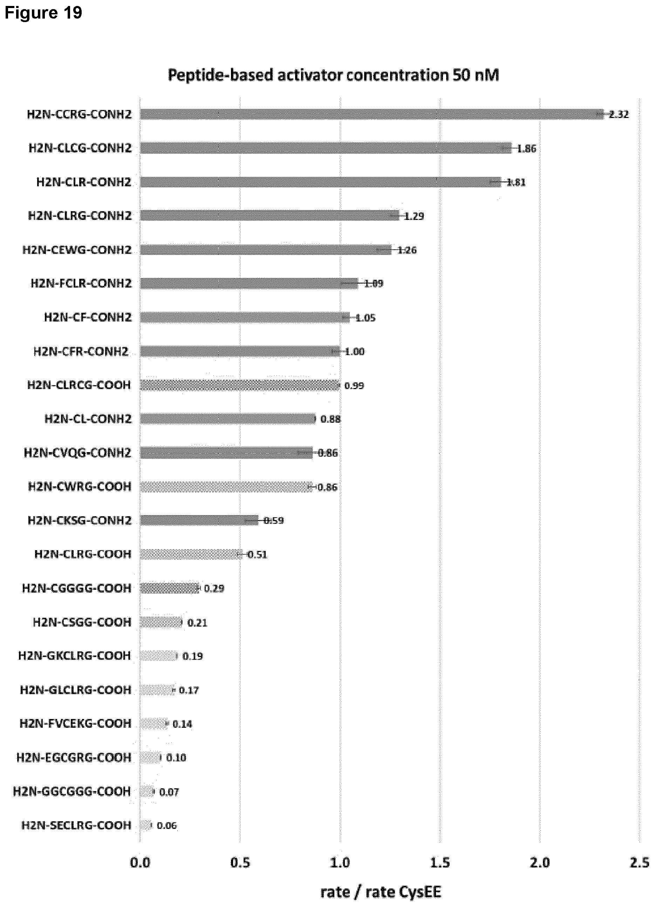

**Fig. 19****:** Normalized ratios of rate/rate cysteine ethyl ester (CysEE) of substrate hydrolysis (2 µM) after reactivation of latent papain P13 (1 nM) with the displayed peptide-based activators at a concentration of 50 nM in well buffer (0.1 mM phosphate buffer pH 7, 4 mM EDTA, 0.01% Brij35. Measurements were performed in duplicate. Different lengths of peptides are colour-coded as follows: Hexapeptides = yellow, pentapeptides = blue, tetrapeptides = green, tripeptides = orange, dipeptides = grey. Peptides with carboxyl group at the C-terminus are displayed in hatched colours.

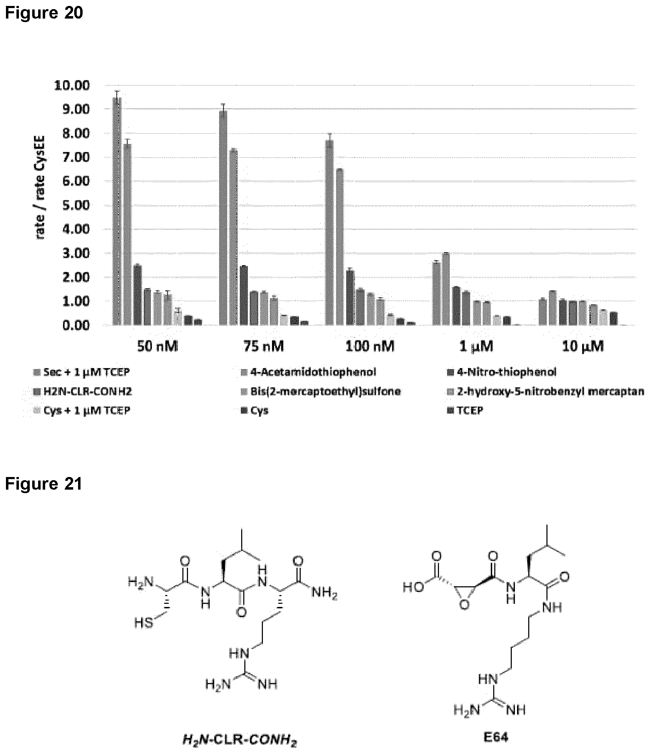

**Fig. 20****:** Normalized ratios of rate/rate cysteine ethyl ester (CysEE) of substrate hydrolysis (2 µM) after reactivation of latent papain P13 (1 nM) with the displayed activators in a concentration series of 50 nM -10 µM in well buffer (0.1 mM phosphate buffer pH 7, 4 mM EDTA, 0.01% Brij35). Measurements were performed in duplicate.
**Fig. 21****:** Structure comparison between the inhibitor E64 and the activator tripeptide *H₂N*-CLR-*CONH₂*.

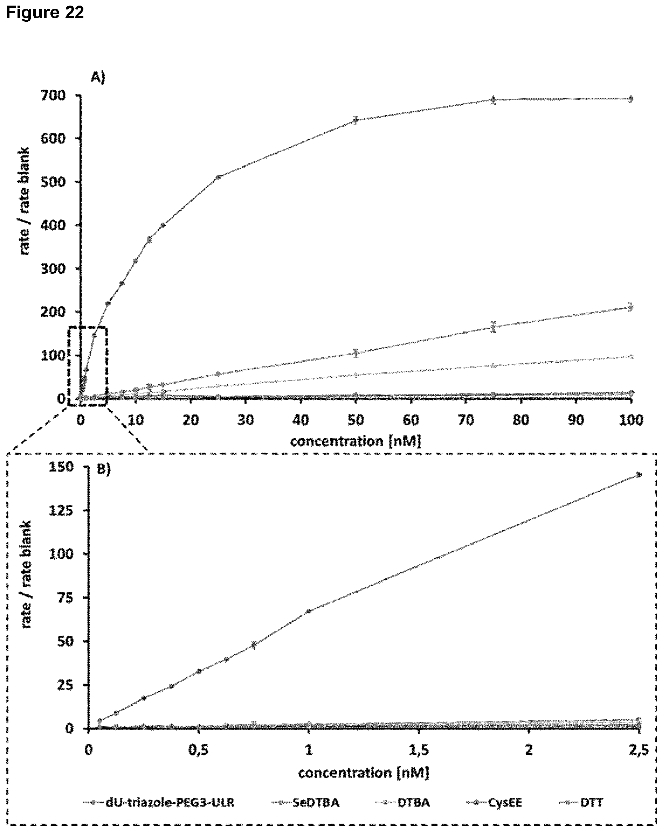

**Fig. 22****:** Normalized ratios of rate/rate blank of substrate hydrolysis (2 µM) after reactivation of latent papain (1 nM) with the displayed activators at concentrations of 50 pM - 100 nM in well buffer (0.1 mM phosphate buffer pH 7, 4 mM EDTA, 0.01% Brij35) in the presence of 1 µM TCEP. Measurements were performed in duplicate and standard deviations are shown. Blank represents the residual background activity of papain without addition of activator. Measuring points were connected for better illustration. The abbreviation "CysEE" stands for L-cysteine ethyl ester. A) Overview of reaction rates of activators in the concentration range of 50 pM - 100 nM. B) Zoom-in of the concentration range between 50 pM and 2.5 nM.

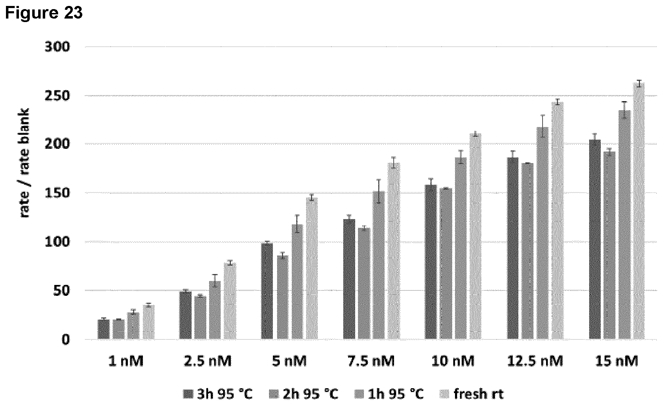

**Fig. 23****:** Normalized ratios of rate/rate blank of substrate hydrolysis (2 µM) after reactivation of latent papain P15 (1 nM) with a concentration series of the activator dU-triazole-PEG3-ULR (21) from 1 nM - 15 nM in the presence of TCEP (1 µM), EDTA (4 mM) and 0.01% Brij 35. Four differently treated aliquots of the activator were prepared: three of them were heated to 95 °C in phosphate buffer pH 7 for 1, 2 and 3 hours as well as one freshly prepared aliquot at room temperature prior to the plate preparation. Standard deviations were calculated from triplicate measurements for each sample.

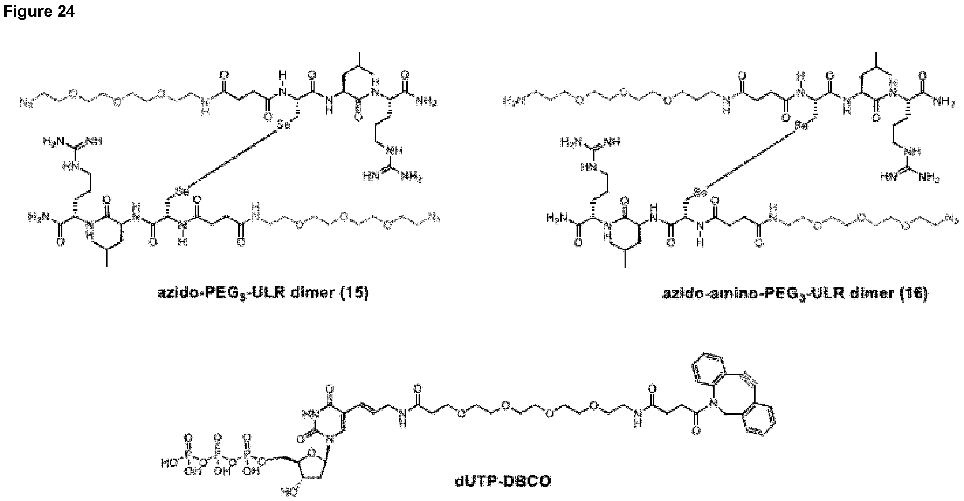

**Fig. 24****:** Structure of the utilized peptide-dimers azido-PEG3-ULR (15) and azido-amino-PEG3-ULR (16) as well as the commercially available dUTP-DBCO nucleotide derivates. The azide linker is marked in blue and the amine linker is coloured in green. The alkyne moiety, marked in red, reacts with the azide moiety to form a triazole-ring in a copper-free ring-strain promoted click reaction. Synthesis of the peptide-dimers is discussed in Example 4, 4.2.

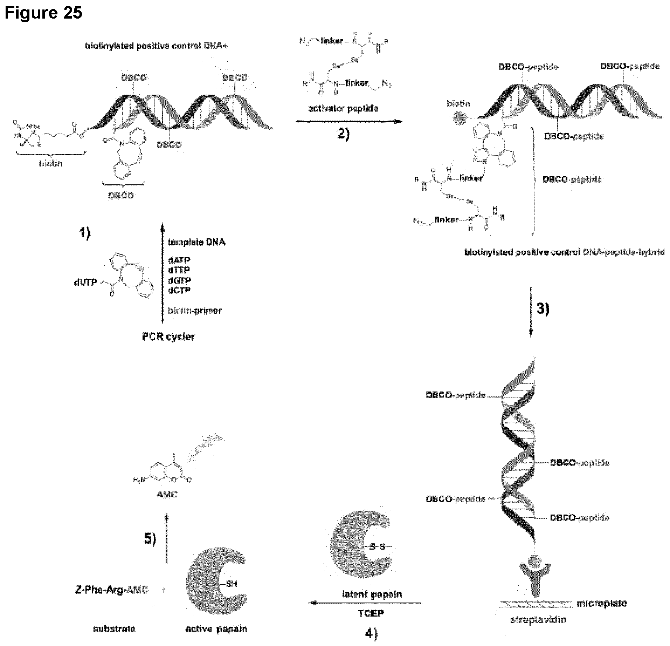

**Fig. 25****:** Overview of the process of a DNA detection assay with a biotinylated positive control DNA. 1) PCR with template DNA, standard nucleotides, biotinylated primers, Taq polymerase and modified dUTP-DBCO nucleotides produce a DNA+ amplificate which is biotinylated and contains DBCO moieties. 2) The PCR mixture is incubated with activator peptide con-taining an azide moiety for the ring-strain promoted click reaction. 3) The raw (or purified) biotinylated DNA-peptide-hybrid (DNA+ph) mixture is transferred to the streptavidin coated microplate and subsequently immobilized. 4) Latent papain, TCEP and substrate is added to the wells. 5) Activated papain processes the substrate and nascent free AMC is detected via a fluorometer.

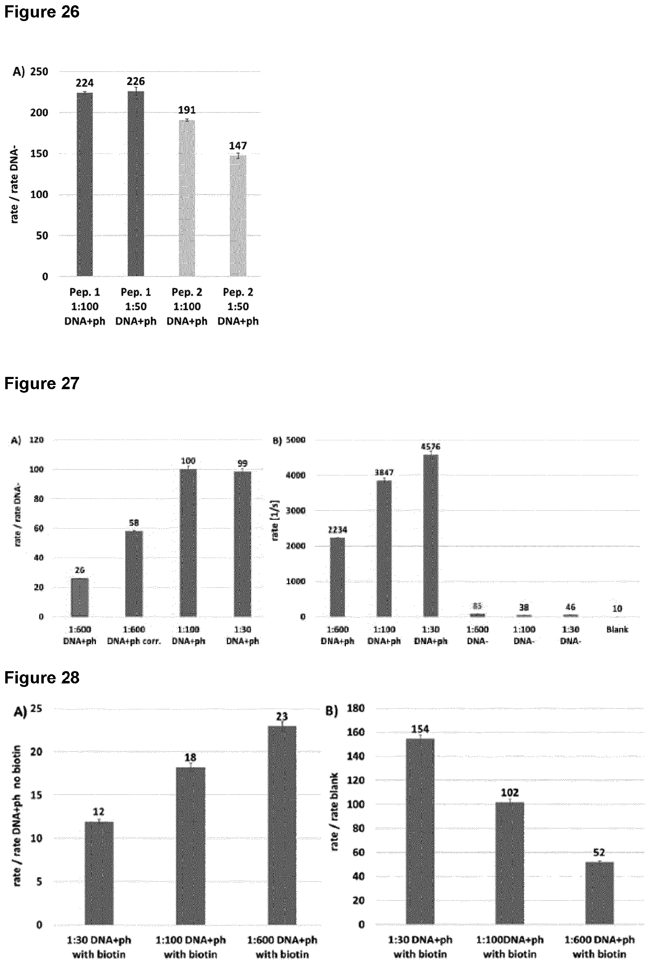

**Fig. 26****:** Latent papain P20 (1 nM) reactivation assay with immobilized DNA-peptide-hybrid (DNA+ph) in well buffer (0.1 mM phosphate buffer pH 7, 4 mM EDTA, 0.01% Brij35) in the presence of 1 µM TCEP and 2 µM substrate. A) Normalized ratios of rate positive control (DNA+ph)/rate negative control (DNA-). Measurements were performed in triplicate and standard devia-tions are shown. The utilized peptides in this assay were azido PEG3-ULR (15) (Pep. 1) and azido amino-PEG3-ULR (16) (Pep. 2). 1:50 and 1:100 refers to the dilution factor of DNA solution that was added to each well after purification.
**Fig. 27****:** Latent papain P20 (1 nM) reactivation assay with immobilized DNA-peptide-hybrid in well buffer (0.1 mM phosphate buffer pH 7, 4 mM EDTA, 0.01% Brij35) in the presence of 1 µM TCEP and 2 µM substrate. The utilized peptide in this assay was azido amino-PEG3-ULR (16). A) Normalized rates of positive control (DNA+ph) against rate of negative control (DNA-). Measurements were performed in triplicate and standard deviations are shown. 1:600, 1:100 and 1:30 refers to the dilution factor of the reaction mixture that was added to each well after the click reaction. The rate of 1:600 DNA- in B (marked in red) shows unnatural high values compared to the higher concentrated negative controls and thus the corresponding normalized rate of 1:600 DNA+ in A (marked in red) is very high. In order to supply a useful estimation, an additional corrected (corr.) rate is supplied by division of the rate of 1:600 DNA+ph through the rate of 1:100 DNA- instead.
**Fig. 28****:** Latent papain P20 (1 nM) reactivation assay with T7-Spike full DNA-peptide-hybrid in well buffer (0.1 mM phosphate buffer pH 7, 4 mM EDTA, 0.01% Brij35) in the presence of 1 µM TCEP and 2 µM substrate. A) Normalized ratios of rate biotinylated positive control (DNA+ with biotin)/rate non-biotinylated positive control (DNA+ no biotin). B) Normalized rates of biotinylated positive control (DNA+)/rate of residual active papain (blank). Measurements were performed in triplicate and standard deviations are shown. The utilized peptide in this assay was azido-amino-PEG3-ULR **(16).** 1:30, 1:100 and 1:600 refers to the dilution factor of DNA solution that was added to each well after the click reaction and subsequent purification.
**Fig. 29****:** Overview of the process of the method of the invention utilizing a biotinylated DNA probe for hybridization to target DNA. 1) PCR with template DNA and modified nucleotide dUTP-DBCO produce a DNA+ amplificate containing DBCO moieties. 2) The PCR mixture is incubated with activator peptide containing an azide moiety for the ring-strain promoted click reaction. 3) The DNA-peptide-hybrid mixture is incubated with a biotinylated DNA probe and hybridized through heating. 4) The reaction mixture is transferred to the streptavidin coated microplate and immobilized while heating. 5) Latent papain, TCEP and substrate are added to the wells. 6) Activated papain processes the substrate and nascent free AMC is detected via fluorescence.

The examples illustrate the invention

### Example 1: Preliminary experiments of the papain reactivation assay

All following described fluorometric assays (Examples 1 - 3) are based on the reaction of reversibly inactivated papain (latent papain) with an activator containing either sulfur or selenium (with the exception of TCEP). Different latent papain forms, buffer compositions and activators in various concentrations were compared. Papain was hereby transferred in buffer (standard well buffer consisted of 0.1 M phosphate pH 7,4 mM EDTA, 0.01 % Brij35 and if activators contained diselenides, 1 µM TCEP) mixed with activator and substrate inside 96 well plates (without prior washing of the plates). After reactivation, active papain hydrolyses the substrate Z-Phe-Arg-AMC (2 µM) and the resulting fluorescence was tracked every minute for 2 h. The slopes of the corresponding curves were calculated for the linear portion within the first 30 minutes and the resulting reaction rates were directly compared. Resulting reaction rates directly reflect reactivation efficiencies of each activator under each reaction condition. While the extent of this work is in no terms a kinetic study of the detailed disulfide exchange reactions (like that of Shaked et al.) of the mixed disulfide group within the active site of latent papain, the comparison of the reaction rates reveals different extents of papain reactivation under the closely defined assay conditions. After addition of activator a fraction of latent papain is immediately reactivated in rather non-stoichiometric proportions. Complete reactivation can generally only be achieved with great excess of activator. The partial reactivation with smaller activator amounts appears almost instantaneously because the resulting reaction curve show linear progression from the start except for very low activator concentrations. After reactivation, all different latent papain forms result in the same active enzyme. If different latent papain forms show different reaction rates in identical activation assays, the efficiency of reactivation with the applied activator can be deduced. The same applies for reactivation of one latent papain form with different activators or reaction conditions. The purpose of these assays was the identification of a sensitive pair of latent papain and activator, which, under the defined reaction conditions, demonstrate a high reactivation efficiency resulting in high reaction rates of substrate hydrolysis with low residual activity of papain.

### 1.1 Preparation of latent papain forms and comparison of reactivation efficiencies

In order to reversibly deactivate commercially purchased papain, reactions with different thiosulfonate derivatives were carried out and different latent papain versions were produced. Papain was first incubated with cysteine to yield complete activated papain which could then be incubated and deactivated with the specific thiosulfonate solutions. A mixture of papain disulfides is otherwise to be expected. An additional step was introduced in this work with the goal of further reduction of residual enzymatic activity. After incubation with either thiosulfonate derivative, papain was thus incubated with equimolar amounts of the more efficient irreversible inhibitor E64 (see Example 5, 5.2.1.2) in order to deactivate possible unmodified papain traces. The excess of small molecules was then separated by Sephadex G25 spin columns. Different thiosulfonates were synthesized (data not shown) and used to produce different latent papain forms, to the authors best knowledge yet unknown to literature (P23, P24 and P26), with their reactivation efficiency directly compared. The four thiosulfonates S-benzyl **(1)-,** S-propyl **(2)-,** S-(2-acetic acid) **(3)-** and S-(2-aminoethyl) **(5)**-ethanethiosulfonate are shown in Figures 6 and 13.

All latent papain forms were synthesized as described (see Example 5, 5.2.1.2) and concentrations of the stock solutions were determined (see Example 5, 5.2.1.3) and are shown in Figure 14. In favor of allowing for precise comparison of all different papain forms, papain P20 - 28 were produced and processed simultaneously, whereas production of P28 differed in lack of pre-activation with cysteine and incubation with E64 in order to examine if those two steps are indeed affecting the latent papain quality (P20 vs. P28).

The successfully produced latent papain forms were then utilized in a reactivation assay in accordance with the method of the invention (see Example 5, 5.2.2.2), fluorometric method 1) with the selenocysteine containing activator dU-triazole-PEG₃-ULR **(21)** in concentrations from 1 nM - 15 nM. The resulting reaction rates of every papain and every activator concentration are directly compared in Figure 15.

At first glance, the results show that papain P23, containing the positively charged amino group, was found to be by far the most efficiently reactivated latent papain form. Papain P24 with its negatively charged residue showed reaction rates close to Papain P20 with the methyl residue, whereas at lower activator concentration P24 reaction rates were higher and with activator concentrations of > 7.5 nM the trend reverts. With rising activator concentration, P20 showed exceedingly higher rates when compared to the propyl variant P25 and the methyl variant P28 which was prepared without cysteine pre-activation and incubation with E64. The papain variant P26 with its benzyl residue showed greatly reduced rates when compared to all other variants. On second look, it becomes clear that the calculated rates have to be considered in relation to the residual papain activity. Without activator present, the rates of papain differ greatly between the latent derivatives. This must be due solely to their chemical properties and not to a result of remaining active papain after incubation with thiosulfonate, because subsequent incubation with equimolar amounts of E64 inhibitor will inevitably deactivate remaining active papain irreversibly (see Example 5, 5.2.1.2). In order to normalize the presented reaction rates, they were divided through the reaction rate of the corresponding residual activity (blank value) and results are shown in Figure 16.

The normalized reaction rates for papain P20 now appeared the highest of all papain forms, making it the most applicable candidate for reactivation. With a residual activity rate of 25% compared to P23, the normalized rates resulted in higher values even when the unnormalized reaction rates of P23 were more than twice as high compared to P20. In activator ranges below 2.5 nM however, the values of P23 were slightly higher than those of P20 and further experiments with lower activator concentration series would be of interest. The introduced positively charged amine affects the reactivation efficiency of papain greatly. This could be due to the nature of the amine itself and its capability to produce hydrogen bonds or it could solely be due to the positive charge. Synthesis of a different amine residue seems feasible and could probably result in less residual activity while retaining high reactivation efficiencies. The negative charge in the active site apparently resulted in very high residual activity and therefore featured low normalized rates as seen for P24. This effect could also be observed with different peptide activators that showed a negatively charged activator near the active site to be suboptimal (see Example 2, 2.2). On the basis of its small residual activity, papain P26 showed the third highest normalized rate values. The aromatic structure seems to be stabilized within the enzyme reducing the reactivation efficiency. The area around the active site is reported to be hydrophobic, with a tryptophan residue shielding the catalytic dyad, which could possibly interact with the benzyl residue of the latent papain form. An interesting latent form could thus be a combination of P26 and P23 with a positively charged aromatic residue at the active site. The second aliphatic residue of P25 showed surprisingly low reactivation potential towards the activator.

Noteworthy is also the difference of P20 (pre-activation + E64) and P28 (no pre-activation, no E64) and the fact that P20 reaches normalized rates twice as high as P28 although the residual activity of both were similar, with P28 even showing slightly higher rates. This proposes that the described difference of latent papain preparation indeed affects latent papain quality. The lack of pre-activation of P28 is supposed to result in two mixed disulfide forms of papain, one with cysteine and one with methyl residue in unknown proportions. Latent papain with cysteine residue thus appears to be less readily reactivated with the deployed activator. Previous experiments (not shown) underlined this theory where the described latent papain forms barely showed any difference in reaction rates after preparation without pre-activation.

An unsuccessful attempt was made in producing a papain form with a selenylsulfide group instead of a disulfide. Bachrach et al., 2004, published a computational study showing that nucleophilic attack at selenium is both kinetically and thermodynamically more favorable than at sulfur. Steinmann et al., 2010, published a comparative study investigating selenium and sulfur in exchange reactions. They pointed out that while the general rate constant for selenolate and thiolate as the leaving group at physiological pH are similar, the microenvironment of the active site can greatly influence that rate by protonation of the leaving group. Deprotonated cysteine is a worse leaving group in a hydrophobic active site but when protonated by the microenvironment, the difference compared to Sec diminishes greatly (Eckenroth et al., 2007). The reaction rate of different nucleophiles with selenium as the electrophile was proven to be up to four or five orders of magnitude greater than those of sulfur and when acting as a nucleophile, the reaction rate of selenium can increase two or three orders of magnitude compared to sulfur (Steinmann et al., 2010). These findings suggest that a selenylsulfide variant of the latent papain would allow for a greatly increased reactivation rate, even more so when coupled with an activator containing selenocysteine instead of cysteine (see Example 2.2). The difficulty lays in the production of such a papain form and has yet to be overcome. The attempt (see Figure 17) of incubating activated papain with dimethyl diselenide in a reaction buffer analogue to the thiosulfonate counterpart (see Example 5, 5.2.1.2, papain inactivation method 3) failed, as papain remained in its active form (results not shown).

Thus, a different strategy appears to be required. As recently described by Mampuys et al., 2020, selenosulfonates, the less explored analogues of thiosulfonates, can be synthesized and readily undergo electrophilic or free-radical addition to unsaturated molecules, but reactions with nucleophiles have not been studied yet. Nevertheless, these analogues might also be used in accordance with the method of the invention. Furthermore, substitution of hydrogen with fluorine in the papain forms with aliphatic residues could lead to similar results as reported by Shaked et al., 1980, and this property could be used to produce even more potent papain forms that can be used in accordance with the method of the invention.

### 1.2 Active site titration of papain

Barret et al., 1980, proved the substrate Z-Phe-Arg-AMC to be very sensitive to the papain-like cysteine protease cathepsin B, outperforming all previous substrates and Ménard et al., 1990, showed that the substrate is also very specific to papain with following kinetic parameters: kcat value of 52 s⁻¹, Km 0.42 mM. Barret et al. presented an improved active site titration protocol for papain which made use of this new substrate (Olson, 2016; Barrett et al., 1981). The protocol was further improved as described herein in Example 5, 5.2.1.3. Papain and its inhibitor E64 could be decreased to low nanomolar concentrations and still show stoichiometric inhibition. After incubation of papain with different concentrations of E64, the concentration series was transferred to the 96 well plate and substrate was added. The fluorescence increase was tracked for each concentration and the slope of the linear section of the curve was calculated. Plotting the slope for each concentration against the E64 concentration ideally produces a linear fit which intercepts the x-axis to produce the specific inhibitor concentration for complete papain deactivation. Over 30 batches of latent papain were produced of which ten are presented herein. The results for each papain with every individual titration experiment are shown in Figure 14.

Since the inhibition is presumed to be equimolar between papain and E64, the papain concentration can be calculated from the x-axis intercept with consideration of the initial dilution factor and the dilution factors of all assay steps. The concentration of each different papain batch was determined in varying titration experiments and results are shown in **Table 3.** The observed concentrations were then compared to the expected concentrations which were calculated with the NanoDrop readings and the molecular weight of papain (MW 23 406 Da). A factor of calculated/observed concentration is presented. Different latent papain forms were produced (see Example 1, 1.1) and the specific latent papain forms with each disulfide derivative are annotated.

**Table 3: Results of all determined papain concentrations. Every latent papain batch (and specific latent papain disulfide form Example 1, 1.1) is shown in the first column.**

| **Papain** | **Inactivation method** | **Assay #** | **Titration method** | **Observed concentration** | **Average concentration** | **NanoDrop concentration** | | **[c] calc. / [c] obs.** |
|---|---|---|---|---|---|---|---|---|
| **MW 23 406 Da** | | | | **[µM]** | **[µM]** | **[mg/mL]** | **[µM]** | **factor** |
| **P3 (-S-S-CH₃)** | **2** | **#1** | **3** | **3,16** | **3.32 ± 0.16** | **0.60** | **25,51** | **7,68** |
| | | **#2** | | **3,48** | | | | |
| **P9 (-S-S-CH₃)** | **3** | **#1** | **3** | **3.31** | **3,31** | **0,49** | **20,93** | **6,32** |
| **P13** | **3** | **#1** | **3** | **7.67** | **8.03 ± 0 33** | **0,55** | **23,50** | **293** |
| | | | | | | | | |
| | | **#3** | | **8,34** | | | | |
| | | **#4** | | **7,72** | | | | |
| **P15 (-S-S-CH₃)** | **1** | **#1** | **2** | **7.76** | **7.67 ± 0.31** | **0,48** | **20,40** | **2,66** |
| | | **#2** | | **7,30** | | | | |
| | | **#3** | | **8,16** | | | | |
| | | **#1** | | **7.46** | | | | |
| **P20 (-S-S-CH₃)** | **1** | **#1** | **1** | **4,37** | **4.33 ± 0.09** | **0,57** | **24,14** | **5,58** |
| | | **#2** | | **4,16** | | | | |
| | | **#3** | | **4,42** | | | | |
| | | **#4** | | **4,37** | | | | |
| | | **#5** | | **4,31** | | | | |
| **P23 (-S-S-(CH₂)₂-NH₂)** | **1** | **#1** | **1** | **2,18** | **2,41 ± 0.22** | **0.46** | **19,55** | **8,16** |
| | | **#2** | | **2,71** | | | | |
| | | **#3** | **4** | **2,34** | | | | |
| **P24 (-S-S-CH₂-COOH)** | **1** | **#1** | **1** | **2,29** | **2.58 ± 0.27** | **0,44** | **18,80** | **7,28** |
| | | **#2** | | **2,94** | | | | |
| | | **#3** | **4** | **2,52** | | | | |
| **P25 (-S-S-(CH₂)₂-CH₃)** | **1** | **#1** | **1** | **2,15** | **2.55 ± 0.31** | **0,44** | **18.80** | **7,37** |
| | | **#2** | | **2,91** | | | | |
| | | **#3** | **4** | **2,59** | | | | |
| **P26 (S-S-CH₂-C₆H₅)** | **1** | **#1** | **1** | **2,13** | **2.66 ± 0.40** | **0,46** | **19,65** | **7,39** |
| | | **#2** | | **3.09** | | | | |
| | | **#3** | **4** | **2,76** | | | | |
| **P28 (-S-S-CH₃)** | **2** | **#1** | **1** | **5,52** | **5.43 ± 0.06** | **0.56** | **23,93** | **4,40** |
| | | **#2** | | **5,41** | | | | |
| | | **#3** | **4** | **5,38** | | | | |

Each papain was inactivated with one of three specific methods (see Example 5, 5.2.1.2). Four different titration methods (see Example 5, 5.2.1.3) were performed in varying titration experiments (assay #). The ratio of the calculated concentration via NanoDrop ([c] calc.) and the observed concentration ([c] obs.) is listed for every papain batch. The NanoDrop absorption ratios of A260/A280 for all papain stock solutions ranged from 0.54 - 0.57 (not shown).

The results in Figure 14 show a clear linear correlation between the hydrolysis rate of papain substrate and the increasing concentration of the inhibitor. This shows that the papain inhibition with E64 is still near stoichiometric, even in concentrations as low as 12.5 - 100 nM. This not only saves synthesized latent papain but also cuts down on usage of the expensive inhibitor. As can be seen, the standard deviation of the triplicates is well within the accepted range and individual titration experiments, even if different methods and pre dilutions were applied, produce very similar results. Both Hanada et al. and Barret et al. reported a discrepancy between calculated and observed papain of roughly factor 2. While the results show a similar factor in cases of papain P13 and P15, other papain batches showed values as high as 8. This could be explained by the fact that both research groups worked solely with freshly prepared active papain solutions. All shown papain batches in **Table 3** are however latent papain forms which were previously prepared from a fresh solution in an elaborate procedure which includes long incubation times as well as physical stress for the enzyme (see Example 5, 5.2.1.2).

### Example 2: Papain reactivation with different activators

### 2.1 Thiol-disulfide exchange reactions between latent papain and thiol activators

Several thiol compounds and cysteine containing peptide activators (see Example 4.1) were compared for their potential to reactivate latent papain. To this end, the thiol compounds and cysteine containing peptide activators were utilized in reactivation assays (see Example 5, 5.2.2.2, fluorometric method 2) of papain P13 (see Example 1, 1.2) with an activator concentration of 10 µM. For thiols not completely soluble in phosphate buffer pH 7 at high concentrations, the 1 mM stock solution was prepared in DMSO, resulting in 1% DMSO in the reaction mixture. In order to normalize the observed reaction rates between assays, a 10 µM cysteine sample was additionally measured in every assay and reaction rates of the examined thiols were divided through the reaction rates produced by the 10 µM cysteine standard. Additionally, a measurement with 1 mM cysteine was performed in order to achieve maximal papain activation and therefore the maximal reaction rate within the confines of the assay. The resulting normalized rates are compared in Figure 18.

The comparison revealed a difference in activator efficiencies. Some of the tested thiol compounds barely showed any increase in normalized rates compared to the residual papain activity (blank). With L-cysteine being the reference, all values below 1.0 correspond to slower reaction rates. Especially noteworthy is the fact, that activation with all cysteine derivatives modified on the N-terminus, like N-Boc-L-cysteine and N-acetyl-L-cysteine, show strongly decreased rates of less than 10% compared to L-cysteine. Addition of methyl and insertion of methylene groups at the cysteine sidechain, as is the case of L-homocysteine and L-penicillamine, results in lower reaction rates as well. The enantiomeric D-cysteine is also less efficient and produces rates of only 70% compared to L-cysteine. On the other hand, cysteine with C-terminal esterification and free amino group resulted in normalized rates almost three times higher compared to L-cysteine. All synthesized cysteine containing peptides produced higher reaction rates compared to free L-cysteine, except for *H₂N*-Cys-COO-PEG-*NH₂* which produced rates 10% lower compared and was therefore only half as efficient compared to the similar but longer peptide *H₂N*-Cys-*CONH2-*PEG₃-*NH₂* (0.91 vs. 1.82). The most potent peptide activators were the tripeptides *H₂N*-CLR-*CONH₂* and *H₂N*-CFR-*CONH₂* which achieved rates more than twice as high when com-pared to L-cysteine.

Reactivation of papain with L-cysteine and DTT resulted in similar rates whereas 2-ME exhibited only 70% of these values. In the case of 4-chlorobenzyl mercaptan, the addition of the electron-withdrawing chloride to benzyl mercaptan cut the rate in half (0.83 vs. 1.69). The dithiol bis(2-mercaptoethyl) sulfone proved to be a potent papain activator and produced rates slightly higher than L-cysteine ethyl ester with a normalized rate of 3.17. More effective reduction was only achieved by various derivatives of thiophenol with thiophenol itself reaching the highest rate of all tested activators (4.86). Since thiophenols are more acidic compared to most other thiols, the results hint to the fact that the pKa value of activators is indeed a driving factor of the reaction rate (*k1,* see **Figure 8** equation 1) of papain reactivation as previously described (Singh and Whitesides, 1993). In order to get a clear overview of the acidity of tested thiols, pKa values of selected thiols are summarized in **Table 4.**

**Table 4: List of pKa values of selected utilized thiols. Published values were obtained from: (a) Danehy and Parameswaran, 1968, (b) Bordwell and Hughes, 1982, (c) Singh et al., 1991, (d) Lamoureux et al., 1993. Values (e) were predicted from https://www.chemicalbook.com/ and values (f) were predicted from https://go.drugbank.com/. Values were rounded to one decimal place.**

| **Thiol compound:** | **pKₐ(H₂O)** | **Thiol compound:** | **pKₐ(H₂O)** |
|---|---|---|---|
| 4-Nltrothiophenol | 4.7^{b} | L-Penidliamine | 7.9^{a} |
| 4-Chlorothiophenol | 5.9^{a} | Bis(2-mercaptoethyl)sulfone | 7.9, 9.0^{d} |
| 4-Bramothiophenol | 6.0^{b} | Cysteamine | 8.2^{a} |
| 3-Mercaptobenzoic acid | 6.3^{a} | *D*/*L*-Cysteine | 8.3^{a} |
| *L*-Cysteine methyl ester hydrochloride | 6.5^{a} | L-Homocysteine | 8.7^{a} |
| Thiophenol | 6.5^{a} | 3-Nitropyridine-2-thiol | 8.8^{e} |
| *L*-Cysteine ethyl ester hydrochloride | 6.5^{a} | *N*-Acetyl-*L*-cysteine methyl ester | 9.1^{e} |
| 2-Aminothiophenol | 6.6^{a} | DTT | 9.2, 10.1^{c} |
| 4-Aminothiophenol | 6.6^{a} | *N*-Boc-*L*-cysteine methyl ester | 9.3^{e} |
| 4-Acetamidothiophenol | 6-7^{e} | 4-Chlorobenzyl mercaptan | 9.3^{e} |
| 4-Methoxythiophenol | 6.8^{b} | 2,2'-(Ethylenedioxy)diethanethiol | 9.3^{e} |
| 2-Chlorothiophenol | 6.9^{a} | Benzylmercaptan | 9.4^{b} |
| 2-Mercapto-5-nitropyhridine | 7.1^{e} | *N*-Acetyl-*L*-cysteine | 9.5^{a} |
| 2-Amino-4-chlorothiophenol | 7.4^{e} | 2-Mercaptoethan-1-ol | 9.6^{c} |
| 2-Mercaptopyridine | 7.6^{f} | *N*-Acetylcysteamine | 9.9^{e} |
| 5-Amino-1,3,4-thiaodiazole-2-thiol | 7.8^{e} | 1,5-Pentanedithiol | 10.1^{e} |

As described by Shaked et al. the protonation of the amino group in *L*-cysteine ethyl ester strongly affects the pKa of its thiol, an effect that does not occur to this extent in natural *L*-cysteine (Shaked et al., 1980). This leads to a reduction of the pKa of *L*-cysteine ethyl ester from 8.3 to 6.5 which can explain the increase in observed rates. The addition of a Boc-protection group to the amine of *L*-cysteine ethyl ester leads to an increase of the pKa from 6.5 to 9.3 and similar results could be observed when *L-*cysteine is acetylated which increases the pKa from 8.3 to 9.5. These differences in pKa values explain why *N*-terminal modified versions of *L*-cysteine reveal much lower rates compared to *C-*terminal modifications as described above. This does however not explain the strong decrease in rates observed for the cysteine derivates *L*-homocysteine and *L*-penicillamine which provide pKa values close to that of *L*-cysteine with *L*-penicillamine even slightly lower. These inconsistencies might possibly be due to steric factors of the larger side chains of the cysteine derivatives. Furthermore, the pKa value of both cysteine enantiomers should be identical but the resulting rates differ by 30% in favour of *L*-cysteine. In addition, the pKa of 2-mercapto-5-nitropyridine is 7.1 and should therefore be optimal for papain reactivation at pH 7 due to optimal balance between acidity and nucleophilicity of the corresponding thiolate, as described previously and explained by Shaked et al. and Singh et al., but this activator only produced rates less than 15% of the rates of *L*-cysteine (Shaked et al., 1980; Singh et al., 1995). The dithiol bis(2-mercaptoethyl) sulfone (BMS) on the other hand produced the highest rates of all tested thiols aside from 3-mercaptobenzoic acid and thiophenol derivatives. This result did again not correlate to the pKa hypothesis because with a pKa of 7.9 it is close to *L*-cysteine and above the pKa of *L*-cysteine ethyl ester, 5-amino-1,3,4-thi-adiazole-2-thiol (which show almost no papain activation potential at all) and identical to *L*-penicillamine.

All thiophenol derivatives showed high potential for papain reactivation. One reason could be the low pKa value of all derivatives but since thiophenol and *L*-cysteine ethyl ester have identical pKa values and thiophenol outperforms the cysteine derivative, other effects such as electrostatic interactions and structural properties might also affect the reactivation mechanism as well. Since the area around the active site is reported to be hydrophobic, with a tryptophan residue shielding the catalytic dyad 64, strong hydrophobic interactions between the thiophenol derivatives and the surroundings of Cys25-S-S-CH₃ in the active site could be a further reason for the effectiveness of this activator family.

The pKa values of the synthesized peptides could not be determined. The difference in reactivation potential between peptides might be better explained by amino acid preferences and interactions between papain subsites.

In summary, two general activator families proved to be potent papain activators: highest rates were achieved after activation with thiophenol derivatives, and second highest rates were achieved with synthesized peptides containing cysteine. Additional peptides with varying sequences and lengths were synthesized and screened (see Example 2, 2.2) and the incorporation of selenocysteine produced even more potent activators compared to thiophenols (see Example 2, 2.3.3). It was observed that the chosen concentration of 10 µM in this experiment was suboptimal. While perfectly applicable for less potent activators, more effective activators in the range of *L*-cysteine ethyl ester and above cannot be perfectly differentiated because with potent activators at high concentrations, the reactions proceed too fast, leading to large and unavoidable calculation errors of the initial rate.

With potent activators in higher concentrations, the activation process might not show a linear correlation. Therefore, all following experiments were conducted in concentration series with a maximal concentration of 10 µM. A particularly good example for this effect is the comparison of rates between the peptide *H₂N*-CLR-*CONH₂* and *L*-cysteine ethyl ester. Both compounds produced similar reaction rates at a concentration of 10 µM in this experiment but the peptide outperformed *L*-cysteine ethyl ester by factor 1.8 at a concentration of 50 nM in a following experiment (see Example 2, 2.2 and Figure 19). Nonetheless, the results of this experiment allow for a broad outline of activator efficiencies.

### 2.2 Screening of peptide-based papain activators

Since the most potent papain inhibitors are peptide-based variants and the natural substrates are proteins or peptides, it was of interest to investigate if this substance class could also provide a useful and efficient tool for papain reactivation that can compete with other thiol containing activators previously tested. Peptide activators feature additional advantages like the non-toxicity and non-odorous nature compared to many other applicable thiol compounds as well as better water solubility.

Since there is no data about peptide-based activators used to reactivate latent papain and no published crystal structures of any of the described latent papain forms (see Example 1, 1.1) are available, virtual calculations and predictions of efficient amino acid sequences for an efficient peptide-based activator are hampered. Thus, a set of cysteine containing peptides was synthesized comprising different lengths and randomly chosen sequences to cover a broad variety of different amino acids in order to evaluate this newly developed activator class and to find efficient activator sequences for cysteine proteases to be used in accordance with the method of the invention. Preceding experiments (not shown) showed activators with C-terminal cysteine to be slightly more potent compared to activators with internal cysteine, so the synthesis strategy was adjusted accordingly.

Furthermore, the synthesis and purification of dipeptides and tripeptides was rather unfeasible, so the main focus was shifted towards tetra- and hexapeptides. Solid-phase peptide synthesis was utilized as described in Example 4, 4.1, and a total of 22 peptides (see **Table 5,** with all *L*-amino acids) were compared under identical assay conditions (see Figure 19). This was realized in individual assays with five different peptides and the known activator *L*-cysteine ethyl ester (approximately 2.25 times more potent than *L*-cysteine, see Figure 20) on one 96 well plate. *L*-Cysteine ethyl ester was utilized in identical peptide concentrations as a standard in order to calculate normalized reaction rates for each synthesized peptide.

**Table 5: Sequence overview of 22 synthesized di- tri- tetra- penta- and hexapeptides containing cysteine. The reactivation efficiency towards latent papain was compared. Cysteine residues are elucidated in bold notation. Two different solid-phases were utilized leading to two different C-terminal functional groups, amides and carboxylic acid, as depicted below.**

| **Hexapeptides** | **Pentapeptides** | **Tetrapeptides** | **Tripeptides** | **Dipeptides** |
|---|---|---|---|---|
| *H₂N-*E-G-C-G-R-G-*COOH* | *H₂N*-C-G-G-G-G-*COOH* | *H₂N*-C-C-R-G-*CONH₂* | *H₂N*-C-L-R-*CONH₂* | *H₂N*-C-F-*CONH₂* |
| *H₂N*-F-V-C-E-K-G-*COOH* | *H₂N*-C-L-R-C-G-*COOH* | *H₂N*-C-E-W-G-*CONH₂* | *H₂N*-C-F-R-*CONH₂* | *H₂N*-C-L-*CONH₂* |
| *H₂N*-G-G-C-G-G-G-*COOH* | | *H₂N*-C-K-S-G-*CONH₂* | | |
| *H₂N*-G-K-C-L-R-G-*COOH* | | *H₂N*-C-L-C-G-*CONH₂* | | |
| *H₂N*-G-L-C-L-R-G-*COOH* | | *H₂N*-C-L-R-G-*CONH₂* | | |
| *H₂N*-S-E-C-L-R-G-*COOH* | | *H₂N*-C-V-Q-G-*CONH₂* | | |
| | | *H₂N*-F-C-L-R-*CONH₂* | | |
| | | *H₂N*-C-W-R-G-*COOH* | | |
| | | *H₂N*-C-L-R-G-*COOH* | | |
| | | *H₂N*-C-S-G-G-*COOH* | | |

The latent papain reactivation assays were conducted in duplicate with peptide concentrations of 10 nM - 10 µM. The assays were conducted in well buffer with 1 nM latent papain P13. Reaction rates were normalized by dividing rate/rate cysteine ethyl ester (CysEE) and are shown in Figure 19. In order to give a clear overview, only the normalized rates for activator concentrations of 50 nM are depicted and compared.

The two most potent activators *H₂N*-CCRG-*CONH₂* and *H₂N*-CLCG-*CONH₂* both contain two cysteine residues and were expected to yield higher reaction rates because one peptide could theoretically activate two papain molecules. They dominate the top of the diagram.

On the other hand, none of the synthesized hexapeptides was found to be particularly efficient in reactivating latent papain and thus are positioned uniformly at the bottom of the diagram. The normalized rates of all hexapeptides are well below 1.0 which means that none of them reach the reactivation potential of cysteine ethyl ester. Given the diversity of sequences, this hints to the length of the peptide being the cause rather than sequence preferences. Additionally, cysteine is integrated within the sequence rather than positioned at the C-terminus although this was observed to be suboptimal in preceding experiments (not shown). With regard to the subsite mapping by Berger et al this sequence constellation was however interesting to examine despite the preceding findings (Berger et al., 1970).

The most prominent trend was observed in regard to the difference in C-termini of the peptides. Peptides with C-terminal carboxyl group were apparently less efficient in reactivating latent papain compared to those with a C-terminal amide group. This might not be due solely to sequence differences of the deployed peptides as can be seen when comparing the strongly reduced efficiency with less than half the rate of *H₂N*-CLRG-*COOH* (0.51) compared to the version with C-terminal amide (1.29). The behaviour of the pentapeptide *H₂N*-CLRCG-*COOH* could be explained in the same way which, although containing two cysteine residues, only reaches rates similar to cysteine ethyl ester (0.99) and performed worse than the other two most potent peptides containing two cysteine residues. This leads to the assumption, that disadvantageous interactions might occur between negatively charged groups (e.g., COOH-moiety) near the activating cysteine and the microenvironment of the papain active site. Nevertheless, peptides comprising C-terminal carboxyl groups are suitable to be used in the method of the invention.

Shorter peptides with N-terminal cysteine generally seem to be more efficient in papain reactivation. The most potent peptide with a single cysteine is *H₂N*-CLR-*CONH₂* which bears resemblance to the structure of the inhibitor E64 as seen in Figure 21.

With rates of 1.81 times that of cysteine ethyl ester, the peptide is a potent papain activator even at concentrations as low as 50 nM. If an additional glycine is added to the sequence to form *H₂N*-CLRG-*CONH₂,* the normalized rate drops to 1.29 which is a reduction of almost 30%. If arginine is omitted to form the dipeptide *H₂N*-CL-*CONH₂* the rate drops even lower to 0.88 which is a reduction of over 50%. This trend does not occur when comparing the peptide pair *H₂N*-CFR-*CONH₂* and *H₂N*-CF-*CONH₂.* If arginine is omitted to form the corresponding dipeptide, the rate does not drop but rises slightly to 1.05. Altogether one can assume that the tripeptide *H₂N*-CLR-*CONH₂* provides beneficial interactions with papain upon activation.

The peptides *H₂N*-CEWG-*CONH₂* and *H₂N*-CLRG-*COOH* demonstrate higher rates compared to sequences with small residues like *H₂N*-CSGG-*COOH*. In a similar assay as described above, the most efficient tripeptide *H₂N*-CLR-*CONH₂* was further compared to free cysteine, selenocysteine and TCEP as well as thiols that were already proven to be potent activators in preceding experiments (see Example 2, 2.1). Additionally, 1 µM TCEP was added to each well of the cysteine concentration series in order to examine the influence of TCEP on reaction rates of thiols. Selenocysteine was mandatorily prepared with TCEP as described in Example 5, 5.2.2.1and 1 µM TCEP was additionally added to each well. The resulting normalized reaction rates are shown in Figure 20 for the activator concentration series of 50 nM - 10 µM.

The results showed that selenocysteine, 4-acetamidothiophenol and 4-nitro-thiophenol were more potent activators compared to the tripeptide *H₂N*-CLR-*CONH₂.* Cysteine reached normalized rates of around 40% compared to cysteine ethyl ester and around 25% compared to the tripeptide. The supplementary 1 µM TCEP did increase the reaction rates of cysteine only slightly and it still remained a less potent activator compared to cysteine ethyl ester or any of the synthesized peptides. The rates for TCEP as a sole papain activator showed very little efficiency. In preceding experiments (not shown) the thiol 4-acetamidothiophenol was proven to be the second most efficient papain activator only surpassed by thiophenol which does not feature any functional groups and is therefore not suitable for the method of the invention since the activator has to be coupled to an amplification product of the target nucleic acid comprising modified nucleotides having a first reactive moiety. Any substitution at the thiophenol scaffold resulted in lower activator efficiency with 4-acetamidothiophenol being one of the most potent of all tested thiophenol derivatives. The activator appeared to be five times as efficient as the tripeptide H2N-CLR-CONH2 but was noticeably surpassed by selenocysteine which in turn showed rates 6.5 times higher than the tripeptide. The nature of selenium appears to enhance the efficiency of papain activators in various ways. The notable decrease in normalized rates with increasing concentration is most likely due to the approximation of the maximal rate as a result of almost complete papain reactivation. It appears that the reactivation of latent papain does not follow linear correlation with increasing activator concentrations (see also Example 2, 2.3). Furthermore, the confinements of the reactivation assay do not allow for proper differentiation of rates at those concentrations because the linear part of the fluorescence curves cannot be clearly observed for potent activators at high concentrations as previously noted. This is why the normalized rates seem to decrease for concentrations above 50 nM for the very potent activators and from 1 µM for the mediocre activators, whereas sub-optimal activators like cysteine still show increasing rates even at a concentration of 10 µM.

It is important to notice, that the concentration determination of peptide stock solutions is not trivial due to the nature of peptides forming salt bridges intra- and intermolecular as well as to TFA molecules within the lyophilizate. This can lead to small concentration differences of peptide stock solutions which could only be avoided by expensive out-sourced amino acid analysis for every single peptide. The sequence probing for potent peptide activators identified the E64 analogue tripeptide *H₂N*-CLR-*CONH₂* to be the most efficient papain activator of all synthesized peptides (with single cysteine). The differences among the tested sequences did however not result in exceptional distinctions similar to the level of sulfur exchange for selenium, observable with selenocysteine and cysteine. 4-Acetamidothiophenol apparently activated papain to a higher extent compared to the tripeptide *H₂N-*CLR-*CONH₂*. Since selenocysteine was an even more potent activator compared to the thiophenol analogue and the tripeptide was almost four times as efficient compared to free cysteine, the incorporation of selenocysteine into the peptide sequence was expected to result in even higher rates which is why further synthesis and experiments were directed towards peptide-based activators instead of thiophenol-based activators. Those highly efficient activators are presented in the following Example 2, 2.3.

### 2.3 Reactivation of latent papain via selenocysteine containing peptides

After the synthesized peptide *H₂N*-CLR-*CONH₂* was identified as a more potent activator for latent papain, compared to free cysteine, and selenocysteine was proven to be the most potent papain activator of all screened compounds (see Example 2, 2.2), the incorporation of selenocysteine into the peptide was expected to result in an even more efficient activator. To this end, the tripeptide *H₂N*-ULR-*CONH₂* (the one letter code for selenocysteine is U) was synthesized, screened and identified as an extraordinary papain activator.

Further, a linker was incorporated in order to attach a functional group, *i.e.,* a first reactive moiety, which is able to interact with the target molecule on the modified nucleotide or nucleoside, or specifically, the second reactive moiety comprised in the modified nucleotide. The solid phase peptide synthesis is described in detail in Example 4, 4.1.

Every intermediate of the multi-step synthesis was analysed and utilized individually in a papain reactivation assay in order to get a clear overview of papain preferences of the synthesized molecules.

Since selenols readily oxidize to diselenides at physiological conditions in the presence of molecular oxygen, the diselenide activators have to be reduced prior to papain activation. This can be achieved via addition of dithiols (most monothiols lack the necessary redox potential for reduction of diselenides as described by Singh et al., unless applied in 1000-fold excess 255) as first reported by Dickson et al. in 1969 and since utilized by other groups (Singh et al., 1992; Dickson et al., 1969; Burnell et al., 1980). DTT is however a fairly potent papain activator in itself, rendering it unsuitable for the reduction of utilized diselenide peptides. Burns et al. presented the phosphine TCEP to be a most efficient disulfide reducing agent with many beneficial properties and with the previously described characteristics of selenium, namely the high electrophilicity, it was assumed that TCEP could also be efficiently used for reduction of the herein utilized diselenides (Burns et al.1991). TCEP was further proven to be a poor papain activator making it an ideal reducing agent.

The assay was conducted in duplicate with the displayed activators in concentrations of 1 - 15 nM and papain P15 (1 nM) in well buffer with TCEP. Resulting reaction rates were normalized by division through the rate of residual papain activity (blank) rather than by comparison to cysteine or cysteine ethyl ester as conducted in Example 2, 2.1 and 2.2. The reason for this variation is because all activators were screened simultaneously on one 96 well plate rather than in separate experiments leading to a perfectly suitable and unified blank reference. Furthermore, future application of the DNA detection assay will not contain a reference activator and observed rates are preferably compared to the background activity within the assay. For this experiment the BioTek plate reader was used (see Example 5, 5.1.6.2).

Activation with the nucleoside-peptide hybrid dU-triazole-PEG3-ULR **(21)** resulted in the highest observed reaction rate of the screened compounds.

The most potent activator dU-triazole-PEG₃-ULR **(21)** was further compared to other compounds described as potent reducing agents in literature (Shaked et al., 1980; Cleland, 1964; Lukesh et al., 2012). To this end, cysteine ethyl ester, DTT, DTBA **(9)** and SeDTBA **(11)** were chosen for a direct comparison. The potent reducing agents BMS, DMH and DTA initially presented by the Whitesides group (Lamoureux and Whitesides, 1993; Lees et al., 1991) were not investigated further due to the lack of functional groups necessary for attachment to a linker structure and ultimately to the nucleotide/nucleoside and the DNA itself (see Example 3). The assay was conducted with three activator concentration series in the ranges of 50 pM - 750 pM, 1 nM - 15 nM and 10 nM - 10 µM. The resulting reaction rates were normalized against the background activity and the resulting normalized rates are presented in Figure 22 for the concentration range of 50 pM - 100 nM.

As seen in Figure 22 A, the reaction rates after activation with the nucleoside-peptide-hybrid dU-triazole-PEG₃-ULR **(21)** were considerably surpassing those of all other commercially available and synthesized activators. SeDTBA **(11)** was found to be the second most efficient activator with rates almost twice as high compared to DTBA **(9).** The commercially purchased and synthesized DTBA **(9)** showed a slight difference in rates which is most likely due to minute concentration differences arising from weighing errors and preparation of the stock solutions. Cysteine ethyl ester and DTT produced no substantial rate increase in this concentration range. Even at low picomolar concentrations the partial reactivation of latent papain with dU-triazole-PEG₃-ULR **(21)** was efficient enough to be distinguishable from the background activity.

Lukesh et al. reported a reactivation rate of latent papain with DTBA **(9)** that is 14 times higher compared to activation with DTT (Lukesh et al., 2012). This claim is corroborated by the findings in this work, where DTBA **(9)** produced rates 13.6 times higher than DTT at a concentration of 50 nM. The nucleoside-peptide-hybrid dU-triazole-PEG₃-ULR **(21)** is however strongly exceeding the potency of DTBA **(9)** at every concentration. At 50 nM the nucleoside-peptide hybrid produced rates 128 times higher than DTT and 9 times higher than DTBA **(9).** At a concentration of 10 nM the difference increased further and the rates are 159 times higher than DTT and 23 times higher than DTBA **(9).** With decreasing concentration, the potential of dU-triazole-PEG₃-ULR **(21)** became even more apparent because all other activators failed to activate latent papain to a degree which produced any significant result. Below the concentration of 2.5 nM none of the other activators produced rates higher than four times the background activity while the nucleoside-peptide-hybrid showed such a significant result at a concentration as low as 50 pM. At a half equimolar concentration of papain (500 pM) the activator still produced a rate 33 times above the background activity.

It can be summarized that the substitution of cysteine for selenocysteine in the potent tripeptide *H₂N-*CLR-*CONH₂* resulted in a highly efficient cysteine protease activator molecule. Extension of this tripeptide with a PEG linker and coupling to a nucleotide yielded dU-triazole-PEG₃-ULR **(21),** a papain activator that was potent even in sub equimolar and low- to mid-picomolar concentrations. To the authors best knowledge, no other in literature reported papain activator shows comparable efficiencies. It was therefore concluded, that the cysteine protease activator peptide azido-PEG3-ULR **(15)** within dU-triazole-PEG₃-ULR **(21)** is suitable for attachment to commercially purchased DBCO-modified dUTP nucleotides via ring-strain promoted click chemistry (see Example 3).

### 2.4 Heat stability of the cysteine protease activator dU-triazole-PEG₃-ULR (21)

With the objective of developing an antibody free method for detecting a target nucleic acid in a sample, the inventors evaluated the suitability of the components utilized in latent papain reactivation assays.

Since the final step of target nucleic acid detection via latent cysteine protease reactivation is typically conducted under similar assay conditions as the reactivation in solution, the only mentionable difference is the hybridization step of biotinylated DNA probe and DBCO containing DNA amplificate after amplification, e.g., by PCR, in accordance with a preferred embodiment of the method of the invention. A detailed standard protocol of PCR-ELISA was presented by Musiani et al. describing every single step and reaction condition (Musiani et al., 2007¹; Musiani et al., 2007²). Melting of DNA requires heat in order to overcome hydrogen bonding between DNA bases. After dissociation of DNA strands, they can rehybridize upon cooling and revert to double-stranded DNA. This requires temperatures well above room temperature and the specific melting temperature depends on the DNA sequence. In the described assays by Musiani et al. the hybridization temperature was 55 °C for a total duration of 70 minutes. The thermo stability of the synthesized most competent papain activator was therefore evaluated. To this end, a standard papain reactivation assay was prepared (see Example 5, 5.2.2.2, fluorescence method 1) and three aliquots of the activator dimer dU-triazole-PEG₃-ULR **(21)** were incubated in phosphate buffer pH 7 for 1, 2 and 3 hours at 95°C and directly compared to a freshly prepared activator aliquot in phosphate buffer pH 7 at room temperature. Because traces of precipitation were observed for the heated aliquots, vigorous mixing was conducted before the dilution of the activator and subsequent preparation of the 96 well plate. The assay was conducted in triplicate with activator concentrations of 1 - 15 nM and papain P15 (1nM). Reaction rates were normalized by dividing rate/rate blank and are shown in Figure 23.

The resulting normalized reaction rates showed that the efficiency of latent papain reactivation did not vary significantly between the different activator treatments. Freshly prepared activator was the most efficient, as was to be expected, but heating to very high temperatures for two to three hours resulted only in a maximal decline of 27 % at an activator concentration of 15 nM. The fact that rates for activator incubated for two hours were slightly lower when compared to activator incubated for three hours, as well as the fact that precipitate was formed during prolonged heating gives rise to the assumption that the activator does in fact not degrade but, in some way, denatures. Through vigorous mixing, the precipitate could almost be completely dissolved again leading to similar reaction rates. Since the applied temperature was deliberately raised beyond the common hybridization temperature and the results prove the activator dU-triazole-PEG₃-ULR **(21)** to work sufficiently at these conditions, it can be summarized that this activator or the actually applied analogue structure within the nucleic acid strand will work efficiently when used in the method of the invention. The heat stability of the diselenide activator was superior to the previously utilized monomeric thiol activators. This was examined in a preliminary assay with cysteine, which resulted in much lower reaction rates after heating (results not shown). It should additionally be noted that the problem of precipitation will most likely not affect the method of the invention because of milder assay conditions and the option of immobilization of the labelled amplification product of the target nucleic acid within the well to which the activator is covalently bound.

### Example 3: DNA detection via papain reactivation assays

Since dU-triazole-PEG₃-ULR showed promising results, the peptide intermediate azido-PEG₃-ULR **(15)** was utilized in DNA-detection assays.To this end, the azide moiety of the peptide dimer was used in a ring-strain promoted click reaction with the DBCO moiety of commercially purchased dUTP-DBCO nucleotide derivative. Since the peptide dimer features two azide-moieties, it was assumed that problems with DNA-crosslinking might arise if one peptide-dimer reacts with two different DNA strands or forms intramolecular bridges. A second peptide-dimer was therefore synthesized (see Example 4, 4.1) consisting of two different monomeric peptides, one identical to azido-PEG₃-ULR **(15)** with an azide moiety and a second one of similar structure containing an amine moiety instead. The mixed peptide-dimer azido-amino-PEG₃-ULR **(16)** and the previously described azido-PEG₃-ULR **(15)** were both utilized in DNA detection assays and their structures are shown in Figure 24.

SARS-CoV-2-S DNA (in the following called Spike DNA) was amplified via PCR (see Example 5, 5.3.1) and DBCO modified dUTP nucleotides were incorporated in the process. The PCR reaction mixture contained two different amounts of dUTP-DBCO. Amplification of a negative control DNA (subsequently referred to as DNA-) was achieved with 100% dTTP and 0% dUTP-DBCO content in the reaction mixture. DNA amplification of a positive control DNA (subsequently referred to as DNA+) contained 75% dUTP-DBCO and 25% dTTP nucleotides. A detailed overview of the process for the DNA detection of biotinylated DNA+ is depicted in Figure 25. In this Example, the biotinylated DNA+ is incubated with activator peptide prior to immobilization in the streptavidin coated wells.

### 3.1 DNA detection with single-step DNA purification via Sephadex

In accordance with the method of the invention, the amplified target nucleic acid may be incubated with activator peptide before being transferred into streptavidin coated wells. In accordance with a preferred embodiment, a purification step may therefore be included in order to remove excess of unreacted free activator and other PCR compounds. G-50 Sephadex Quick Spin columns were chosen because they efficiently separate modified DNA from excess peptide, primer and nucleotides in a fast and simple process. Biotinylated Spike DNA+ and DNA- were prepared by PCR (see Example 5, 5.3.1.1) and then divided into two equal portions of 50 µL each. The raw PCR solution was subsequently incubated with equimolar amounts of either azido-PEG₃-ULR **(15)** or azido-amino-PEG₃-ULR **(16)** (see Example 5, 5.2.3.1). After incubation, all four DNA portions were purified via sephadex columns (see Example 5, 5.3.4) and two different dilutions of each DNA solution (1:100 and 1:50 in well buffer) were prepared. 300 µL of each solution was added to the streptavidin coated Greiner-Bio-One plate. After immobilization and washing steps, the reactivation assay was conducted with latent papain P20 (see Example 5, 5.2.4.2). The resulting reaction rates were normalized against the rates of the corresponding negative control DNA-. These normalized rates are shown in Figure 26. Measurements were performed in triplicate and standard deviations are shown.

As seen in **Figure 26** doubling of DNA-peptide-hybrid per well resulted only in an insignificant increase in reaction rates in both cases. Further diluting DNA samples will resolve this (see Figure 27). This is possibly due to a maximal biotin binding capacity of the wells. Normalized rates of positive controls incubated with azido-PEG₃-ULR **(15)** were slightly higher compared to rates reached with azido-amino-PEG₃-ULR **(16).** Even the lowest activation rate with azido-amino-PEG₃-ULR **(16)** was 147 times higher compared to the negative control, whereas with azido-PEG₃-ULR **(15)** rates 220 times higher were achieved. These values prove, that the single-step purification is suitable for the assay procedure and yield pure samples.

The method steps as described above result in a very sensitive DNA-detection system that can differentiate positive (DNA+, DNA with DBCO moieties) and negative (DNA-, DNA without DBCO moieties) control DNA by over factor 200.

### 3.2 Omitting preceding DNA purification

Although the purification with sephadex columns is quick, easy and efficient, omitting purification altogether accelerates the process even more and abolish the need for specific additional chemicals and consumables.

To this end, biotinylated Spike DNA+ and DNA- (see Example 5, 5.3) were prepared by PCR (see Example 5, 5.3.1.1) and subsequently incubated with equimolar amounts of azido-amino-PEG₃-ULR **(16).** Three different dilutions of each DNA solution were prepared (1 :600, 1:100 and 1:30 in well buffer) and 300 µL of each dilution was added to the streptavidin coated plate in triplicate. After immobilization and washing steps, the reactivation was conducted with papain P20 (see Example 5, 5.2.4.1). The reaction rates were normalized against the rates of the corresponding negative control DNA-. These normalized rates are shown in Figure 27A. Measurements were performed in triplicate and standard deviations are shown.

As seen in Figure 27 A, the normalized rate of 1:600 DNA+ph (marked in red) was approximately 50% lower compared to the rate with 1:100 DNA+ph. Normalized rates of 1:100 and 1:30 DNA+ph were almost identical. The term DNA+ph, refers to a peptide hybrid of the DNA+, wherein the peptide is attached to the modified DNA as the final result after the click reaction. The observed normalized rates were generally lower by factor 2 compared to the Example 3.1. This most likely means that residual peptide has not been completely removed from the wells after several washing steps and may unspecifically bind to the wells, if no preceding purification step is introduced. Furthermore, the small differences between dilutions of 1:100 to 1:30 DNA+ shows that, as previously described, the highest DNA+ph concentration most likely reaches the maximal binding capacity of the wells.

Considering all factors, the method of the invention can be conducted without purification, and, despite minor shortcomings, still allows for a very sensitive differentiation of negative and positive control nucleic acid by factor 50 - 100.

### 3.3 Comparison between biotinylated and non-biotinylated positive control DNA

In the this experiment it was examined if biotinylated positive control DNA+ph and a non-biotinylated positive control DNA+ph can be differentiated and if the non-biotinylated DNA+ph could possibly affect papain activation. To this end, biotinylated Spike DNA+ and non-biotinylated Spike DNA+ (see Example 5, 5.3) were prepared by PCR (see Example 5, 5.3.1.1). Both PCR solutions were subsequently incubated with equimolar amounts of azido-amino-PEG₃-ULR **(16)** (see Example 5, 5.2.3.1). Both DNA solutions were then purified via sephadex column and the eluates were diluted 1:600, 1:100 and 1:30 in well buffer and 300 µL of each solution was added to the streptavidin coated plate in triplicate. After incubation and washing steps, the reactivation assay was prepared with latent papain P20 (1nM) in well buffer (see Example 5, 5.2.4.1). The resulting rates of the biotinylated DNA+ph were normalized against the rates of the corresponding non-biotinylated DNA+ph (see Figure 28 A) as well as against the rate of residual active papain without addition of DNA or peptide (blank) as seen in Figure 28 B.

The results show that the normalized rates against those of the corresponding non-biotinylated DNA+ph were rising with decreasing DNA+ph concentrations as seen in Figure 28A whereas normalized rates with the blank rate increased with increasing DNA+ph amounts. This means that the non-biotinylated DNA+ph forms unspecific interactions with the well surface in a similar manner as observed for the free activator in the previous experiment. Unlike residual peptide, non-biotinylated DNA-peptide-hybrid is not separated during the purification procedure and thus affects the reactivation of papain. This effect decreases the sensitivity and both DNA+ph samples can only be differentiated by a maximal factor of 23.

With regard to the method of the invention, the effect of unspecific interactions of DNA+ with the well surface should be no obstacle because without the corresponding template DNA no amplificate would be produced during amplification of the target nucleic acid.

### 3.4 General conclusion of the DNA experiments

During PCR, Taq polymerase readily incorporated a biotin tag (linked to the forward primer) as well as the modified nucleotide dUTP-DBCO (50 or 75% dUTP-DBCO with 50 or 25% dTTP) into the amplificate. The click reaction between amplificated dUTP-DBCO containing DNA+ and the actual papain activator peptide was found to be highly efficient and should be performed before dilution and addition to the streptavidin coated plates. The immobilization of the DNA was found to be very efficient. Excess peptide adheres to the well surface in an apparently unspecific manner and the interaction appears mostly irreversible, since excessive washing did not result in lower interactions (see 2.11). If the click reaction is performed in equimolar amounts, the residual peptide binding to the well will affect papain reactivation only to a small extent which can be decreased to a minimum if DNA-peptide-hybrid is separated from excess peptide through a quick and easy purification via a sephadex column. This way, the rate of positive control DNA+ph and negative control DNA- can be differentiated by more than factor 200. According to one embodiment, the method of the invention utilizes a biotinylated DNA probe for hybridization and immobilization of corresponding target DNA-peptide-hybrid (see Figure 29). This enhances the specificity twofold: first by specific primers used during amplification, e.g.,during PCR and secondly by specific interactions of the biotin-probe and the amplificate. Furthermore, this enables the possibility for multiplex screening of different DNA targets in one solution. Results were promising and the targeted DNA-peptide-hybrid activated papain more efficiently compared to the residual control DNA-peptide-hybrid which presumably adhered unspecifically to the well surface, but rate differences were still rather small.

The utilized amount of template DNA in each conducted PCR was 10 ng (50 µL of a 0.2 ng/µL solution) which is 10 - 20-fold below the amounts that are recovered by RNA or DNA extraction of standard nasal swabs as performed in SARS-CoV-2 diagnostics (Gorzynski et al., 2020; Klingen et al., 2021). In assays with preceding purification steps the concentration of the DNA amplificate was determined and varied between 80 ng (see Example 4.3) and a maximum of 936 ng (see Example 4.4) per well. The applied DNA amount of samples that are not purified cannot be determined. The results of the experiments hinted to an exceeded well capacity which could mean that the DNA concentration could be further decreased, highlighting the sensitivity of the assay.

Altogether, it can be stated that the developed tools and method of the invention are a suitable alternative or even advantageous technique compared to PCR-ELISA. The method is completely viable via standard laboratory equipment and the peptide synthesis, PCR and detection assay could even be fully automated.

### Example 4: Compound synthesis

### 4.1 Solid-phase synthesis (SPPS)

For the standard peptides, the solid-phases of use were TentaGel HL Ram resin for all peptides with C-terminal amide group as well as Fmoc-Gly-Wang resin for all peptides with C-terminal glycine and a free carboxyl group. All steps were performed manually. TentaGel (65.8 mg, 25 µmol, 1 eq.) or Wang resin (37.9 mg, 25 µmol, 1 eq.) was weighed in for each reactor and initially swelled and shaken in 4 mL DMF for 2 min. In each of the following steps, the reactors were shaken for the specified incubation times. Fmoc protecting groups were removed with 3 × 4 mL of 20 % piperidine in DMF for 3 min each. Afterwards the solid-phase was washed extensively with 3 × 4 mL DMF, 3 × 4 mL DCM and 1 × 4 mL DMF for 2 min each. Each of the protected amino acids as well as the coupling reagents OxymaPure and DIC were used in 3-fold molar excess for each coupling step. The corresponding amino acid, OxymaPure and DIC were dissolved in 4 mL DMF/DCM (1:1), added to the reactor and shaken at 1500 rpm for one hour. After coupling of each amino acid, the resin was washed with 3 × 4 mL DMF for one minute each, before subsequent deprotection of Fmoc. After coupling of the last amino acid and deprotection of the N-terminal Fmoc, the solid-phase was prepared for cleavage by extensive washing with 3 × 4 mL DMF, 3 × 4 mL DCM, 2 × 4 mL EtOH for 2 min each and subsequent drying via suction pump.

Cleavage of the peptide from the resin with simultaneous deprotection of side chain protection groups was realised through incubation with a cleavage cocktail containing TFA/TIPS/water (90:5:5). Each reactor was incubated with 4 mL for one hour. The solution was then transferred to a round-bottom-flask and the process was repeated with 2 × 2 mL for 30 min each. Afterwards the resin was washed with 2 × 4 mL DCM and all combined fractions were concentrated under reduced pressure to a volume of 1.5 mL. The remaining solution or suspension was then transferred into 30 mL of -80 °C cooled diethyl ether, followed by centrifugation (2383 g, 3500 rpm) for 10 min at room temperature. After decanting of the clear ether supernatant, the precipitated peptide pellet was dried over argon, dissolved in ACN/water and lyophilized. Purification of the peptides was achieved via preparative HPLC (see Example 5, 5.1.3) and analysed by analytical HPLC (see Example 5, 5.1.2) as well as HRMS (see Example 5, 5.1.4). Analytical characteristics of all synthesized peptides are summarized in **Table 6.**

**Table 6: Overview of analytical data of synthesized standard peptides. Retention time tᵣₑₜ, was determined via analytical HPLC, mass analyses were performed via ESI-HRMS. [M+H]⁺ (calc.) refers to the protonated exact mass, [M+H]⁺ (found) refers to the mass signals obtained by mass spectrometry. Purity was calculated via aHPLC peak area %. Purity values in brackets (--) could not be determined due to interference with the injection peak. aHPLC method 1: 5-95% B in 21 min, method 2: 5-20% B in 21 min.**

| **Peptide sequence** | **aHPLC t_{ret.}** [min] | **aHPLC-method** | **[M+H]⁺ (calc.)** [Da] | **[M+H]⁺ (found)** [Da] | **Purity aHPLC** [%] |
|---|---|---|---|---|---|
| *H₂N-*C-C-R-G-*CONH₂* | 4.09 | 1 | 437.1748 | 437.1738 | (--) |
| *H₂N*-C-E-W-G-*CONH₂* | 8.98 | 1 | 493.1864 | 493.1849 | 93 |
| *H₂N*-C-F-*CONH₂* | 13.62 | 2 | 268.1114 | 268.1287 | 97 |
| *H₂N*-C-E-R-*CONH₂* | 7.76 | 1 | 424.2125 | 424.2150 | 95 |
| *H₂N*-C-G-G-G-G-*COOH* | 3.56 | 1 | 350.1129 | 350.1116 | (--) |
| *H₂N*-C-K-S-G-*CONH₂* | 3.03 | 1 | 393.1915 | 393.1883 | (-) |
| *H₂N*-C-L-C-G-*CONH₂* | 7.05 | 1 | 394.1577 | 394.1538 | 99 |
| *H₂N*-C-L-*CONH₂* | 8.22 | 2 | 234.1271 | 234.1270 | 94 |
| *H₂N*-C-L-K-*CONH₂* (unstable) | 6.04 | 1 | 362.2220 | 362.2230 | 73 |
| *H₂N*-C-L-R-C-G-*COOH* | 7.44 | 1 | 551.2428 | 551.2401 | 99 |
| *H₂N*-C-L-R-*CONH₂* | 5.70 | 1 | 390.2282 | 390.2287 | 99 |
| *H₂N*-C-L-R-G-*CONH₂* | 5.81 | 1 | 447.2496 | 447.2436 | 95 |
| *H₂N*-C-L-R-G-*COOH* | 7.32 | 1 | 448.2337 | 448.2315 | 99 |
| *H₂N*-C-S-G-G-*COOH* | 3.17 | 1 | 323.1020 | 323.1014 | (--) |
| *H₂N*-C-V-Q-G-*CONH₂* | 4.17 | 1 | 405.1915 | 405.1878 | (--) |
| *H₂N*-C-W-R-G-*COOH* | 9.22 | 1 | 521.2289 | 521.2276 | 98 |
| *H₂N*-E-G-C-G-R-G-*COOH* | 4.81 | 1 | 578.2351 | 578.2359 | (--) |
| *H₂N*-F-C-L-R-*CONH₂* | 9.80 | 1 | 537.2966 | 537.2948 | 93 |
| *H₂N*-F-V-C-E-K-G-*COOH* | 8.71 | 1 | 682.3229 | 682.3249 | 98 |
| *H₂N*-G-G-C-G-G-G-*COOH* | 3.83 | 1 | 407.1343 | 407.1327 | (--) |
| *H₂N*-G-K-C-L-R-G-*COOH* | 7.26 | 1 | 633.3501 | 633.3461 | 97 |
| *H₂N*-G-L-C-L-R-G-*COOH* | 10.20 | 1 | 618.3392 | 618.3281 | 92 |
| *H₂N*-S-E-C-L-R-G-*COOH* | 8.08 | 1 | 664.3083 | 664.3033 | 99 |
| *H₂N-*V-C-L-R-*CONH₂* | 7.66 | 1 | 489.2966 | 489.2878 | 94 |
| *H₂N*-Y-C-L-R-*CONH₂* | 8.33 | 1 | 553.2915 | 553.2861 | 97 |
| *H₂N*-Y-W-C-L-V-G-*COOH* | 14.03 | 1 | 740.3436 | 740.3382 | 95 |

Furthermore, different linker structures were attached in order to enable coupling to modified nucleosides/nucleotides (see Example 4, 4.2).

The peptide synthesis of selenocysteine containing peptides was conducted similarly to the previously described peptides. Due to its reactive nature, succinic anhydride was coupled without additional coupling reagents in the presence of N-methylmorpholine (NMM) and yielded the resin bound suc-ULR intermediate. Four different linkers were attached in three reaction mixtures under identical reaction conditions. The previously utilized coupling system with DIC and OxymaPure was exchanged for the phosphonium salt PyOxim ([ethyl cyano(hydroximino)acetato-O]tri-1-pyrrolidinylphosphonium hexafluorophosphate) which combines properties of both OxymaPure and phosphonium salts like TBTU. This change was mandatory because synthesis with DIC and OxymaPure did not yield the expected products. Azido-PEGa-ULR **(15)** was produced by addition of *N₃*-PEG₃-*NH₂* and azido-amino-PEG₃-ULR **(16)** was produced by addition of equimolar amounts of *N₃-*PEG₃-*NH₂* and Boc-*HN*-PEG₃-*NH₂.*

After attachment of the linker, the peptides and remaining protection groups were simultaneously cleaved from the solid-phase by incubation with TFA/DMSO (9:1). This cleavage cocktail differed from the one used for standard peptides which consisted of TFA/TIPS/water (90:5:5). The modification was necessary since cleavage of the Mob (p-methoxybenzyl) protection group of selenocysteine was not possible with the standard cleavage cocktail. In fact, efficient cleavage of the Mob protection group was subject of discussion for a long period of time. Fujii et al. presented the first successful method for deprotection in 1987 involving TMSBr (bromotrimethylsilane) or TMSOTf (trimethylsilyl trifluoromethanesulfonate) in combination with m-cresol, thioanisole and TFA, a method widely utilized ever since. Because the reaction conditions are very harsh, the reaction mixture has to be cooled on ice, complicating the handling and prolonging reaction times. Otaka and Koide et al. presented a more convenient method featuring a cleavage cocktail consisting of TFA and DMSO. Contrary to the previous method, this one yields the oxidized version of cysteine or selenocysteine leading to peptides containing inter- or intramolecular disulfides and diselenides respectively.

Since it was assumed that the diazide peptide dimer azido-PEG₃-ULR **(15)** might crosslink DNA when utilized, the asymmetric peptide dimer azido-amino-PEG₃-ULR **(16)** was synthesized featuring only one azide moiety for the ring-strained click reaction (see Example 5, 5.2.3). This was possible by adding two different linker moieties in the last coupling step, leading to a mixture of two different resin bound peptides. During cleavage with TFA and DMSO, these two peptides form three different peptide dimers (azido-PEGs-ULR **(15),** azido-amino-PEG₃-ULR **(16)** and amino-PEGs-ULR) with statistically distributed yields. After purification and separation via preparative HPLC, the asymmetric peptide dimer was recovered in >98% purity.

In summary, the described synthesis of Azido-PEG₃-ULR **(15)** and azido-amino-PEG₃-ULR **(16)** was successful and yielded all three selenocysteine containing peptides in their oxidized dimeric form. All were purified via preparative HPLC, identified via ESI-HRMS, utilized in papain reactivation assays (see Example 2, 2.3) and deployed in further syntheses of nucleoside/nucleotide-peptide-hybrids (see Example 4, 4.2). Additionally, the intermediate tripeptide ULR **(12)** and succinyl-coupled peptide suc-ULR **(13)** were separately isolated, characterized and utilized in papain reactivation assays (see Example 2, 2.3). A list of all synthesized selenocysteine containing peptides and their analytic data is shown in **Table 7.**

**Table 7: Overview of analytical data of synthesized selenocysteine containing peptides. Retention time tᵣₑₜ. was determined via analytical HPLC, mass analyses were performed via ESI-HRMS. [M+H]⁺ (calc.) refers to the calculated protonated exact mass, [M+H]⁺ (found) refers to the mass signals obtained by mass spectrometry. Purity was calculated via analytical HPLC peak area %. Azido-amino-PEG₃-ULR (16) underwent diselenide exchange reactions leading to partial degradation upon lyophilization, hence the purity value in brackets.**

| **Peptide dimer** | **Sequence** | **aHPLC tᵣₑₜ [min]** | **pHPLC tᵣₑₜ [min]** | **[M+H]⁺ (calc.) [Da]** | **[M+H]⁺ (found) [Da]** | **Purity aHPLC [%]** |
|---|---|---|---|---|---|---|
| **ULR(12)** | **(*H₂N*-Sec-Leu-Arg-*CONH₂*)₂** | **755** | **19.42 (15mL/min)** | **873.32.23** | **873.1913** | **99** |
| **Suc-ULR(13)** | **(Succinyl-Sec-Leu-Arg-*CONH₂*)*₂*** | **10.37** | **24.58 (15mL/min)** | **1073.3544** | **1073.3522** | **99** |
| **Azido-PEG₃-ULR(15)** | **(Azido-PEG₂-*HN*-succinuyl-Sec-Leu-Arg-*CONH₂*)*₂*** | **14.14** | **30.88 (15mL/min)** | **1473.6091** | **1473.5996** | **95** |
| **Azido-amino-PEG₃-ULR(16)** | **(Azido-PEG₂-*HN*-succinyl-Sec-Leu-Arg-*CONH₂*)-(*H*₂*N-*PEG₂-*HN*-succinyl-Sec-Leu-Arg-*CONH₂*)** | **12.54** | **26.62 (80mL/min)** | **1475.6499** | **1475.6060** | **98 (70)** |

### 4.2 Synthesis of two nucleotide-peptide-hybrids for papain reactivation

### Synthesis of dU-DBCO (29)

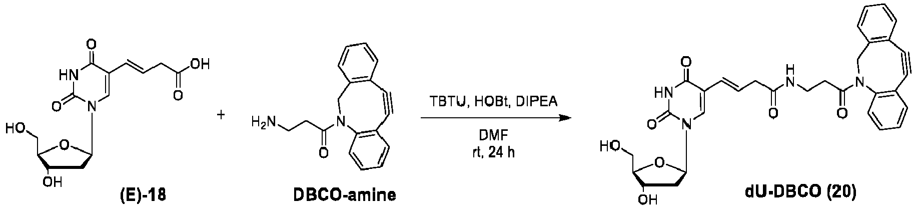

Compound **(E)-18** (25 mg, 80.7 µmοl, 1 eq.) was dissolved in 5 mL DMF and mixed together with TBTU (64.8 mg, 202 µmοl, 2.5 eq.) in 3 mL DMF and DIPEA (351 µL 10% solution in DMF, 202 pmol, 2.5 eq.). The mixture was stirred for 5 min, after which HOBt (27.3 mg, 202 µmοl, 2.5 eq.) in 3 mL DMF was added and the solution was kept stirring for another 5 min. Afterwards dibenzocyclooctyne-amine (DBCO-amine) (26.7 mg, 96.8 pmol, 1.2 eq.) in 5 mL DMF was added and the complete reaction mixture was stirred at room temperature for 24 h. The solvent was removed by distillation *in vacuo* and the crude product was dissolved in 4 mL of 25% ACN in water. Purification was achieved via preparative HPLC to yield **20** (11.3 mg, 19.8 µmοl, 25%).

**Analytical HPLC** (5-95% B in 21 min, 1 mL/ min): ***t***_{ret.} = 15.7 min.

**HRMS:** calculated C₃₁H₃₀N₄O₇ 571.2187 [M+H]⁺, found m/z 571.2131.

### Synthesis of the dU-triazole-PEG₃-ULR dimer (21)

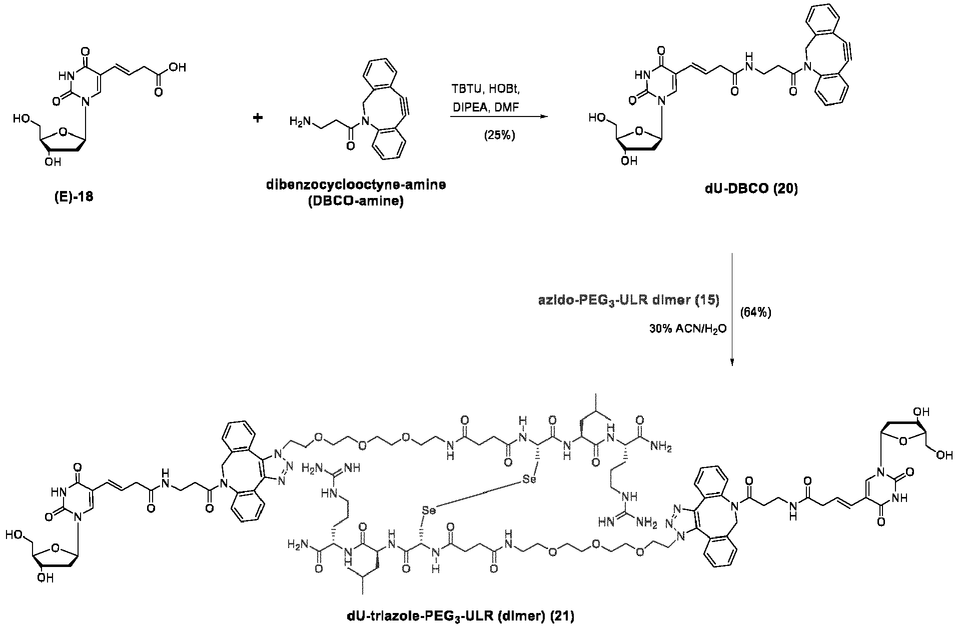

The azido-PEG₃-ULR peptide dimer **(15)** (see Example 4,4.1) (9.38 mg, 6.37 µmοl, 1 eq.) was dissolved in 1.4 mL 30% ACN/H₂O and added to the nucleoside **20** (8 mg, 14 µmοl, 2.2 eq.). The solution was shaken for 2 h after which the reaction mixture was diluted with 4 mL 25% ACN/H₂O and directly purified via preparative HPLC to yield the product **21** as a white solid (10.7 mg, 4.1 pmol, 64%).

**Analytical HPLC** (5-95% B in 21 min, 1 mL/ min): ***t***ᵣₑₜ. = 14.7 min.

**HRMS:** calculated C₁₁₆H₁₆₀N₃₀O₃₀Se₂ 1307.5197 [M+2H]²⁺, found m/z 1307.5130.

### Example 5 - Material and Methods

### 5.1 General chemical methods

### 5.1.1 NMR spectroscopy

Synthesized compounds were characterized by nuclear magnetic resonance spectroscopy (NMR) with either the Bruker DRX200 (200 MHz), Bruker Avance II (400 MHz) or Avance III (600 MHz) spectrometers at the Inorganic Chemical Institute of the University of Heidelberg. Chemical shifts δ in ppm are given relative to the tetramethylsilane standard for both ¹H and ¹³C nuclei and coupling constants J are given in Hz. Deuterated solvents were used and the residual proton signal was referenced for the ¹H spectra whereas the ¹³C signals of the deuterated solvent were referenced for the ¹³C spectra respectively with values given in literature and software. NMRs were analysed via the ACD/NMR Processor Academic Edition v.12 software. The following abbreviations were used for the observed multiplicity of NMR signals: s = singlet, d = doublet, t = triplet, dt = doublet of triplet, dd = doublet of doublet, q = quartet, sext = sextet, m = multiplet. For ¹H spectra, protons of unnumbered atoms are shown in *italic* notation.

### 5.1.2 Analytical HPLC

Analytical high-performance liquid chromatography (HPLC) was performed on a Shimadzu LC20 system with LC-20AD pumps, SPD-M20A diode-array detector, DGU-11A degasser and a CTO-10ASvp column oven. The C18 column of use was an EC 250/4 Nucleosil 100-5 C18 (5 µm, 250 x 4 mm) from Macherey-Nagel. Two solvents were used as eluents, solvent HPLC-A (0.1% TFA in water) and solvent HPLC-B (80% ACN, 0.1% TFA in water). The absorption was detected at 218 nm. Different analytical gradients were used and defined as the following HPLC methods:
aHPLC method 1: 5-95% HPLC-B in 21 min, flow rate 1 mL/min.
aHPLC method 2: 5-20% HPLC-B in 21 min, flow rate 1 mL/min.

Chromatograms were recorded via the LabSolutions 5.54 SP5 software and exported as .txt files. The chromatograms were then edited and visualized with OriginPro 9.0.0 SR2 and exported as .png pictures.

### 5.1.3 Preparative HPLC

Preparative purifications were conducted on a Shimadzu LC20 system with LC-20AP pumps, SPD-20A UV-detector, CBM-20A controller and FCV-20AH2 valve unit. Solvents HPLC-A and HPLC-B were used with two different C18 columns: VP 250/50 Nucleosil 100-5 C18 column (5 µm, 250 x 50 mm) and VP 250/21 Nucleosil 100-5 C18 column (5 µm, 350 x 21 mm) (both Macherey-Nagel). The absorption was detected at 218 nm and the fractions were collected manually and analysed via ESI-MS. The two different columns were used with different solvent gradients and are defined as the following HPLC methods:
pHPLC method 1 (21 mm column): 5-95% HPLC-B in 34 min, flow rate 15 mL/min.
pHPLC method 2 (50 mm column): 5-95% HPLC-B in 34 min, flow rate 80 mL/min.

Chromatograms were recorded via the LabSolutions 5.54 SP5 software.

### 5.1.4 ESI mass spectrometry

Electrospray ionization (ESI) mass spectrometry (MS) was performed with a Bruker micrOTOF focus II mass spectrometer (ESI and High-Resolution-ESI). Samples were prepared in different mixtures of ACN, MeOH and water, as well as HPLC-A and HPLC-B solvents. Measurements were carried out in positive and negative ion mode, depending on the compound. Spectra were recorded via the Bruker Compass 1.3 SR 1 for micrOTOF software. The files were then converted into .mzML files via the SeeMS 3.0.20351.0 software, annotated via mMass 5.5 and exported as .pdf files which were then edited via Adobe Illustrator CC v.23 and exported as .png pictures.

### 5.1.5 Thin-layer chromatography and flash chromatography

Analytical thin-layer chromatography (TLC) was carried out on POLYGRAM^{®}SIL G/UV₂₅₄ TLC plates as well as on Alugram RP-18W plates (both Macherey-Nagel). TLC visualization was accomplished using UV-light at 254 nm and three different staining solutions:
Ninhydrin-solution: Ninhydrin (0.3 g), EtOH (100 mL), AcOH (3 mL)
Cerium-Molybdenum-solution: (NH₄)₆Mo₇O₂₄ · 4 H₂O (5 g), Ce(SO₄)₂ (2 g), H₂SO₄ (10%, 200 mL)
Permanganate-solution: KMnO₄ (3 g), K₂CO₃ (20 g), H₂O (300 mL), NaOH (5%, 200 mL)
Flash column chromatography was performed for purification and desalting purposes. Two different solid-phases were used: Silica gel (40-63 µm, 60 Å pore size, Sigma-Aldrich) and Polygoprep 60-50 C18 (60 Å pore size, Macherey-Nagel).

### 5.1.6 NanoDrop 2000 measurements

A quick and easy approximation of the papain concentration was achieved via spectrophotometric measurements with NanoDrop 2000. The software package for all measurements was NanoDrop2000 version 1.6.0.198. Measurements were realized with the Protein A280 option and the extinction coefficient E^{1%}₂₈₀ₙₘ = 25 (E^{1%}₂₈₀ₙₘ is the mass extinction coefficient; the absorbance of a 1% solution by mass with the unit g⁻¹ · L · cm⁻¹). The molar absorption coefficient ε₂₈₀ₙₘ = 58 096 M⁻¹ · cm⁻¹ and the extinction coefficient E^{1%}₂₈₀ₙₘ = 25 were given in literature.

Initially 3 µL of the blank solution (e.g., 0.1 M phosphate buffer at pH 7) were applied onto the NanoDrop 2000 pedestal and defined as internal blank value. Then 3 µL of a clear papain solution (in the same buffer) were added onto the pedestal and measured. All measurements were realized in triplicate and the average value was noted.

### 5.1.7 Fluorometric measurements

### 5.1.7.1 EnSpire^{®} Multimode Plate Reader

Most measurements were carried out with an EnSpire^{®} Multimode Plate Reader and the original software version used was 4.13.3005.1482. Measurements were performed at an excitation wavelength of 370 nm and the corresponding emission wavelength of 460 nm specific for 7-amino-4-methylcoumarin as part of the papain substrates Z-Phe-Arg-AMC and Suc-Leu-Leu-Val-Tyr-AMC (Bachem). Temperature inside the machine was set to 27 °C, which resulted in an average well temperature of 25 °C. Plate type was set to 96 general, well volume was 380 µL, measuring height was 12.1 mm, number of flashes was set to 100 and measuring intervals were either 30 seconds or 60 seconds over the course of 15-120 min, depending on the assay (specifics can be found in the dedicated chapters). Four different 96-well plates were used in the course of this work. For the standard papain reactivation assays, black FLUOTRAC^{™} 200 96W Microplates from Greiner Bio-One were used in two different versions, the medium binding and the non-binding flat F-bottom plates. For the immobilization experiments, two different versions of streptavidin coated plates were used. One was made by Greiner Bio-One, the C-bottom black Streptavidin-coated 96 Well Plates (Biotin binding capacity >20 pmol/well, coating volume 300 µL) and the second ones were made by Thermo Scientific, namely the black Pierce^{™} Streptavidin Coated High Capacity 96 Well Plates (Biotin binding capacity >125 pmol/well, coating volume 100 µL).

### 5.1.7.2 BioTekTMSynergyTM MX Microplate Spectrophotometer

Due to temporary technical defects of the EnSpire Plate Reader, some measurements (Example 2, 2.3) were taken with a second plate reader, the BioTek^{™}Synergy^{™} MX Microplate Spectrophotometer with software version Gen5 2.01.14, under similar conditions as described previously (Example 5, 5.1.7.1). Emission wavelength was set to 370 nm and excitation wavelength was set to 460 nm with slit width set to 20. Gain was set to 73 and measuring height was set to 12 mm. Measuring intervals were 60 seconds over the duration of two hours. The plates used were the medium binding FLUOTRAC^{™} 200 96W Microplates from Greiner Bio-One.

### 5.2 Biochemical methods

### 5.2.1 Papain preparation

### 5.2.1.1 Size exclusion filtration with Sephadex G-25

Sephadex G-25 (medium size particles) was purchased from Sigma Aldrich (Munich, Germany). Separation of papain and smaller molecules was achieved via size exclusion filtration with self-made Sephadex G-25 columns. Preparation of Sephadex was done in 50 mL falcon tubes, wherein 15 mL of solid Sephadex was swelled in 40 mL of 0.05 M phosphate buffer pH 7 with NaCl (0.15 M) at room temperature overnight. 5 mL of Sephadex suspension was then poured into fritted 5 mL Syringes and centrifuged at 4000 rpm (2665 g) for 3 min in a swing bucket centrifuge. After discarding the eluted buffer, the columns were ready for immediate use with a column volume of 4 mL.

### 5.2.1.2 Reversible inactivation of papain to yield latent papain

Papain from papaya latex was purchased in several batches from Sigma Aldrich (Munich, Germany) as a buffered aqueous solution as well as lyophilized powder. The lyophilized powder was used for the first experiments, but all described papain batches in this work were synthesized using the buffered papain solutions. All steps were executed at room temperature.

Part of the crude papain was diluted in approx. 500 µL of 0.1 M phosphate buffer pH 7 and the concentration was determined via NanoDrop (see Example 5, 5.1.6). Once the clear papain solution showed a concentration of around 4.5 mg/ mL, 10 µL of a cysteine solution was added (0.1 M in phosphate buffer pH 7, 2 mM final conc.) and the mixture was shaken for 15 min at 500 rpm in order to completely activate papain. Excess of cysteine was removed via size exclusion filtration through 2 x 4 mL Sephadex G-25 (see Example 5, 5.2.1.1) columns using a swing bucket centrifuge (3 min, 4000 rpm, 2665 g). Subsequently two solutions were combined in order to prepare the reaction buffer. Solution 1 containing EDTA (0.1 M, 300 µL) together with KCI (1 M, 1.5 mL) and phosphate buffer (0.1 M, pH 7, 13.2 mL) was degassed with argon for 15 min. Solution 2 contained the specific thiosulfonate derivative (53 µmol) in 100 µL DMSO. The combined solutions gave the reaction buffer with the final concentrations of 3.5 mM thiosulfonate derivative, 2 mM EDTA and 0.1 M KCI. 500 µL of papain eluate and 500 µL of reaction buffer (resulting in approx. 20-fold excess of thiosulfonate) were then mixed and shaken over night at 500 rpm. The concentration of papain was again determined via NanoDrop. A solution of the irreversible papain inhibitor E64 (1 mM in DMSO) was added to the reaction mixture in equimolar proportion with respect to papain, in order to ensure there are no traces of active papain. The reaction mixture was incubated for 45 min at 500 rpm. The excess of thiosulfonate as well as E64 were removed by 4 x 4 mL Sephadex-G25 columns as described above. The concentration was again determined and the papain solution was further diluted if necessary, to achieve a final concentration of around 0.5 mg/mL. The stock solution was then aliquoted and stored at -80 °C.

Three different variations for papain inactivation were applied with minor modifications between experiments within the course of this work. Methods will be annotated for each experiment discussed in the corresponding results chapter (see Example 1, 1.1). The three main methods were as follows:
**Papain inactivation method 1:** As described
**Papain inactivation method 2:** No cysteine and no E64
**Papain inactivation method 3:** As described but 2 nmol E64 instead of equimolar amounts

### 5.2.1.3 Determination of papain concentrations, active site titration with E64

In order to determine a suitable starting dilution for each papain titration, initial NanoDrop measurements were good reference points and further approaches were accomplished by trial and error. Determination of the concentration of actually functional enzyme was thus initialized by diluting the papain solution with 0.1 M phosphate buffer (pH 7). Then 75 µL of the diluted papain was mixed and incubated with 300 µL of 0.1 M phosphate pH 7 activation buffer (8 mM cysteine, 0.1% Brij35, 4 mM EDTA) and shaken for 15 min at 300 rpm. Meanwhile a concentration series of E64 in well buffer (0.1 M phosphate buffer pH 7, 4 mM EDTA, 0.01% Brij35) was prepared in 8 reaction tubes (the E64 stock solution was stored at -20 °C as a 10 µM solution in 0.1 M phosphate pH 7 buffer) following **Table 8.** 100 µL of each reaction tube was transferred into an 8 x PCR strip and from there, 30 µL of each mixture was transferred into a second PCR strip. After the 15 min incubation time of papain, a third PCR strip was prepared and 40 µL of the papain solution was transferred to each tube. 30 µL of each tube was then transferred onto the prepared PCR strip containing each 30 µL of the E64 solutions. The reaction mixture was incubated at room temperature for 30 min. Meanwhile a 200 µM substrate solution in water was prepared using the 5 mM stock solution of Z-Phe-Arg-AMC in DMSO (stock solution was stored at -20 °C). After mixing vigorously, the solution was transferred to a PCR strip with 60 µL in each tube. After the 30 min incubation time of papain and E64, the 96 well plate was prepared for a triplicate measurement. 371.2 µL of well buffer was transferred in 3 x 8 wells each. Then 5 µL of each papain/E64 mixture was transferred into every well. After mixing, the assay was started by adding 3.8 µL of the prepared AMC substrate solution to each well and mixing. The fluorescence increase was tracked with an Enspire Multimode Fluorescence Plate Reader (see Example 5, 5.1.7.1) over a time of 30 min with 30 sec intervals. The slope of the initial linear region was calculated (corresponding to the reaction rate) and plotted against the concentration. With a linear fit through the given points and factoring in the dilution factors, the actual concentration of the papain stock solution could be determined.

Four different variations of determining papain concentration were used with minor differences in the course of this work. The different methods were as follows:
**Titration method 1:** As described
**Titration method 2:** Additional 1 mM cysteine in final well buffer
**Titration method 3:** E64 concentrations twice as high (0 - 400 nM), Triton X-100 instead of Brij35
**Titration method 4:** Triton X-100 instead of Brij35

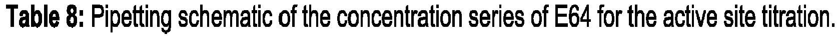

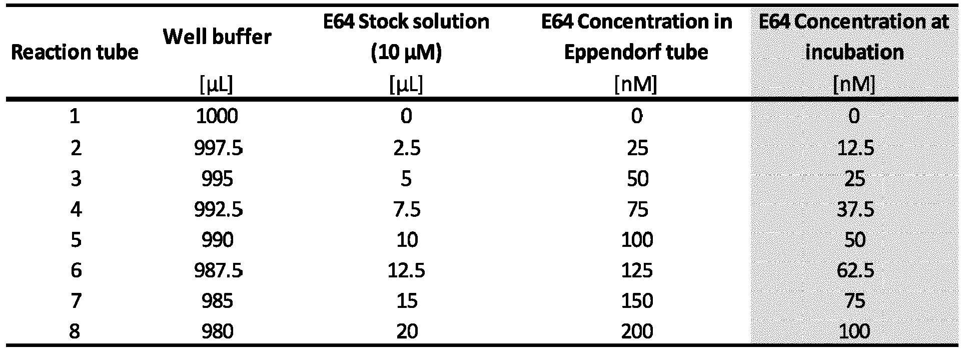

### 5.2.2 Fluorometric papain reactivation assays

### 5.2.2.1 Reduction of selenocysteine and cysteine

Due to the higher oxidizability of selenols compared to thiols, free selenocysteine is not readily available. Reduction of selenocystine is therefore required shortly before usage and realized most efficiently through TCEP. First, a solution of 50 mM TCEP in 0.1 M Tris-HCl buffer pH 8 with additional 150 mM NaOH was prepared. Adding the appropriate volume to solid weighed selenocystine yielded a concentration of 10 mM selenocystine. The dimer is not soluble in the aqueous buffer, though after 30 min of mixing at 1000 rpm at room temperature, reduction of the diselenide through TCEP resulted in a clear solution of 20 mM water soluble selenocysteine. The 20 mM selenocysteine stock solution was then diluted further for use in papain reactivation assays. Cystine was reduced in the same manner and durations of both reductions were compared.

### 5.2.2.2 Papain reactivation with dilution series of activators

Different assays of reactivating latent papain in solution were conducted in the course of this work. Various buffer compositions, supplements, 96 well plates, substrates, activators, concentrations as well as different latent papain forms were compared. In order to get an overview of all experiments, a standard method is described in the following and variations of this method are subsequently defined. First, the well buffer was prepared (0.1 M phosphate buffer pH 7, 4 mM EDTA, 0.01% Brij35). A 1 mM TCEP solution was prepared with 3 mM NaOH in 0.1 M Tris-HCl buffer pH 8. Papain substrate (Z-Phe-Arg-AMC or Suc-Leu-Leu-Val-Tyr-AMC) was prepared as a 200 µM solution in water and transferred equally to a PCR strip. Three serial dilutions of the activating agent with well buffer were used in the different experiments and the corresponding pipetting schemes are shown in **Table 9.** Papain was diluted to 0.1 µM with well buffer and transferred equally to a PCR strip. After preparation of the abovementioned solutions, TCEP (1 mM solution) was added to well buffer to yield a final TCEP concentration of 1.1 µM. Finally, the 96 well plate (different plates see Example 5, 5.1.7) was prepared. 342.4 µL of TCEP containing well buffer was transferred to each well, followed by 3.8 µL papain solution. After mixing, 30 µL of the prepared activator concentrations (see **Table 9)** were added and mixed. In the last step, 3.8 µL of the substrate solution was added and mixed, to yield a final well composition of 0.1 M phosphate buffer pH 7, 1 nM papain, 2 µM substrate, activator (see **Table 9)**, 4 mM EDTA, 0.01% Brij35 and 1 µM TCEP. For experiments with only one activator concentration of 10 µM, the transferred well buffer for each well was 368.6 µL, followed by 3.8 µL of papain, substrate and activator (1 mM in well buffer) respectively. The fluorescence increase was tracked with an Enspire Multimode Fluorescence Plate Reader (see Example 5, 5.1.7.1).

Some experiments for analysis of the papain background activity (of residual active papain) were adopted without any activator present. The major differences of the fluorometric methods were as follows:
**Fluorometric method 1:** As described
**Fluorometric method 2:** No TCEP
**Fluorometric method 3:** Different surfactants
**Fluorometric method 4:** No TCEP, different or no surfactant
**Fluorometric method 5:** Different TCEP concentrations

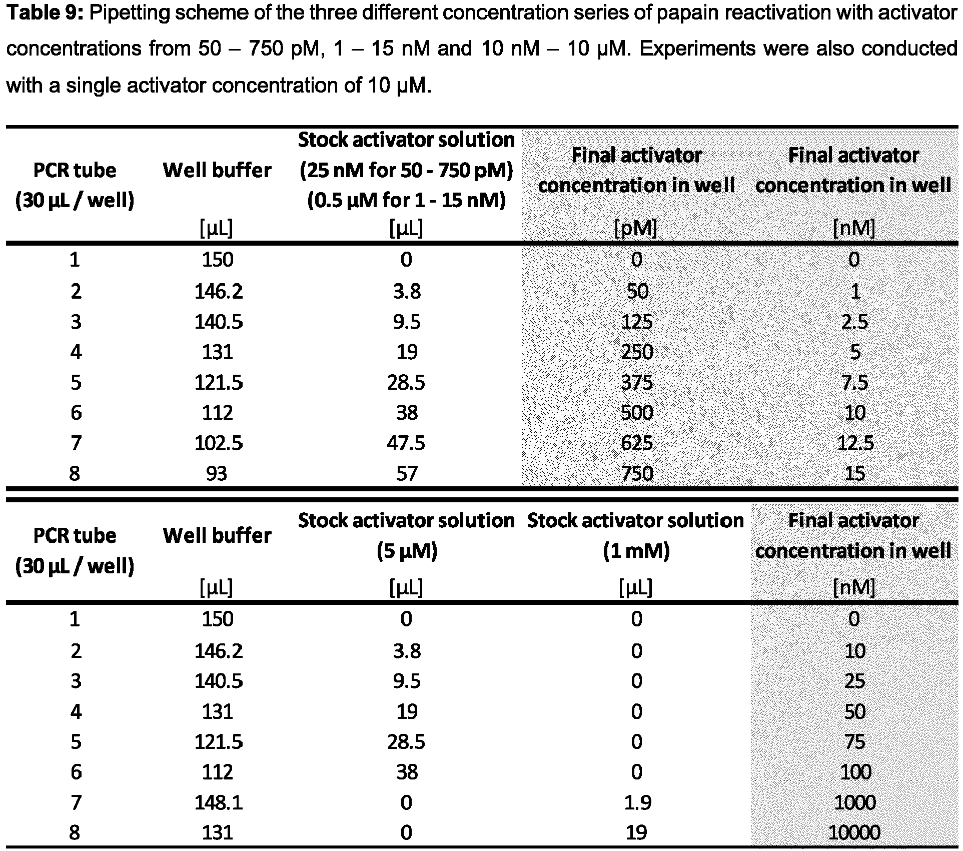

### 5.2.3 Copper-free ring-strain promoted click reaction

### 5.2.3.1 Click reaction of DBCO containing DNA and azide containing peptides in solution

In order to yield the DNA-peptide-hybrid which can reactivate papain, the dUTP-DBCO containing amplificate (see Example 5, 5.3.1.1) had to be fused to the different papain activators through copper-free strain-promoted [3+2] azide-alkyne cycloaddition click chemistry. For all assays with 75% of dUTP-DBCO and 25% of dTTP nucleotides, 50 µL of PCR reaction mixture contained 7.5 nmol (150 µM) of the DBCO nucleotide (see Example 5, 5.3.1.1). Since the click reaction progresses rapidly at this concentration, an equimolar amount of either azido-PEG₃-ULR **(15)** or azido-amino-PEG₃-ULR **(16)** was used by adding 1.88 µL of the 4 mM peptide stock solution to the 50 µL PCR mixture. After shaking the reaction mixture at room temperature and 300 rpm for 45 min, the clicked DNA-peptide hybrid was ready for purification and usage (see Example 5, 5.2.4).

### 5.2.4 Utilization of fluorometric papain reactivation assays

### 5.2.4.1 Papain reactivation with immobilized biotinylated DNA-peptide-hybrids

Different assays of reactivating latent papain through DNA-bound activator were performed in the course of this work. Different DNA-sequences were amplified (see Example 5, 5.3) and different processing and purification methods were utilized (see Example 5, 5.3.4). The azido-PEGs-ULR peptide dimer **(15)** and azido-amino-PEG₃-ULR peptide dimer **(16)** was coupled (see Example 5, 5.2.3.1), yielding slightly different DNA-linked activators via copper-free-click chemistry (see Example 5, 5.2.3). The preceding processing and amount of DNA varied between assays.

First, well buffer was prepared (0.1 M phosphate buffer pH 7, 4 mM EDTA, 0.01% Brij35). A 1 mM TCEP solution was prepared with 3 mM NaOH in 0.1 M Tris-HCl buffer pH 8. Z-Phe-Arg-AMC was prepared as a 200 µM solution in water and transferred equally to a PCR strip. The streptavidin coated 96 well plate (see Example 5, 5.1.7) was initially washed twice with 380 µL well buffer per well. Biotinylated DNA-peptide-hybrid activator (volume range of 0.5 µL - 50 µL) as well as negative control DNA without DBCO and bound activator (same volumes) was diluted in a total volume of 300 µL and transferred into the well. Immobilization was achieved by shaking the plate at room temperature and 300 rpm for 30 min. Afterwards the reaction mixture was discarded and the plate was washed 3 times with 380 µL well buffer. Papain was then diluted from the corresponding stock solution to a 0.1 µM solution with well buffer and transferred equally to a PCR strip. Well buffer (+ 1 µM TCEP, 372.4 µL) was transferred into each well, after which 3.8 µL of papain solution (0.1 µM) was added and mixed. Through addition of 3.8 µL substrate and mixing, the assay was started. The fluorescence increase was tracked with an Enspire Multimode Fluorescence Plate Reader (see Example 5, 5.1.6.1).

### 5.2.4.2 Papain reactivation in accordance with the method of the invention

Different assays of reactivating latent papain through hybridization of DNA-peptide-hybrid and immobilized biotinylated-DNA probe (see Example 5, 5.3) were performed in the course of this work. Different DNA-sequences were amplified (see Example 5, 5.3) and different processing and purification methods were utilized (see Example, 5, 5.3.4). The azido-PEGs-ULR peptide dimer (15) and azido-amino-PEG₃-ULR peptide dimer **(16)** were coupled to DNA containing DBCO, yielding slightly different DNA-linked activators via copper-free-click chemistry. The buffer composition and amount of applied DNA varied between different assays. The biotinylated-DNA probe remained the same in each experiment. In order to get an easy and accessible overview of all experiments, a standard method is described in the following and the varying details are annotated for each experiment discussed in the results chapter (see Example 3, 3.4).

Buffer system 1: Well buffer for every step, 0.1 M phosphate pH 7, 4mM EDTA and 0.01% Brij35.

Buffer system 2: Different buffers used for each step. Denaturing solution: 100 mM NaOH and 0.1% Tween. Hybridization buffer: 300 mM NaCl, 100 mM Tris-HCl pH 6.5, 10 mM EDTA, 0.1% Tween. Washing buffer: 150 mM NaCl, 100 mM Tris-HCl pH 7.5, 0.1% Tween. Measurement was conducted in well buffer (+ 1µM TCEP).

First, well buffer was prepared (0.1 M phosphate buffer pH 7, 4 mM EDTA, 0.01% Brij35). A 1 mM TCEP solution was prepared with 3 mM NaOH in 0.1 M Tris-HCl buffer pH 8. Z-Phe-Arg-AMC was prepared as a 200 µM solution in water and transferred equally to a PCR strip. 1 - 5 µL of the amplified, clicked and processed DNA was combined with 10 µL of denaturing solution and incubated at room temperature for 10 min. Meanwhile, 2 µL of the biotinylated DNA-probe (1 pmol/µL, see Example 5, 5.3) was dissolved in 280 µL hybridization buffer and after 10 min combined with the denatured DNA amplificate. The mixture was then heated to 60 °C and shaken for 30 min. The streptavidin coated plate was washed twice with 380 µL washing buffer per well. After 30 min the DNA mixture was transferred into the wells and immobilized at room temperature for 30 min while shaking the plate at 300 rpm. After discarding the DNA solution, washing the plates was achieved with 4 x 380 µL washing buffer per well. Papain was then diluted from the corresponding stock solution to a 0.1 µM solution with well buffer and transferred equally to a PCR strip. The plate was prepared for measurement. Well buffer (+ 1µM TCEP, 372.4 µL) was transferred into each well, after which 3.8 µL of papain solution (0.1 µM) was added and mixed. Through addition of 3.8 µL substrate and mixing, the assay was started. The fluorescence increase was tracked with an Enspire Multimode Fluorescence Plate Reader (see Example 5, 5.1.6.1). The main differences of the fluorometric methods were as follows:
**Probe method 1:** As described
**Probe method 2:** Well buffer for every step
**Probe method 3:** Replacing heating step of 30 min at 60 °C for gradually heating to 95 °C and cooling down to room temperature within 30 min on a thermomixer. Well buffer for every step
**Probe method 4:** Same as probe method 3 with additional separation of excess peptide via sephadex purification

### 5.3 Molecular biological methods

10x GenTherm buffer without MgCl₂ (Rapidozym GmbH) for usage with the Taq polymerase, GoTaq polymerase and corresponding buffer (Promega), Q5 polymerase and corresponding buffer (New England Biolabs NEB), Phusion polymerase and corresponding buffer (Thermo Fisher Scientific), 5-DBCO-PEG₄-dUTP (Jena Bioscience, also referred to as dUTP-DBCO), 1 kb plus DNA ladder (Thermo Fisher Scientific), TriTrack DNA Loading Dye (6X) (Thermo Fisher Scientific), Ethidium Bromid Solution (Carl Roth), Mobicol spin columns M2110 and M1003 (Mobitec GmbH), Wizard^{®} SV Gel and PCR Clean-Up System (Promega), Micro Bio-Spin^{®} Columns with Bio-Gel^{®} P-30 (Bio-Rad), Quick Spin Columns with Sephadex G-50 (Roche), pcDNA3-SARS-CoV-2-S-RBD-sfGFP plasmid (Addgene), primers and probe (Integrated DNA Technologies). DNA templates, DNA amplificants, corresponding primers and their sequences are shown in **Tables 10-11.**

**Table 10: Nucleotide sequences of DNA templates and PCR amplificates. In all DNA experiments, the SARS-plasmid was used as template directly. (see Example 3, 3.4).**

| **DNA template ID** | **Sequence (5' - 3' direction)** | **Nucleotides** | **Comments** |
|---|---|---|---|
| **SARS-CoV-2-S-RBD-sfGFP plasmid** | CAGAGCTCTCTGGCTAACTAGAGAACCCACTGCTTACTGGCTTATCGAAATTAATACGAC TCACTATAGGGAGACCCAAGCTGGCTAGCGCCACCATGAAGACCATCATCGCCCTGAG CTACATCTTCTGCCTGGTGTTCGCCACTAATCTTTGTCCGTTCGGTGAGGTTTTTAACGC GACAAGGTTCGCTAGTGTATATGCTTCGAACCGAAAGAGAATCTCCAATTGCGTAGCTG ATTACTCCGTTCTCTATAACAGTGCGTGCTTTTTCAACCTTGAAGTGTTACGGCGTTTCTCC AACGAAGCTGAATGATCTCTGTTTTACGAACGTGTAAGCTGACTCTTTCGTTATACGAGG GGACGAAGTGAGCAGATAGCAGCAGGTCAGACTGGGAAGATAGCGGATTACAACTAT AAGTTGGCCGATGATTTTACGGCGGTGCGTAATCGCATGGAACTCAAACAACCTCGACTC CAAAGTAGGTGGTAATTATAATTACTTGTATCGCCTGTTTCGAAAGAGCAATTTGAAGCC TTTTGAGCGGGATATTTCAACCGAAATTTACCAAGCAGGCAGTACGCCATGTAACGGAG TAGAGGGATTTAATTGCTACTTTCCTCTTCAATCTTATGGCTTTCAACCAACAAACGGAGT GGGGTATCAACCTTATAGAGTGGTAGTATTGTCCTTTGAGCTCCTCCACGCCGCCGGCTA CAGTTTGTGGGCCCAAAAAGGGATCCGGTGGGTCCGGTAGTGGTCGGTTCCAAAGGAGA AGAACTTTTCACTGGAGTTGTCCCAATTCTTGTTGAATTAGATGGTGATGTTAATGGGCA CAAATTTTCTGTCCGTGGAGAGGGTGAAGGTGATGCTACAAACGGAAAACTCACCCTTA AATTTATTTGCACTACTGGAAAACTACCTGTTCCATGGCCAACACTTGTCACTACTCTGAC CTATGGTGTTCAATGC | **968** | **Full ORF sequence of SARS-CoV-2 protein S (spike) fused to sfGFP with T7 promotor sequence** |

| **PCR product ID** | **Sequence (5'- 3' direction)** | **Nucleotides** | **Comments** |
|---|---|---|---|
| **BiotinylatedT7-Spike DNA** | bto-TAATACGACTCACTATAGGGAGACCCAAGCTGGCTAGCGCCACCATGAAGACCAT CATCGCCCTGAGCTACATCTTCTGCCTGGTGTTCGCCACTAACTTTGTGCCGTTCGGTGA GGTTTTTAACGCGACAAGGTTCGCTAGTCTATATGCTTGGAACCGAAAGAGAATCTCCA ATTGCGTAGCTCATTACTCCGTTCTCGATAACAGTGCGTGCTTTTCAACCTTTAAGTGTTA CGGCGTTTCTCCAACGAAGCTGAATGATCTCTGTTTTACGAACGTGTATGCTGACTCTTT CGTTATACGGGGGCGACGAAGTGAGACAGATAGCACCAGGTCAGACTGGGAAGATAGC GGATTACAACTATAAGTTACCCGATGATTTTACGGGCTGCGTAATCGCATGGAACTCAA ACAACCTCGACTCCAAAGTAGGTGGTAATTATAATTACTTGTATCGCCTGTTTCGAAAGA GCAATTTGAAGCCTTTTGAGCGGGATATTTCAACCGAAATTTACCAAGCAGGCAGTACG CCATGTAACGGCAGTAGAGGGATTTAATTGCTACTTTCCTCTTCAGCTTATGGCTTTCAAC CAACAAACGGAGTGGGGTATCAACCTTATAGAGTGGTAGTATTGTCCTTTCAGCTCCTC CACGCCCCGGCTACAGTTTGTGGGACCAAAAAGCGATCCGGTGGGTCCGGTAGTGGTG GTTCC | **713** | **Biotin T7 forward + Spike reverse primer product, 373 passible dUTP-DB CO per double strand, biotinylated on 5'** |
| **Spike DNA** | ATGAAGACCATCATCGCCCTGAGCTACATCTTCTGCCTGGTGTTCGCCAACATCACTAACTTGTGT CCGTTCGGTGAGGTTTTTAACGCGACCAGGTTCGCTAGTGTATATGCTTGGAACCGAAA GAGAATCTCCAATTGCGTAGCTGATTACTCCGTTCTCTATAACAGTGCGTCCTTTTCAACC TTTAAGTGTTACGGCGTTTCTCCAACGAAGCTGAATGACCTCTGTTTTACGAACGTGTAT GCTGACTCTTTCGTTATACGGGGGGACGAAGTGAGACAGATAGCACCAGGTCAGACTG GGAAGATAGCGGATTACAACTATAAGTTGCCCGATGATTTTACGGGGTGCGTAATCGCA TGGAACTCAAACAACCTCGACTCCAAAGTAGGTGGTAATTATAATTACTTGTATCGCCTG TTTCGAAAGAGCAATTTGAAGCCTTTTGAGCGGGATATTTCAACCGAAATTTACCAAGCA GGCAGTACGCCATGTAACGGAGTAGAGGGATTTAATTGCTACTTTCCTCTTCAATCTTAT GGCTTTCAACCCACAAACGGAGTGGGGTATCAACCTTATAGAGTGGTAGTATTGTCCTTT GAGCTCCTCCACGCCCCGGCTACAGTTTGTGGGCCCAAAAAGGGATCCGGTGGGTCCG GTAGTGGTGGTTCC | **669** | **Spike forward + reverse primer product, 355 possible dUTP-DBCO per double strand** |

**Table 11: Nucleotide sequences of primers and probes. Tₘ for usage with Taq was calculated via the "Thermofischer Tₘ Calculator" tool.**

| **Primer ID** | **Sequence (5' - 3' direction)** | **Nucleotides** | **Tₘ[°C]** | **Comments** |
|---|---|---|---|---|
| **Biotin T7 forward** | **bio-TAATACLGACTCACTATAGGG** | **20** | **49** | **primer for T7 promoter, biotinylated on 5 , T7 promotor is in front of spike gene** |
| **Spike forward** | **ATGAAGACCATCATCGC** | **17** | **51,2** | **start of spike gene** |
| **Spike reverse** | **GGAACCACCACTACC** | **15** | **51,8** | **end of spike gene** |

### 5.3.1 Polymerase chain reaction

Polymerase chain reaction (PCR) was used to amplify specific DNA templates and incorporate the DBCO containing modified dUTP nucleotides. Amplification was achieved in 50 µL reaction volumes with a MJ Research PTC-200 Thermal Cycler as well as a Bio-Rad T100 Thermal Cycler.

### 5.3.1.1 PCR with Taq Polymerase

The Taq polymerase was used for the incorporation of dUTP-DBCO in all following experiments. A standard PCR program and reaction mixture with 0% and 75% dUTP-DBCO content was defined for this polymerase with only minor changes between experiments regarding annealing temperatures as well as extension durations, depending on the different primers and templates used (see Example 5, 5.3). The composition of the reaction mixtures as well as the corresponding standard PCR program is listed in **Table 12.** After adding polymerase, the reaction mixture was immediately transferred to the PCR cycler and amplified. The reaction mixtures were then prepared for further use and analysis via agarose gel.

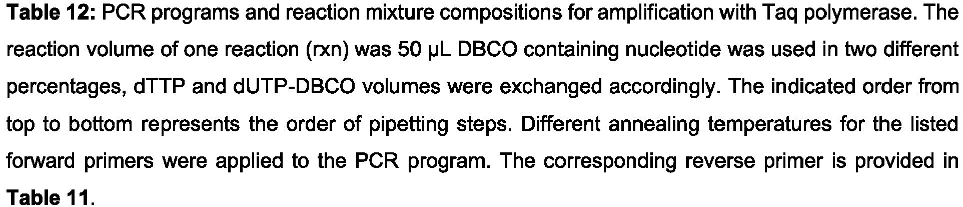

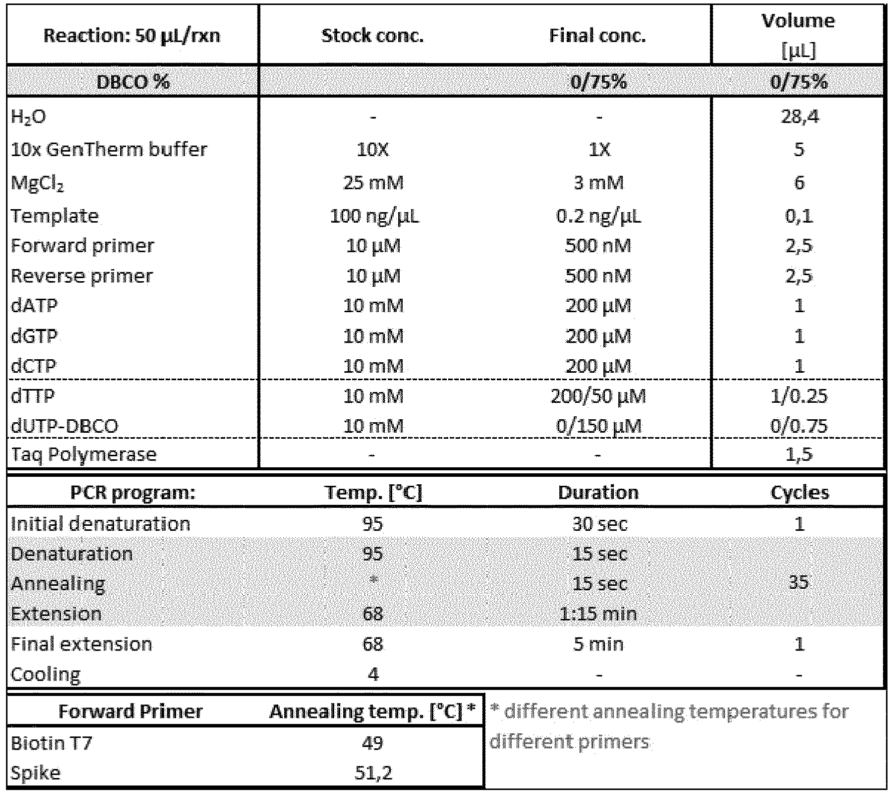

### 5.3.2 Agarose gel electrophoresis

Standard agarose gel electrophoresis was applied for the analysis of PCR amplificates. After assembling the gel chamber and chassis, 1.6 g agarose was mixed with 80 mL of 1X TBE buffer pH 8 (89 mM Tris-Borate, 2 mM EDTA) to yield a 2% gel mixture. The agarose was slowly dissolved by carefully heating the solution to the boiling point in a microwave oven. After cooling down to ≈ 60°C, one drop of ethidium bromide solution (250 µg/mL, 0.025%) was added to the gel. The gel was mixed and then poured into the prepared chassis. After further cooling and solidification, the chassis was transferred into the chamber, which in turn was subsequently filled with 1X TBE buffer until the gel was fully submerged. The amplified DNA (5 µL DNA + 1 µL TriTrack DNA Loading Dye (6X) as well as the specific DNA ladder (5 µL 1 kb plus DNA ladder) was then loaded into the gel pockets. The gel was run at 120 V for 45 - 50 min, after which a picture was taken with an Alpha Innotech Alphalmager Image Analysis System.

### 5.3.3 Recovery of DNA from agarose gel via the "freeze and squeeze" method

In cases where the amplified DNA (0% DBCO content) and the amplified template DNA (from plasmid) needed to be recovered from the agarose gel, extraction via freeze and squeeze was applied. The desired DNA band was cut out of the gel, transferred to a prepared Mobicol spin filter column (M2110 + M1003, Mobitec GmbH) and subsequently frozen. The filter tubes were then centrifuged at 14 000 rpm (16 000 g) for 5 min at room temperature, after which the eluted volume was measured and 10% v/v of NaOAc buffer (3 M, pH 5.2) was added to the eluate, followed by adding 100% EtOH with 3x the combined volume of eluate and NaOAc buffer. The mixture was incubated for 30 min at -20 °C and centrifuged afterwards for 30 min at 14 000 rpm and 4 °C. After discarding the supernatant, the precipitated DNA pellet was washed with 1 mL of 75% EtOH and centrifuged again for one min at 14 000 rpm and 4 °C. The supernatant was discarded and the pellet was air dried for 10 min. The extracted DNA was gently dissolved in 10 µL nuclease-free water over the time of 5 min.

### 5.3.4 DNA purification methods following PCR

### G-50 Sephadex Quick Spin columns:

The Sephadex G-50 columns have a cut-off point of 72 bp. All steps were performed according to the kit manual. The column was inverted several times and then the top cap and after that the bottom tip was removed. The storage buffer was drained and the column centrifuged at 1 100 g in a swing bucket rotor for 2 min. After discarding the eluate, the 50 or 100 µL DNA sample was carefully loaded onto the center of the column. Centrifugation at 1 100 g in a swing bucket rotor for 4 min resulted in elution of the purified DNA in STE buffer (10 mM Tris-HCl, pH 7.5, 1 mM EDTA, 100 mM NaCl).

After purification, the DNA solutions were further analysed in fluorometric assays (see Example 5, 5.2.4.1) and the efficiency of the purification methods were thereby determined.

### Further references

Saiki, R. K. et al. Enzymatic amplification of beta-globin genomic sequences and restriction site analysis for diagnosis of sickle cell anemia. Science 230, 1350-1354; 10.1126/science.2999980 (1985).

Mullis, K. B. & Faloona, F. A. Specific synthesis of DNA in vitro via a polymerase-catalyzed chain reaction. Methods in Enzymology 155, 335-350; 10.1016/0076-6879(87)55023-6 (1987).

Kwok, S. et al. Identification of human immunodeficiency virus sequences by using in vitro enzymatic amplification and oligomer cleavage detection. Journal of virology 61, 1690-1694; 10. 1 128/JVI.61.5.1690- 1694.1987 (1987).

Saiki, R. K. et al. Primer-directed enzymatic amplification of DNA with a thermostable DNA polymerase. Science 239, 487-491; 10.1126/science.239.4839.487 (1988).

Sachse, K. Specificity and performance of diagnostic PCR assays. Methods in Molecular Biology 216, 3-29; 10.1385/1-59259-344-5:03 (2003).

Heid, C. A., Stevens, J., Livak, K. J. & Williams, P. M. Real time quantitative PCR. Genome research 6, 986-994; 10.1101/gr.6.10.986 (1996).

Espy, M. J. et al. Real-time PCR in clinical microbiology: applications for routine laboratory testing. Clinical microbiology reviews 19, 165-256; 10.1128/CMR.19.1.165-256.2006 (2006).

Deepak, S. et al. Real-time PCR: Revolutionizing detection and expression analysis of genes. Current Genomics 8, 234-251; 10.2174/138920207781386960 (2007).

Corman, V. M. et al. Detection of 2019 novel coronavirus (2019-nCoV) by real-time RT-PCR. Eurosurveillance 25; 10.2807/1560-7917.ES.2020.25.3.2000045 (2020).

Engvall, E. & Perlmann, P. Enzyme-linked Immunosorbent assay, ELISA. III. Quantitation of specific antibodies by enzyme-labeled anti-immunoglobulin in antigen-coated tubes. The Journal of Immunology 109, 129-135 (1972).

Birch, J. R. & Racher, A. J. Antibody production. Advanced Drug Delivery Reviews 58, 671-685; 10.1016/j.addr.2005.12.006 (2006).

Rodrigues, M. E., Costa, A. R., Henriques, M., Azeredo, J. & Oliveira, R. Technological progresses in monoclonal antibody production systems. Biotechnology Progress 26, 332-351; 10.1002/btpr.348 (2010).

Stills, H. F. Polyclonal antibody production. In The laboratory rabbit, guinea pig, hamster, and other rodents, edited by M. A. Suckow, K. Stevens & R. Wilson (Academic, Oxford, 2012), pp. 259-274.

Dhamad, A. E. & Abdal Rhida, M. A. COVID-19: molecular and serological detection methods. PeerJ 8, e10180; 10.7717/peerj.10180 (2020).

Coutlée, F., Bobo, L., Mayur, K., Yolken, R. H. & Viscidi, R. P. Immunodetection of DNA with biotinylated RNA probes: A study of reactivity of a monoclonal antibody to DNA-RNA hybrids. Analytical Biochemistry 181, 96-105; 10.1016/0003-2697(89)90399-0 (1989).

Menotti, J. et al. Evaluation of a new 5'-nuclease real-time PCR assay targeting the Toxoplasma gondii AF146527 genomic repeat. Clinical Microbiology and Infection 16, 363-368; 10.1111/j.1469-0691.2009.02809.x (2010).

Hairul Aini, H., Omar, A. R., Hair-Bejo, M. & Aini, I. Comparison of Sybr Green I, ELISA and conventional agarose gel-based PCR in the detection of infectious bursal disease virus. Microbiological Research 163, 556-563; 10.1016/j.micres.2006.08.003 (2008).

Cosan, D. T. et al. Effects of various agents on DNA fragmentation and telomerase enzyme activities in adenocarcinoma cell lines. Molecular Biology Reports 38, 2463-2469; 10.1007/s11033-010-0382-x (2011).

Raji, N., Sadeghizadeh, M., Tafreshi, K. N. & Jahanzad, E. Detection of human Papillomavirus 18 in cervical cancer samples using PCR-ELISA (DIAPOPS). Iranian Journal of Microbiology 3, 177-182 (2011).

Chan, P. J., Seraj, I. M., Kalugdan, T. H. & King, A. Prevalence of mycoplasma conserved DNA in malignant ovarian cancer detected using sensitive PCR-ELISA. Gynecologic Oncology 63, 258-260; 10.1006/gyno.1996.0316 (1996).

Yie, S.-M., Luo, B., Ye, N.-Y., Xie, K. & Ye, S.-R. Detection of Survivin-expressing circulating cancer cells in the peripheral blood of breast cancer patients by a RT-PCR ELISA. Clinical & Experimental Metastasis 23, 279-289; 10.1007/s10585-006-9037-7 (2006).

Hadrich, I. et al. Comparison of PCR-ELISA and Real-Time PCR for invasive aspergillosis diagnosis in patients with hematological malignancies. Medical Mycology 49, 489-494; 10.3109/13693786.2010.540724 (2011).

Badiee, P. et al. Study on invasive fungal infections in immunocompromised patients to present a suitable early diagnostic procedure. International Journal of Infectious Diseases 13, 97-102; 10.1016/j.ijid.2008.04.011 (2009).

Löffler, J. et al. Detection of PCR-amplified fungal DNA by using a PCR-ELISA system. Medical Mycology 36, 275-279; 10.1080/02681219880000441 (1998).

Bagheri, R. et al. PCR-ELISA: A diagnostic assay for identifying Iranian HIV seropositives. Molecular Genetics, Microbiology and Virology 28, 127-131; 10.3103/S0891416813030026 (2013).

Tahk, H. et al. Development of reverse transcriptase polymerase chain reaction enzyme-linked immunosorbent assay for the detection of hepatitis A virus in vegetables. Food Control 23, 210-214; 10.1016/j.foodcont.2011.07.012 (2012).

Tahk, H., Lee, M. H., Lee, K. B., Cheon, D.-S. & Choi, C. Development of duplex RT-PCR-ELISA for the simultaneous detection of hepatitis A virus and hepatitis E virus. Journal of Virological Methods 175, 137-140; 10.1016/j.jviromet.2011.04.030 (2011).

Bazzichi, A., Vigna Guidi, F., Rindi, L., Incaprera, M. & Garzelli, C. PCR ELISA for the quantitative detection of Epstein-Barr virus genome. Journal of Virological Methods 74, 15-20; 10.1016/S0166-0934(98)00060-3 (1998).

Zhou, Y.-C. et al. A rapid and accurate detection approach for multidrug-resistant tuberculosis based on PCR-ELISA Microplate Hybridization Assay. Laboratory Medicine 51, 606-613; 10.1093/labmed/lmaa016 (2020).

Daeschlein, G., Assadian, O., Daxboeck, F. & Kramer, A. Multiplex PCR-ELISA for direct detection of MRSA in nasal swabs advantageous for rapid identification of non-MRSA carriers. European Journal of Clinical Microbiology & Infectious Diseases 25, 328-330; 10.1007/s10096-006-0131-1 (2006).

St-Louis, M. PCR-ELISA for high-throughput blood group genotyping. Methods in Molecular Biology 496, 3-13; 10.1007/978-1-59745-553-4_1 (2009).

Gill, P. et al. Detection of four beta-thalassemia point mutations in Iranians using a PCR-ELISA genotyping system. Molecular and Cellular Probes 22, 103-109; 10.1016/j.mcp.2007.10.001 (2008).

Chansiri, K., Khuchareontaworn, S. & Sarataphan, N. PCR-ELISA for diagnosis of Trypanosoma evansi in animals and vector. Molecular and Cellular Probes 16, 173-177; 10.1006/mcpr.2002.0412 (2002).

Kobets, T. et al. Leishmania parasite detection and quantification using PCR-ELISA. Nature Protocols 5, 1074-1080; 10.1038/nprot.2010.68 (2010).

Medeiros, F. A. C. et al. Development and validation of a PCR-ELISA for the diagnosis of symptomatic and asymptomatic infection by Leishmania (Leishmania) infantum. Journal of Tropical Medicine 2017, 7364854; 10.1155/2017/7364854 (2017).

Al Dahouk, S., Tomaso, H., Nöckler, K. & Neubauer, H. The detection of Brucella spp. using PCR-ELISA and real-time PCR assays. Clinical Laboratory 50, 387-394 (2004).

Daly, P., Collier, T. & Doyle, S. PCR-ELISA detection of Escherichia coli in milk. Letters in Applied Microbiology 34, 222-226; 10.1046/j.1472-765x.2002.01074.x (2002).

Perelle, S. et al. Comparison of PCR-ELISA and LightCycler real-time PCR assays for detecting Salmonella spp. in milk and meat samples. Molecular and Cellular Probes 18, 409-420; 10.1016/j.mcp.2004.07.001 (2004).

Liu, G. et al. Liquid-phase hybridization based PCR-ELISA for detection of genetically modified organisms in food. Food Control 15, 303-306; 10.1016/S0956-7135(03)00081-1 (2004).

Laitinen, R., Malinen, E. & Palva, A. PCR-ELISA I: Application to simultaneous analysis of mixed bacterial samples composed of intestinal species. Systematic and Applied Microbiology 25, 241-248; 10.1078/0723-2020-00118 (2002).

Gill, P., Forouzandeh, M., Rahbarizadeh, F., Ramezani, R. & Rasaee, M. J. Production of anti-digoxigenin antibody HRP conjugate for PCR-ELISA DIG detection system. Journal of Immunoassay & Immunochemistry 27, 303-318; 10.1080/15321810600861910 (2006).

Goggins, S. & Frost, C. G. Approaches towards molecular amplification for sensing. The Analyst 141, 3157- 3218; 10.1039/c6an00348f (2016).

Li, J. et al. Enzyme-based multi-component optical nanoprobes for sequence-specific detection of DNA hybridization. Adv. Mater. 20, 497-500; 10.1002/adma.200701918 (2008).

Saghatelian, A., Guckian, K. M., Thayer, D. A. & Ghadiri, M. R. DNA detection and signal amplification via an engineered allosteric enzyme. Journal of the American Chemical Society 125, 344-345; 10.1021/ja027885u (2003).

Rawat, A., Roy, :, Jyoti, A., Kaushik, S., Verma, K., Srivastava, V.K. Cysteine proteases: Battling pathogenic parasitic protozoans with omnipresent enzymes. Microbiological Research 249, 126784.

Rawlings, N. D. & Barrett, A. J. Families of cysteine peptidases. In Proteolytic enzymes: serine and cysteine peptidases, edited by A. J. Barrett (Acad. Press, San Diego, Calif., 1994), Vol. 244, pp. 461-486.

Mitchel, R. E.J., Chaiken, I. M. & Smith, E. L. The complete amino acid sequence of papain. Journal of Biological Chemistry 245, 3485-3492; 10.1016/S0021-9258(18)62954-0 (1970).

Novinec, M. & Lenarcic, B. Papain-like peptidases: structure, function, and evolution. Biomolecular Concepts 4, 287-308; 10.1515/bmc-2012-0054 (2013).

Fernández-Lucas, J., Castañeda, D. & Hormigo, D. New trends for a classical enzyme. Papain, a biotechnological success story in the food industry. Trends in Food Science & Technology 68, 91-101; 10.1016/j.tifs.2017.08.017 (2017).

Pickersgill, R. W., Harris, G. W. & Garman, E. Structure of monoclinic papain at 1.60 angstroms resolution. Acta Crystallographica 48, 59-67; 10.2210/pdb1ppn/pdb (1992).

Zimmerman, M., Yurewicz, E. & Patel, G. A new fluorogenic substrate for chymotrypsin. Analytical Biochemistry 70, 258-262; 10.1016/S0003-2697(76)80066-8 (1976).

Zimmerman, M., Ashe, B., Yurewicz, E. C. & Patel, G. Sensitive assays for trypsin, elastase, and chymotrypsin using new fluorogenic substrates. Analytical Biochemistry 78, 47-51; 10.1016/0003-2697(77)90006-9 (1977).

Morita, T., Kato, H., Iwanaga, S., Takada, K. & Kimura, T. New fluorogenic substrates for alpha-thrombin, factor Xa, kallikreins, and urokinase. Journal of Biochemistry 82, 1495-1498; 10.1093/oxfordjournals.jbchem.a131840 (1977).

Barrett, A. J. Fluorimetric assays for cathepsin B and cathepsin H with methylcoumarylamide substrates. Biochemical Journal 187, 909-912 (1980).

Tchoupé, J. R., Moreau, T., Gauthier, F. & Bieth, J. G. Photometric or fluorometric assay of cathepsin B, L and H and papain using substrates with an aminotrifluoromethylcoumarin leaving group. Biochimica et Biophysica Acta (BBA) - Protein Structure and Molecular Enzymology 1076, 149-151; 10.1016/0167-4838(91)90232-0 (1991).

Green & Sambrook, Molecular Cloning: A Laboratory Manual, 4th edition, New York, Cold Spring Harbor Laboratory Press, 2012 (ISBN: 978-1-936113-42-2).

Ménard, R. et al. A protein engineering study of the role of aspartate 158 in the catalytic mechanism of papain. Biochemistry 29, 6706-6713; 10.1021/bi00480a021 (1990).

Blomquist, A. T. & Liu, L. H. Many-mebered carbon rings. Journal of the American Chemical Society 75, 2153-2154; 10.1021/ja01105a039 (1953).

Georg Wittig & Adolf Krebs. Zur Existenz niedergliedriger Cycloalkine, I. Chemische Berichte 94, 3260-3275; 10.1002/cber.19610941213 (1961).

Agard, N. J., Prescher, J. A. & Bertozzi, C. R. A strain-promoted 3 + 2 azide-alkyne cycloaddition for covalent modification of biomolecules in living systems. Journal of the American Chemical Society 126, 15046- 15047; 10.1021/ja044996f (2004).

Baskin, J. M. et al. Copper-free click chemistry for dynamic in vivo imaging. Proceedings of the National Academy of Sciences of the United States of America 104, 16793-16797; 10.1073/pnas.0707090104 (2007).

Dommerholt, J., Rutjes, F. P. J. T. & van Delft, F. L. Strain-promoted 1,3-dipolar cycloaddition of cycloalkynes and organic azides. In Cycloadditions in bioorthogonal chemistry, edited by M. Vrabel & T. Carell (Springer, Cham, 2016), pp. 57-76.

Ning, X., Guo, J., Wolfert, M. A. & Boons, G.-J. Visualizing metabolically labeled glycoconjugates of living cells by copper-free and fast Huisgen cycloadditions. Angewandte Chemie International Edition in English 120, 2285-2287; 10.1002/ange.200705456 (2008).

Sletten, E. M. & Bertozzi, C. R. A hydrophilic azacyclooctyne for Cu-free click chemistry. Organic Letters 10, 3097-3099; 10.1021/ol801141k (2008).

Wu, P. & Fokin, V. V. Catalytic azide-alkyne cycloaddition: reactivity and applications. Chemlnform 38; 10.1002/chin.200736242 (2007).

Debets, M. F. et al. Aza-dibenzocyclooctynes for fast and efficient enzyme PEGylation via copper-free (3+2) cycloaddition. Chemical Communications 46, 97-99; 10.1039/b917797c (2010).

Jewett, J. C. & Bertozzi, C. R. Cu-free click cycloaddition reactions in chemical biology. Chemical Society Reviews 39, 1272-1279; 10.1039/b901970g (2010).

Shelbourne, M., Chen, X., Brown, T. & El-Sagheer, A. H. Fast copper-free click DNA ligation by the ring-strain promoted alkyne-azide cycloaddition reaction. Chemical Communications 47, 6257-6259; 10.1039/c1cc10743g (2011).

Merkel, M., Peewasan, K., Arndt, S., Ploschik, D. & Wagenknecht, H.-A. Copper-free postsynthetic labeling of nucleic acids by means of bioorthogonal reactions. Chembiochem 16, 1541-1553; 10.1002/cbic.201500199 (2015).

Eeftens, J. M., van der Torre, J., Burnham, D. R. & Dekker, C. Copper-free click chemistry for attachment of biomolecules in magnetic tweezers. BMC Biophysics 8, 9; 10.1186/s13628-015-0023-9 (2015).

Sluyterman, L.A.Æ. The activation reaction of papain. Biochimica et Biophysica Acta (BBA)-Enzymology 139, 430-438; 10.1016/0005-2744(67)90046-0 (1967).

Drenth, J., Jansonius, J. N., Koekoek, R. & Wolthers, B. G. The structure of papain. In Advances in protein chemistry. Volume 25, edited by C. B. Anfinsen, F. M. Richards & J. T. Edsall (Academic Press, New York, London, 1971), Vol. 25, pp. 79-115.

Smith, D. J., Miggio, E. T. & Kenyon, G. L. Simple alkanethiol groups for temporary blocking of sulfhydryl groups of enzymes. Biochemistry 14, 766-771; 10.1021/bi00675a019 (1975).

Shaked, Z., Szajewski, R. P. & Whitesides, G. M. Rates of thiol-disulfide interchange reactions involving proteins and kinetic measurements of thiol pKa values. Biochemistry 19, 4156-4166; 10.1021/bi00559a004 (1980).

Lukesh, J. C., Palte, M. J. & Raines, R. T. A potent, versatile disulfide-reducing agent from aspartic acid. Journal of the American Chemical Society 134, 4057-4059; 10.1021/ja211931f (2012).

Lukesh, J. C., Vanveller, B. & Raines, R. T. Thiols and selenols as electron-relay catalysts for disulfidebond reduction. Angewandte Chemie International Edition in English 52, 12901-12904; 10.1002/anie.201307481 (2013).

Jacob, C., Giles, G. I., Giles, N. M. & Sies, H. Sulfur and selenium: the role of oxidation state in protein structure and function. Angewandte Chemie International Edition in English 42, 4742-4758; 10.1002/anie.200300573 (2003).

Huber, R. E. & Criddle, R. S. Comparison of the chemical properties of selenocysteine and selenocystine with their sulfur analogs. Archives of Biochemistry and Biophysics 122, 164-173; 10.1016/0003-9861(67)90136-1 (1967).

Maroney, M. J. & Hondal, R. J. Selenium versus sulfur: Reversibility of chemical reactions and resistance to permanent oxidation in proteins and nucleic acids. Free Radical Biology & Medicine 127, 228-237; 10.1016/j.freeradbiomed.2018.03.035 (2018).

Pleasants, J. C., Guo, W. & Rabenstein, D. L. A comparative study of the kinetics of selenol/diselenide and thiol/disulfide exchange reactions. Journal of the American Chemical Society 111, 6553-6558; 10.1021/ja00199a012 (1989).

Rabenstein, D. L. & Weaver, K. H. Kinetics and Equilibria of the Thiol/Disulfide Exchange Reactions of So-matostatin with Glutathione. The Journal of Organic Chemistry 61, 7391-7397; 10.1021/jo960917 (1996).

Nauser, T., Steinmann, D. & Koppenol, W. H. Why do proteins use selenocysteine instead of cysteine? Amino Acids 42, 39-44; 10.1007/s00726-010-0602-7 (2012).

Hondal, R. J., Marino, S. M. & Gladyshev, V. N. Selenocysteine in thiol/disulfide-like exchange reactions. Antioxidants & Redox Signaling 18, 1675-1689; 10.1089/ars.2012.5013 (2013).

Nauser, T., Dockheer, S., Kissner, R. & Koppenol, W. H. Catalysis of electron transfer by selenocysteine. Biochemistry 45, 6038-6043; 10.1021/bi0602260 (2006).

Steinmann, D. et al. Kinetics of tyrosyl radical reduction by selenocysteine. Biochemistry 47, 9602-9607; 10.1021/bi801029f (2008).

Burns, J. A., Butler, J. C., Moran, J. & Whitesides, G. M. Selective reduction of disulfides by tris(2-carboxy-ethyl)phosphine. The Journal of Organic Chemistry 56, 2648-2650; 10.1021/jo00008a014 (1991).

Hansen, P. R. & Oddo, A. Fmoc solid-phase peptide synthesis. Methods in Molecular Biology 1348, 33-50; 10.1007/978-1-4939-2999-3_5 (2015).

Stawikowski, M. & Fields, G. B. Introduction to Peptide Synthesis. Current Protocols in Protein Science Chapter 18, Unit-18.1; 10.1002/0471140864.ps1801s26 (2002).

Bachrach, S. M., Demoin, D. W., Luk, M. & Miller, J. V. Nucleophilic attack at selenium in diselenides and selenosulfides. A computational study. The Journal of Physical Chemistry A 108, 4040-4046; 10.1021/jp037972o (2004).

Steinmann, D., Nauser, T. & Koppenol, W. H. Selenium and sulfur in exchange reactions: a comparative study. The Journal of Organic Chemistry 75, 6696-6699; 10.1021/jo1011569 (2010).

Eckenroth, B. E., Rould, M. A., Hondal, R. J. & Everse, S. J. Structural and biochemical studies reveal differences in the catalytic mechanisms of mammalian and Drosophila melanogaster thioredoxin reductases. Biochemistry 46, 4694-4705; 10.1021/bi602394p (2007).

Mampuys, P., McElroy, C. R., Clark, J. H., Orru, R. V. A. & Maes, B. U. W. Thiosulfonates as emerging reactants: Synthesis and applications. Advanced Synthesis & Catalysis 362, 3-64; 10.1002/adsc.201900864 (2020).

Olson, B. J. S. C. Assays for determination of protein concentration. Current Protocols in Pharmacology 73, A.3A.1-A.3A.32; 10.1002/cpph.3 (2016).

Barrett, A. J. & Kirschke, H. Cathepsin B, cathepsin H, and cathepsin L. In Proteolytic enzymes, edited by L. Lorand (Acad. Press, New York, NY, 1981), Vol. 80, pp. 535-561.

Hanada, K. et al. Structure and synthesis of E-64, a new thiol protease inhibitor. Agricultural and Biological Chemistry 42, 529-536; 10.1080/00021369.1978.10863015 (1978).

Singh, R. & Whitesides, G. M. Thiol-Disulfide Interchange. In The Chemistry of Sulfur-Containing Functional Groups, edited by S. Patai, Vol. 25, 633-658, (1993).

Danehy, J. P. & Parameswaran, K. N. Acidic dissociation constants of thiols. Journal of Chemical & Engineering Data 13, 386-389; 10.1021/je60038a025 (1968).

Bordwell, F. G. & Hughes, D. L. Thiol acidities and thiolate ion reactivities toward butyl chloride in dimethyl sulfoxide solution. The question of curvature in Broensted plots. The Journal of Organic Chemistry 47, 3224-3232; 10.1021/jo00138a005 (1982).

Singh, R. & Whitesides, G. M. A reagent for reduction of disulfide bonds in proteins that reduces disulfide bonds faster than does dithiothreitol. The Journal of Organic Chemistry 56, 2332-2337; 10.1021/jo00007a018 (1991).

Lamoureux, G. V. & Whitesides, G. M. Synthesis of dithiols as reducing agents for disulfides in neutral aqueous solution and comparison of reduction potentials. The Journal of Organic Chemistry 58, 633-641; 10.1021/jo00055a015 (1993).

Singh, R., Lamoureux, G. V., Lees, W. J. & Whitesides, G. M. Reagents for rapid reduction of disulfide bonds. In Monothiols and dithiols, protein thiols, and thiyl radicals, edited by L. Packer (Acad. Press, San Diego, 1995), Vol. 251, pp. 167-173

Berger, A. & Schechter, I. Mapping the active site of papain with the aid of peptide substrates and inhibitors. Philosophical Transactions of the Royal Society B 257, 249-264; 10.1098/rstb.1970.0024 (1970).

Singh, R. & Whitesides, G. M. Selenols catalyze the interchange reactions of dithiols and disulfides in water. The Journal of Organic Chemistry 56, 6931-6933; 10.1021/jo00024a041 (1991).

Dickson, R. C. & Tappel, A. L. Reduction of selenocystine by cysteine or glutathione. Archives of Biochemistry and Biophysics 130, 547-550; 10.1016/0003-9861(69)90068-X (1969).

Burnell, J. N., Karle, J. A. & Shrift, A. Reduction of DL-selenocystine and isolation of L-seleoncysteine. Journal of inorganic biochemistry 12, 343-351 (1980).

Burns, J. A., Butler, J. C., Moran, J. & Whitesides, G. M. Selective reduction of disulfides by tris(2-carboxyethyl)phosphine. The Journal of Organic Chemistry 56, 2648-2650; 10.1021/jo00008a014 (1991).

Musiani, M., Gallinella, G., Venturoli, S. & Zerbini, M. Competitive PCR-ELISA protocols for the quantitative and the standardized detection of viral genomes. Nature Protocols 2, 2511-2519; 10.1038/nprot.2007.312 (2007).

Musiani, M., Venturoli, S., Gallinella, G. & Zerbini, M. Qualitative PCR-ELISA protocol for the detection and typing of viral genomes. Nature Protocols 2, 2502; 10.1038/nprot.2007.311 (2007).

Cleland, W. W. Dithiothreitol, a new protective reagent for SH groups dithiothreitol. Biochemistry 3, 480-482; 10.1021/bi00892a002 (1964)

Lamoureux, G. V. & Whitesides, G. M. Synthesis of dithiols as reducing agents for disulfides in neutral aqueous solution and comparison of reduction potentials. The Journal of Organic Chemistry 58, 633-641; 10.1021/jo00055a015 (1993).

Lees, W. J., Singh, R. & Whitesides, G. M. meso-2,5-Dimercapto-N,N,N',N'-tetramethyladipamide: a readily available, kinetically rapid reagent for the reduction of disulfides in aqueous solution. The Journal of Organic Chemistry 56, 7328-7331; 10.1021/jo00026a026 (1991).

Gorzynski, J. E. et al. High-throughput SARS-CoV-2 and host genome sequencing from single nasopharyngeal swabs. medRxiv : the preprint server for health sciences; 10.1101/2020.07.27.20163147 (2020).

Klingen, R. L. et al. Over 90% of clinical swabs used for SARS-CoV-2 diagnostics contain sufficient nucleic acid concentrations. Journal of medical virology 93, 2848-2856; 10.1002/jmv.26706 (2021).

Fujii, N., Otaka, A., Sugiyama, N., Hatano, M. & Yajima, H. Studies on peptides. CLV. Evaluation of trimethylsilyl bromide as a hard-acid deprotecting reagent in peptide synthesis. Chemical and Pharmaceutical Bulletin 35, 3880-3883; 10.1248/cpb.35.3880 (1987).

Fujii, N. et al. Trimethylsilyl trifluoromethanesulphonate as a useful deprotecting reagent in both solution and solid phase peptide syntheses. Journal of the Chemical Society, 274-275; 10.1039/c39870000274 (1987).

## Claims

1. A method for detecting a target nucleic acid in a sample, the method comprising the following steps:
(a) coupling of an amplification product of the target nucleic acid, wherein said amplification product is generated if said target nucleic acid is in the sample, comprising modified nucleotides having a first reactive moiety to a second reactive moiety that is linked to a cysteine protease activator molecule to produce amplification products of the target nucleic acid labelled with a cysteine protease activator molecule;
(b) optionally immobilizing the labelled amplification products on a surface;
(c) contacting the optionally immobilized, labelled amplification products with a reversibly inactivated cysteine protease, wherein the catalytic cysteine residue of the reversibly inactivated cysteine protease is blocked by a disulfide bond or a selenylsulfide bond, and reactivating the proteolytic activity of said cysteine protease by said cysteine protease activator molecule by disulfide bond or selenylsulfide bond reduction;
(d) contacting the reactivated cysteine protease with a cysteine protease substrate, wherein said substrate provides a detectable signal when hydrolysed by said reactivated cysteine protease; and
(e) monitoring said detectable signal, wherein the presence of the detectable signal is indicative of the presence of said target nucleotide sequence in said sample.

2. The method of claim 1, further comprising step (a1) before step (a): amplifying the target nucleic acid via polymerase chain reaction, wherein modified nucleotides comprising said first reactive moiety are incorporated into the amplification product.

3. The method of claim 2, further comprising step (a2) after step (a1) and before step (b): essentially removing unreacted cysteine protease activator molecules and/or unreacted polymerase chain reaction components.

4. The method of claim 2 or 3, wherein step (a1) comprises amplifying the target nucleic acid sequence in the presence of primers comprising a terminal surface binding moiety at the 5' end.

5. The method of claim 2 or 3, further comprising a step (b1) after step (a) and before step (b): hybridizing the labelled amplification product to a nucleic acid probe that is complementary to a portion of the amplification product, wherein the nucleic acid probe comprises a surface binding moiety.

6. The method of claim 4 or 5, wherein the surface binding moiety is a biotin moiety and wherein the surface in step (b) is a streptavidin solid phase.

7. The method of any one of claims 1 to 6, wherein the cysteine protease is papain or a papain-like protease comprising or consisting of SEQ ID NO: 1 or of an amino acid sequence which is at least 90% identical to the amino acid sequence of SEQ ID NO: 1, wherein preferably, the catalytic cysteine residue at position 25 and the catalytic histidine at position 159 are retained.

8. The method of any one of claims 1 to 7, wherein the first reactive moiety of said modified nucleotides is a terminal alkyne moiety and the second reactive moiety that is linked to said cysteine protease activator molecule is an azide moiety.

9. The method of claim 8, wherein the reactive moiety of the modified nucleotide is dibenzocyclooctyne (DBCO).

10. The method of any one of claims 1 to 9, further comprising step (c1) after step (b) and before step (c): reversibly inactivating a proteolytically active cysteine protease by reaction with a thiosulfonate derivative, wherein preferably, the thiosulfonate derivative is S-Methyl methanethiosulfonate.

11. The method of any one of claims 1 to 10, wherein the cysteine protease activator molecule comprises a free thiol or selenol residue.

12. The method of claim 11, wherein the cysteine protease activator molecule is:
a) a non-peptide activator (DTBA), seleno-dithiobutylamin (SeDTBA) or an aminothiophenol; or
b) a peptide activator comprising a cysteine moiety or a selenocysteine moiety; or
c) the cysteine protease activator molecule of any one of claims 16 to 22; or
d) the cysteine protease activator molecule of claim 23.

13. The method of claim 12 (b), wherein the peptide activator comprises or consists of 2 to 5 amino acids in length, a cysteine or a selenocysteine moiety and a C-terminal arginine, glycine or phenylalanine residue or a C-terminal arginine amide, glycine-amide or phenylalanine residue.

14. The method of any one of claims 1 to 13, wherein the cysteine protease substrate is Z-Phe-Arg 7-amino-4-Methylcoumarin (Z-Phe-Arg-AMC).

15. The method of any one of claims 1 to 14, wherein step c) further comprises the addition of Tris(2-carboxyethyl)phosphine (TCEP).

16. A cysteine protease activator molecule for reactivating a reversibly inactivated cysteine protease comprising: a tripeptide comprising a selenocysteine moiety and a C-terminal arginine or arginine-amide.

17. The cysteine protease activator molecule of claim 16, wherein the tripeptide is H₂N-ULR-COOH or H₂N-ULR-CONH₂.

18. The cysteine protease activator molecule of claims 16 or 17, further comprising a second of said tripeptides, wherein the selenocysteine moieties of said tripeptides are coupled via a diselenide bond.

19. The cysteine protease activator molecule of any one of claims 16 to 18, wherein the tripeptide(s) is (are) N-terminally coupled to a linker comprising an azide moiety.

20. The cysteine protease activator molecule of claim 19, wherein the linker comprises or consists of a polyethylenglycol moiety and an azide moiety.

21. The cysteine protease activator of claim 20, wherein the linker is O-2-Azidoethyl-O'-[2-(succinylamino)-ethyl]dimethyleneglycol.

22. The cysteine protease activator molecule of claim 21, wherein the linker is coupled to a dNTP via DBCO.

23. A cysteine protease molecule comprising a peptide comprising 2 to 5 amino acids in length, a cysteine moiety and a C-terminal arginine, glycine or phenylalanine or a C-terminal arginine amide, glycine amide or phenylalanine amide, wherein peptide is N-terminally coupled to a modified nucleotide comprising a DBCO moiety.

24. A kit of parts for detecting a target nucleotide sequence in a sample comprising:
(a) a reversibly inactivated cysteine protease, wherein the catalytic cysteine residue of the reversibly inactivated cysteine protease is blocked by a disulfide or selenylsulfide bond;
(b) a modified nucleotide having a first reactive moiety, wherein the first reactive moiety is preferably an alkyne moiety and even more preferably, wherein the first reactive moiety is DBCO;
(c) the cysteine protease activator molecule as defined in claim 11, wherein the cysteine protease activator molecule is linked to a second reactive moiety, wherein the second reactive moiety preferably is an azide moiety; or the cysteine protease activator molecule of any one of claims 20 to 21;
(d) optionally, Tris(2-carboxyethyl)phosphine (TCEP); and
(e) a cysteine protease substrate, wherein said substrate provides a detectable signal when hydrolysed by said reactivated cysteine protease.
